# EUROPEAN PATENT SPECIFICATION

(11) **EP 2 970 982 B1**
(45) Date of publication and mention of the grant of the patent: **28.10.2020**
(21) Application number: 14763831.6
(22) Date of filing: 15.03.2014
(51) Int. Cl.: C12N 15/52, C12N 9/00, C12N 1/21

(54) **ACETYL-COA CARBOXYLASE MUTATIONS**
ACETYL-COA-CARBOXYLASEMUTATIONEN
MUTATIONS D'ACÉTYL-COA CARBOXYLASE

(30) Priority: 15.03.2013 US 201361852387 P; 15.03.2013 US 201361791743 P
(43) Date of publication of application: 20.01.2016
(73) Proprietor: Cargill, Incorporated, Wayzata, MN 55391 (US)
(72) Inventor: LIAO, Hans, Superior, CO 80027 (US); MERCOGLIANO, Chrisopher Patrick, Minneapolis, MN 55403 (US); WOLTER, Travis Robert, Denver, CO 80211 (US); LOUIE, Michael Tai Man, Broomfield, CO 80020 (US); RIBBLE, Wendy Kathleen, Arvada, CO 80003 (US); LIPSCOMP, Tanya, Boulder, CO 80303 (US); SPINDLER, Eileen Colie, Lafayette, CO 80026 (US); LYNCH, Michael, Durham, NC 27708 (US)
(74) Representative: Forresters IP LLP
(86) International application number: PCT/US2014/030074
(87) International publication number: WO 2014/145334

(56) References cited:
- WO-A1-2011/038364
- WO-A1-2013/003608
- WO-A1-2014/146026
- WO-A2-00/39287
- WO-A2-2013/152051
- WO-A2-2013/152052
- US-A1- 2009 325 248
- US-A1- 2010 210 017
- US-A1- 2012 116 108
- US-B1- 6 306 636
- STEPHANIE C. WEATHERLY ET AL: "Expression and characterization of recombinant fungal acetyl-CoA carboxylase and isolation of a soraphen-binding domain", BIOCHEMICAL JOURNAL, vol. 380, no. 1, 15 May 2004 (2004-05-15), pages 105-110, XP055302533, GB ISSN: 0264-6021, DOI: 10.1042/bj20031960
- ZHA W ET AL: "Improving cellular malonyl-CoA level in Escherichia coli via metabolic engineering", METABOLIC ENGINEERING, ACADEMIC PRESS, US, vol. 11, no. 3, 1 May 2009 (2009-05-01), pages 192-198, XP026034671, ISSN: 1096-7176, DOI: 10.1016/J.YMBEN.2009.01.005 [retrieved on 2009-02-05]
- IKUO MIYAHISA ET AL: "Efficient production of (2S)-flavanones by Escherichia coli containing an artificial biosynthetic gene cluster", APPLIED MICROBIOLOGY AND BIOTECHNOLOGY, SPRINGER, BERLIN, DE, vol. 68, no. 4, 1 September 2005 (2005-09-01), pages 498-504, XP019331939, ISSN: 1432-0614, DOI: 10.1007/S00253-005-1916-3
- JAN PODKOWINSKI ET AL: "OPINIONS Acetyl-coenzyme A carboxylase - an attractive enzyme for biotechnology", BIOTECHNOLOGIA, vol. 4, 1 January 2011 (2011-01-01), pages 321-335, XP055303418, PL ISSN: 0860-7796, DOI: 10.5114/bta.2011.46549
- JULIANO ALVES ET AL: "Cloning, expression, and enzymatic activity ofandacetyl-coenzyme A carboxylases", ANALYTICAL BIOCHEMISTRY, ACADEMIC PRESS INC, NEW YORK, vol. 417, no. 1, 25 May 2011 (2011-05-25), pages 103-111, XP028245778, ISSN: 0003-2697, DOI: 10.1016/J.AB.2011.05.041 [retrieved on 2011-06-01]
- JAMES, ETHAN S. ET AL.: 'Expression of two Escherichia coli acetyl-CoA carboxylase subunits is autoregulated' J. BIOL. CHEM. vol. 279, no. 4, 31 October 2003, pages 2520 - 2527, XP007903432

## Description

This application claims priority to U.S. Provisional Patent Application No. 61/852,387, filed on March 15, 2013; and U.S. Provisional Patent Application No. 61/791,743, filed on March 15, 2013.

### BACKGROUND OF THE INVENTION

There is a need for alternative production methods of industrial chemicals used for various consumer products and fuels that are currently made from petroleum. One alternative method is the use of engineered microorganisms to produce industrial chemicals. Currently, in the field of bioproduced chemicals there is a need to improve microbial enzyme performance, enhanced production rate in order to reach the goal of becoming an at-cost replacement basis for petro-based chemicals. Weatherly et al. "Expression and characterization of recombinant fungal acetyl-CoA carboxylase and isolation of a soraphen-binding domain" Biochemical Society (2004) 380, 105-110 discloses the recombinant expression of enzymatically active, full-length, fungal (*Ustilago maydis)* ACC in *Escherichia coli.* Zha et al. "Improving cellular malonyl-CoA level in Escherichia coli via metabolic engineering" Metabolic Engineering 11 (2009) 192-198 discloses the creation of an *E. coli* strain with 15-fold elevated cellular malonyl-CoA level.

A common challenge faced in field of bio-produced chemicals in microorganisms is that any one modification to a host cell may require coordination with other modifications in order to successfully enhance chemical bioproduction.

Provided herein are, *e.g*., methods, systems of fermentation, genetically modified microorganisms, and modified enhanced enzymes for chemical production, all of which may be used *e.g.,* in various combinations to increase chemical production of a desired chemical product.

### SUMMARY OF THE INVENTION

The invention is defined in the appended claims. Any embodiments disclosed herein that fall outside the scope of the claims are not part of the invention. The inventive embodiments provided in this Summary of the Invention are meant to be illustrative only and to provide an overview of selected embodiments disclosed herein. The Summary of the Invention, being illustrative and selective, does not limit the scope of any claim, does not provide the entire scope of inventive embodiments disclosed or contemplated herein, and should not be construed as limiting or constraining the scope of this disclosure or any claimed inventive embodiment.

Provided herein are genetically modified organisms capable of producing an industrial chemical product of interest, wherein the genetic modification includes introduction of nucleic acid sequences coding for polynucleotides encoding the following: (1) an acetyl-CoA carboxylase gene with one or more of its subunits fused together in the genetic structure of the organism; and (2) an acetyl-CoA carboxylase gene having a predefined stoichiometric ratio of each of the four ACCase subunits relative to one another.

Also provided herein are methods of producing a chemical product using a genetically modified organism capable of producing an industrial chemical product of interest, wherein the genetic modification can include introduction of nucleic acid sequences coding for polynucleotides encoding the following: (1) an acetyl-CoA carboxylase gene with one or more of its subunits fused together in the genetic structure of the organism; and (2) an acetyl-CoA carboxylase gene having a predefined stoichiometric ratio of each of the four ACCase subunits relative to one another.

Further provided in the present disclsoure are products made from a genetically modified organisms capable of producing an industrial chemical product of interest, wherein the genetic modification includes introduction of nucleic acid sequences coding for polynucleotides encoding one or more of the following: (1) an acetyl-CoA carboxylase gene with one or more of its subunits fused together in the genetic structure of the organism; and/or (2) an acetyl-CoA carboxylase gene having a predefined stoichiometric ratio of each of the four ACCase subunits relative to one another. The present disclosure also relates to products made from such methods.

Provided herein are genetically modified organisms (GMOs) capable of producing an industrial chemical product of interest, wherein the GMOs comprise a genetic modification including introduction of one or more nucleic acid sequences encoding following: (1) an acetyl-CoA carboxylase (ACCase) with one or more of its subunits fused together; and (2) an acetyl-CoA carboxylase having a predefined stoichiometric ratio of each of the four ACCase subunits relative to one another.

Also provided in the present disclosure are methods of producing a chemical product using a genetically modified organism capable of producing an industrial chemical product of interest, wherein the genetic modification includes introduction of at least one nucleic acid sequence encoding one or more enzymes selected from the group consisting of: (1) a malonyl-CoA reductase that is mutated to enhance its activity at lower temperatures; (2) a salt-tolerant enzyme; (3) a polypeptide that facilitates the exportation of a chemical product of interest or the export of an inhibitory chemical from the cell; and/or (4) a polypeptide that facilitates the importation into a cell of a reactant, precursor, and/or metabolite used for producing a chemical product of interest.

Further provided in the present disclosure are products made from a genetically modified organism capable of producing an industrial chemical product of interest, wherein the genetic modification includes introduction of at least one nucleic acid sequence encoding one or more enzymes selected from the group consisting of: (1) a malonyl-CoA reductase that is mutated to enhance its activity at lower temperatures; (2) a salt-tolerant enzyme; (3) a polypeptide that facilitates the exportation of a chemical product of interest or the export of an inhibitory chemical from the cell; and/or (4) a polypeptide that facilitates the importation into a cell of a reactant, precursor, and/or metabolite used for producing a chemical product of interest.

Provided in the present disclosure are (1) a monofunctional malonyl-CoA reductase gene capable of catalyzing the conversion of malonyl-CoA to malonate semialdehyde and one or more genes encoding one or more of the following enzymes: ydfG, mmsB, NDSD, rutE, and/or nemA; (2) a monofunctional malonyl-CoA reductase gene capable of catalyzing the conversion of malonyl-CoA to malonate semialdehyde and one or more genes encoding one or more enzymes capable of converting malonate semialdehyde keto form to 3-HP, and one or more genes encoding one or more enzymes capable of converting either the malonate semialdehyde enol form to 3-HP and/or the malonate semialdehyde hydrated form to 3-HP; (3) a monofunctional malonyl-CoA reductase enzyme fused to a dehydrogenase enzyme that is either: (a) NADPH-dependent, (b) NADH-independent, (c) has the capability of using NADPH, (d) flavin-dependent, (e), flavin-independent, (f) has the capability of using flavin, (g) less susceptible to 3-HP inhibition at high concentration, and/or (h) catalyzes a reaction pathway to 3-HP that is substantially irreversible; and/or (4) a monofunctional malonyl-CoA reductase enzyme fused to one or more malonate semialdehyde dehydrogenase enzymes.

Also provided in the present disclosure are genetically modified organisms capable of producing an industrial chemical product of interest, wherein the genetically modified organisms comprise (1) a monofunctional malonyl-CoA reductase gene capable of catalyzing the conversion of malonyl-CoA to malonate semialdehyde and one or more genes encoding one or more of the following enzymes: ydfG, mmsB, NDSD, rutE, and nemA; (2) a monofunctional malonyl-CoA reductase gene capable of catalyzing the conversion of malonyl-CoA to malonate semialdehyde and one or more genes encoding one or more enzymes capable of converting malonate semialdehyde keto form to 3-HP, and one or more genes encoding one or more enzymes capable of converting either the malonate semialdehyde enol form to 3-HP and/or the malonate semialdehyde hydrated form to 3-HP; (3) a monofunctional malonyl-CoA reductase enzyme fused to a dehydrogenase enzyme that is either: (a) NADPH-dependent, (b) NADH-independent, (c) has the capability of using NADPH, (d) flavin-dependent, (e), flavin-independent, (f) has the capability of using flavin, (g) less susceptible to 3-HP inhibition at high concentration, and/or (h) catalyzes a reaction pathway to 3-HP that is substantially irreversible; and/or (4) a monofunctional malonyl-CoA reductase enzyme fused to one or more malonate semialdehyde dehydrogenase enzymes.

Further provided in the present disclosure are methods of producing a chemical product using a genetically modified organism capable of producing an industrial chemical product of interest, wherein the genetically modified organism can comprise (1) a monofunctional malonyl-CoA reductase gene capable of catalyzing the conversion of malonyl-CoA to malonate semialdehyde and one or more genes encoding one or more of the following enzymes: ydfG, mmsB, NDSD, rutE, and/or nemA; (2) a monofunctional malonyl-CoA reductase gene capable of catalyzing the conversion of malonyl-CoA to malonate semialdehyde and one or more genes encoding one or more enzymes capable of converting malonate semialdehyde keto form to 3-HP, and one or more genes encoding one or more enzymes capable of converting either the malonate semialdehyde enol form to 3-HP and/or the malonate semialdehyde hydrated form to 3-HP; (3) a monofunctional malonyl-CoA reductase enzyme fused to a dehydrogenase enzyme that is either: (a) NADPH-dependent, (b) NADH-independent, (c) has the capability of using NADPH, (d) flavin-dependent, (e), flavin-independent, (f) has the capability of using flavin, (g) less susceptible to 3-HP inhibition at high concentration, and/or (h) catalyzes a reaction pathway to 3-HP that is substantially irreversible; and/or (4) a monofunctional malonyl-CoA reductase enzyme fused to one or more malonate semialdehyde dehydrogenase enzymes.

Further provided in the present disclosure are products made from a genetically modified organism capable of producing an industrial chemical product of interest, wherein the genetically modified organism can comprise (1) a monofunctional malonyl-CoA reductase gene capable of catalyzing the conversion of malonyl-CoA to malonate semialdehyde and one or more genes encoding one or more of the following enzymes: ydfG, mmsB, NDSD, rutE, and/or nemA; (2) a monofunctional malonyl-CoA reductase gene capable of catalyzing the conversion of malonyl-CoA to malonate semialdehyde and one or more genes encoding one or more enzymes capable of converting malonate semialdehyde keto form to 3-HP, and one or more genes encoding one or more enzymes capable of converting either the malonate semialdehyde enol form to 3-HP and/or the malonate semialdehyde hydrated form to 3-HP; (3) a monofunctional malonyl-CoA reductase enzyme fused to a dehydrogenase enzyme that is either: (a) NADPH-dependent, (b) NADH-independent, (c) has the capability of using NADPH, (d) flavin-dependent, (e), flavin-independent, (f) has the capability of using flavin, (g) less susceptible to 3-HP inhibition at high concentration, and/or (h) catalyzes a reaction pathway to 3-HP that is substantially irreversible; and/or (4) a monofunctional malonyl-CoA reductase enzyme fused to one or more malonate semialdehyde dehydrogenase enzymes.

Provided in the present disclosure are genetically modified organisms ("GMOs") capable of producing an industrial chemical product of interest, wherein the genetic modification can include introduction of at least one nucleic acid sequence encoding one or more enzymes selected from the group consisting of: (1) a malonyl-CoA reductase that is mutated to enhance its activity at lower temperatures; (2) a salt-tolerant enzyme; (3) a polypeptide that facilitates the exportation of a chemical product of interest or the export of an inhibitory chemical from the cell; and/or (4) a polypeptide that facilitates the importation into a cell of a reactant, precursor, and/or metabolite used for producing a chemical product of interest.

Also provided herein are methods of producing a chemical product using the genetically modified organisms herein. Further provided herein are products made from these methods. In certain embodiments, the products can be acetyl-CoA, malonyl-CoA, malonate semialdehyde, 3-hydroxypropionic acid (3-HP), acrylic acid, 1,3 propanediol, malonic acid, ethyl 3-HP, propiolactone, acrylonitrile, acrylamide, methyl acrylate, a polymer including super absorbent polymers and polyacrylic acid, and/or a consumer product.

Provided in the present disclosure are methods of producing a chemical product from a renewable carbon source through a bioproduction process that can comprise a controlled multi-phase production process wherein the initiation and/or completion of one or more phases of the production process can be controlled by genetic modifications to the organism producing the chemical product and/or can be controlled by changes made to the cell environment. The bioproduction process may include two or more of the following phases: (1) growth phase; (2) induction phase; and (3) production phase. Also provided are products made from these methods.

Also provided in the present disclosure are methods of making GMOs herein.

Additionally, provided herein are methods of producing a chemical product using a GMO herein.

Further provided in the present disclosure are products produced using a GMO herein which can be, *e.g.,* capable of producing an industrial chemical product of interest.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
**FIG. 1** Depicts some embodiments of the metabolic pathways to produce 3-hydroxypropionic acid ("3-HP").
**FIG. 2** Depicts some embodiments of the of various equilibrium states in the malonate semialdehyde to 3-HP reaction in a cell environment.
**FIG. 3** Depicts some embodiments of the reaction catalyzed by acetyl-CoA carboxylase (ACCase)
**FIG. 4** Shows the inhibition of ACCase enzyme activity by high salt concentration
**FIG. 5** Depicts some embodiments of the fusion ACCase subunit gene constructs overexpressed in *E. coli.* CAT = chloramphenicol resistance marker; p15a rep = replication origin; red arrow = promoter.
**FIG. 6** Show improved production of 3-HP by genetically modified organism with DA fusion ACCase.
**FIG. 7** Shows improved production of 3-HP by genetically modified organism with overexpression of rhtA exporter.
**FIG. 8** Shows various embodiments of the genetic modules used for optimizing expression in host cells.

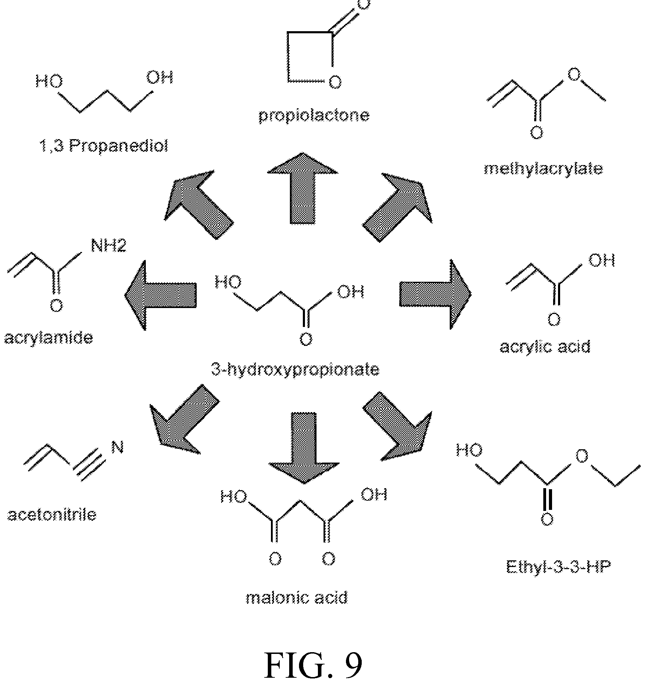

**FIG. 9** Shows various chemical products that can made from various embodiments of the invention.
**FIG. 10** Shows an enzymatic reaction of the transport of homoserine (threonine and homoserine exporters).
**FIG. 11** Shows results from the overexpression of 33 transporters (adapted from Yamada S, Awano N, Inubushi K, Maeda E, Nakamori S, Nishino K, Yamaguchi A, Takagi H. (2006))
**FIG. 12** Shows the average OD after 24 hours of cultures grown in M9 in the presence of different levels of 3-HP at different pHs.
**FIGs. 13A-D** Shows multiple sequence alignments for rhtA.
**FIGs. 14A-K** Shows pileup sequence comparisons for rhtA.

**Table** 1. Lists the accession numbers for genes encoding ACCase subunits from *Halomonas elongate* (*"H. elongate"*).
**Table 2.** Depicts some embodiments of the RBS sequences used to enhance expression of *H. elongate* ACCase subunits.
**Table 3.** Shows improvement in 3-HP production by RBS-optimized expression of *H. elongata* ACCase subunits.
**Table 4.** Shows some embodiments of the ACCase subunit fusions that increase and ACCase enzyme complex activity.

### DETAILED DESCRIPTION OF THE INVENTION

### DEFINITIONS

As used herein, the term "homology" can refer to the optimal alignment of sequences (either nucleotides or amino acids), which may be conducted by computerized implementations of algorithms. "Homology", with regard to polynucleotides, for example, may be determined by analysis with BLASTN version 2.0 using the default parameters. "Homology", with respect to polypeptides (*i.e*., amino acids), may be determined using a program, such as BLASTP version 2.2.2 with the default parameters, which aligns the polypeptide or fragments (and can also align nucleotide fragments) being compared and determines the extent of amino acid identity or similarity between them. It will be appreciated that amino acid "homology" includes conservative substitutions, *i.e.* those that substitute a given amino acid in a polypeptide by another amino acid of similar characteristics. Typically seen as conservative substitutions are the following replacements: replacements of an aliphatic amino acid such as Ala, VaI, Leu and Ile with another aliphatic amino acid; replacement of a Ser with a Thr or vice versa; replacement of an acidic residue such as Asp or Glu with another acidic residue; replacement of a residue bearing an amide group, such as Asn or Gln, with another residue bearing an amide group; exchange of a basic residue such as Lys or Arg with another basic residue; and replacement of an aromatic residue such as Phe or Tyr with another aromatic residue. For example, homologs can have about: 99%, 98%, 97%, 96%, 95%, 94%, 93%, 92%, 91%, 90%, 89%, 88%, 87%, 86%, 85%, 84%, 83%, 82%, 81%, 80%, 79%, 78%, 77%, 76%, 75%, 74%, 73%, 72%, 71%, 70%; or at least 70%, at least 75%, at least 80%, at least 85%, at least 90%, at least 91%, at least 92%, at least 93%, at least 94%, at least 95%, at least 96%, at least 97%, at least 98%, or at least 99% overall amino acid or nucleotide identity to the gene or proteins of the invention; or can have about: 99%, 98%, 97%, 96%, 95%, 94%, 93%, 92%, 91%, 90%, 89%, 88%, 87%, 86%, 85%, 84%, 83%, 82%, 81%, 80%, 79%, 78%, 77%, 76%, 75%, 74%, 73%, 72%, 71%, or 70% overall amino acid or nucleotide identity to the essential protein functional domains of the gene or proteins of the invention; or at least: 99%, 98%, 97%, 96%, 95%, 94%, 93%, 92%, 91%, 90%, 89%, 88%, 87%, 86%, 85%, 84%, 83%, 82%, 81% 80%, 79%, 78%, 77%, 76%, 75%, 74%, 73%, 72%, 71%, or 70%, overall amino acid or nucleotide to the essential binding amino acids within an essential functional domain of the gene or proteins of the invention.

The above descriptions and methods for sequence homology are intended to be exemplary and it is recognized that this concept is well-understood in the art. Further, it is appreciated that nucleic acid sequences may be varied and still provide a functional enzyme, and such variations are within the scope of the present invention. The term "enzyme homolog" can also mean a functional variant.

The term "functional homolog" can describe a polypeptide that is determined to possess an enzymatic activity and specificity of an enzyme of interest but which has an amino acid sequence different from such enzyme of interest. A corresponding "homolog nucleic acid sequence" may be constructed that is determined to encode such an identified enzymatic functional variant.

The term "3-HP" means 3-hydroxypropionic acid.

The term "heterologous DNA," "heterologous nucleic acid sequence," and the like as used herein can refer to a nucleic acid sequence wherein at least one of the following is true: (a) the sequence of nucleic acids is foreign to (*i.e*., not naturally found in) a given host microorganism; (b) the sequence may be naturally found in a given host microorganism, but in an unnatural (*e.g*., greater than expected) amount; and/or (c) the sequence of nucleic acids comprises two or more subsequences that are not found in the same relationship to each other in nature. For example, regarding instance (c), a heterologous nucleic acid sequence that is recombinantly produced can *e.g.,* have two or more sequences from unrelated genes arranged to make a new functional nucleic acid. Embodiments of the present disclosure may result from introduction of an expression vector into a host microorganism, wherein the expression vector contains a nucleic acid sequence coding for an enzyme that is, or is not, normally found in a host microorganism. With reference to the host microorganism's genome prior to the introduction of the heterologous nucleic acid sequence, then, the nucleic acid sequence that codes for the enzyme is heterologous (whether or not the heterologous nucleic acid sequence is introduced into that genome). The term "heterologous" is intended to include the term "exogenous" as the latter term is generally used in the art as well as "endogenous".

As used herein and unless otherwise indicated, the singular forms "a," "an," and "the" include plural referents (*e.g.*, mean one or more). Thus, for example, reference to an "expression vector" includes a single expression vector as well as a plurality of expression vectors, either the same *(e.g.,* the same operon) or different; reference to "microorganism" includes a single microorganism as well as a plurality of microorganisms; and the like.

As used herein and unless otherwise indicated, the term "organism" refers to any contiguous living system. Examples of organisms can include, but are not limited to, animals, fungus, microorganisms, and/or plants. The term organism is meant to encompass unicellular and/or multicellular entities, including but not limited to, prokaryotes (including but not limited to bacteria and fungus) and/or eukaryotes (including, but not limited to, viruses).

As used herein and unless otherwise indicated, terms such as "contain", "containing", "include", "including", and the like mean comprising.

Some embodiments herein contemplate numerical ranges. When ranges are provided, the ranges include the range endpoints unless otherwise indicated. Unless otherwise indicated, numerical ranges include all values and subranges therein as if explicitly written out.

Some values herein are modified by the term "about." In some instances, the term "about" in relation to a reference numerical value can include a range of values plus or minus 10% from that value. For example the amount "about 10" can include amounts from 9 to 11. In other embodiments, the term "about" in relation to a reference numerical value can include a range of values plus or minus 10%, 9%, 8%, 7%, 6%, 5%, 4%, 3%, 2%, or 1% from that value.

Carbon sources can include *e.g.,* sugars such as *e.g.,* glucose, fructose, galactose, dextrose, maltose, glycerol, and/or a solution containing equal to or less than 50% glycerol.

A disruption of gene function may also be effectuated, in which the normal encoding of a functional enzyme by a nucleic acid sequence has been altered so that the production of the functional enzyme in a microorganism cell has been reduced or eliminated. A disruption may broadly include a gene deletion, and also includes, but is not limited to gene modification (e.g., introduction of stop co dons, frame shift mutations, introduction or removal of portions of the gene, introduction of a degradation signal), affecting mRNA transcription levels and/or stability, and altering the promoter or repressor upstream of the gene encoding the polypeptide. In some embodiments, a gene disruption is taken to mean any genetic modification to the DNA, mRNA encoded from the DNA, and the amino acid sequence resulting there from that result in at least a 50 percent reduction of enzyme function of the encoded gene in the microorganism cell.

### I. INTRODUCTION

Provided herein are, *e.g*., genetically modified microorganisms, methods for making the same, and use of the same in making industrial products. Any and all of the microorganisms herein may include a combination of genetic alterations as described herein. The present disclosure contemplates, for example, a genetically modified microorganism having one or more of the following genetic modifications: (i) an alteration that affects the stoichiometric ratio, expression or production of one or more ACCase enzyme genes (ii) a recombinant ACCase gene having at least: 70%, 75%, 80%, 85%, 90%, 91%, 92%, 93%, 94%, 95%, 96%, 97%, 98%, or 99% sequence homology to an ACCase gene from a salt tolerant organism and/or (iii) a genetic alteration in one or more non-ACCase genes.

The present disclosure contemplates, for example, a genetically modified microorganism having one or more genetic alterations that encode for one or more exporters capable of exporting 3-HP out of a cell.

The present disclosure also relates to methods of fermentation. The genetically modified microorganisms can be cultured under conditions that optimized a host cell for increase chemical production. The bio-production process may include two or more of the following phases of fermentation: (1) growth phase where the culture organism replicates itself and the carbon intermediate product is built up; (2) the induction phase, where the expression of key enzymes critical to the chemical production is induced and the enzymes accumulate within the organism to carry out the engineered pathway reactions required to further produce the chemical product (3) production phase is where the organism expresses proteins that provide for continuously production the desired chemical product. The above phases can be further controlled by (1) addition and amount of the initiating reactant added to the reaction vessel and (2) key enzymes engineered into the organism using promoters that are sensitive to (*e.g.,* activated by) the depletion of the initiating reactant. Addition details about the fermentation process of the disclosure are disclosed below.

Provided herein are, *e.g*., genetically modified microorganisms, methods for making the same, and use of the same in making industrial products. Any and all of the microorganisms herein may include a combination of genetic alterations as described herein. The present disclosure contemplates, for example, (i) new hybrid molecules or co-expressed molecules of a mono-functional malonyl-CoA reductase enzyme with various 3-HP dehydrogenase proteins that: (a) exhibit less inhibition by high 3-HP concentrations (b) can catalyze a reaction pathway to 3-HP that is less reversible, substantially irreversible and/or irreversible (c) can utilize NADH (d) enzymes that utilize and/or can utilize flavin; and (ii) can have one or more genetic alterations that can be used to channel a carbon within in standard cellular metabolic pathway into a pathway engineered to produce a desired chemical.

When utilizing certain organisms to create certain products, it may be advantageous to control each phase discretely. For example, depending on the pathway involved, reactions, reactants, intermediates and byproducts created during cell growth can inhibit enzyme induction and/or the organism's ability to produce the desired chemical product. Similarly, reactions, reactants, intermediates and byproducts created as part of the production pathway can impact cell growth, and even the increased concentration of the chemical product as it is produced can impede cell replication.

### II. ACETYL-CoA CARBOXYLASE

### Malonyl-CoA Flux

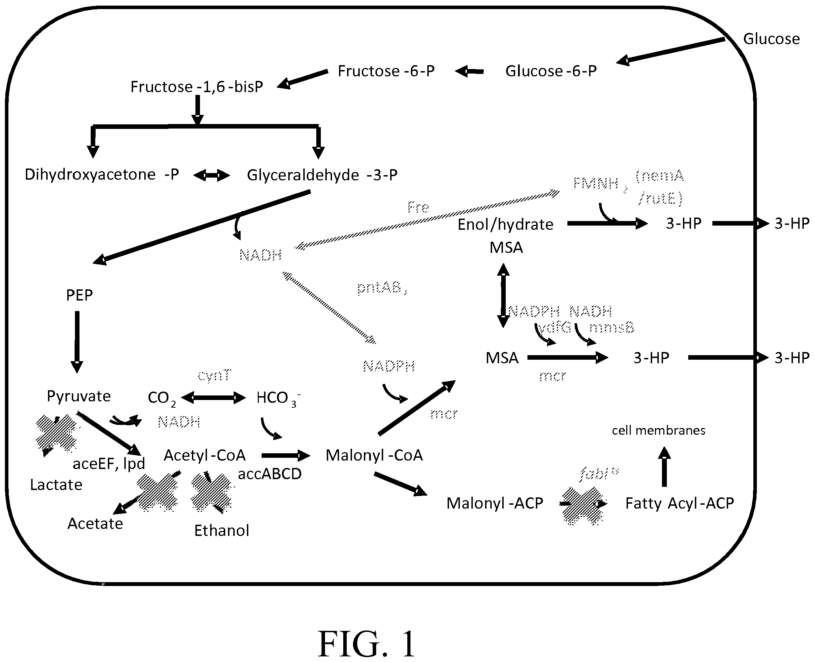

One of the steps in the biosynthesis of 3-HP involves the reaction catalyzed by acetyl-CoA carboxylase enzyme. ACCase is a primary control point in the 3-HP pathway shown in FIG. 1 (previously described in) for the converting acetyl-CoA to malonyl-CoA and hence to malonate semialdehyde and 3-HP. Inventive embodiments herein contemplate the use of genetic modifications that increase activity of ACCase complex enzymes to thereby increase 3-HP production in a host cell.

### Fused Subunits

The acetyl-CoA carboxylase complex is a multi-subunit protein. Prokaryotes and plants have multi-subunit acetyl-CoA carboxylase complexes composed of several polypeptides encoded by distinct genes. However, humans and most other eukaryotes, such as yeast, have evolved an acetyl-CoA carboxylase complex with CT and BC catalytic domains and biotin carboxyl carrier domains on a single polypeptide. The biotin carboxylase (BC) activity, biotin carboxyl carrier protein (BCCP), and carboxyl transferase (CT) activity are each contained on a different subunit. In *E. coli* the ACCase complex is derived from multi polypeptide transcribed by distinct, separable protein components known as accA, accB, accC, and accD.

Acetyl-CoA carboxylase is a biotin-dependent enzyme that catalyzes the irreversible carboxylation of acetyl-CoA to produce malonyl-CoA through its two catalytic activities, biotin carboxylase and carboxyltransferase. The first reaction is carried out by BC and involves the ATP-dependent carboxylation of biotin with bicarbonate. The carboxyl group is transferred from biotin to acetyl-CoA to form malonyl-CoA in the second reaction, which is catalyzed by CT. The main function of ACCase complex in the cell is to provide the malonyl-CoA substrate for the biosynthesis of fatty acids.

The conversion of acetyl-CoA to malonyl-CoA is an important step in the bioconversion of a renewable carbon source (such as, for example, sugar or natural gas) to a useful industrial chemical (such as, for example, 3-hydroxypropionic acid (3-HP)). In certain organisms, such as *E. coli* or yeast, the native ACCase expression from the chromosome alone is insufficient to enable the organism to produce chemicals such as 3-HP at a rate to support a commercial scale operation. Overexpression of the ACCase complex has been shown to provide some advantage. *See, e.g.,* US Patent Application Nos.: 12/891,760; 12/891,790; and 13/055,138.

Applicants have discovered that the introduction of an acetyl-CoA carboxylase gene with one or more of its subunits fused into *e.g.,* an organism or GMO is beneficial to the production of a chemical product in a host cell *(e.g.,* the organism or GMO). In any embodiment herein, fusion can be of two gene products produced from a single polynucleotide controlled by a single promoter, which *e.g.,* can further enhance an organism's bioproduction of an industrial chemical. In any embodiment herein, fusion can be of two gene products, enhanced by at least one promoter, which can further enhance an organism's bioproduction of an industrial chemical. In any embodiment herein, fusion can be of two gene products produced from a single polynucleotide controlled by at least one inducible promoter, which can further enhance an organism's bioproduction of an industrial chemical. Keeping components of the ACCase complex fused together in the genetic structure of an organism can be advantageous because *e.g*., it can enhance the stability of the non-native ACCase genetic modification and it can facilitate equimolar expression of the fused acc subunits.

In any embodiment herein, the subunit-fused ACCase *(e.g.,* in the organism, cell or GMO) *e.g.,* may be or contain an accA-accB, accA-accC, accA-accD, accB-accC, accB-accD, accC-accD, accA-accB-accC, accA-accB-accD, accA-accC-accD, accB-accC-accD, or accA-accB-accC-accD fused subunit having *e.g*., 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%, at least about 70%, at least about 71%, at least about 72%, at least about 73%, at least about 74%, at least about 75%, at least about 76%, at least about 77%, at least about 78%, at least about 79%, at least about 80%, at least about 81%, at least about 82%, at least about 83%, at least about 84%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, or at least about 99% sequence homology to the same combination of *E.coli* accA, accB, accC and accD, or their functional homologs. In addition, in any embodiment herein, the organism may include any combination of these fused subunits, or any combination of these fused subunits together with one or more of the four non-fused subunits. When such combinations are used, the subunits (fused and non-fused) may be expressed on the same plasmid or on different plasmids or on one or more of the organism's chromosome.

In any embodiment herein, the subunit-fused ACCase *(e.g.,* in the organism, cell or GMO) *e.g.,* may be or contain an accA-accB, accA-accC, accA-accD, accB-accC, accB-accD, accC-accD, accA-accB-accC, accA-accB-accD, accA-accC-accD, accB-accC-accD, or accA-accB-accC-accD fused subunit having *e.g*., about: 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%, at least about 70%, at least about 71%, at least about 72%, at least about 73%, at least about 74%, at least about 75%, at least about 76%, at least about 77%, at least about 78%, at least about 79%, at least about 80%, at least about 81%, at least about 82%, at least about 83%, at least about 84%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, or at least about 99% sequence homology to the same combination of *E.coli* accA, accB, accC and accD, or their functional homologs. In addition, in any embodiment herein, the organism may include any combination of these fused subunits, or any combination of these fused subunits together with one or more of the four non-fused subunits. When such combinations are used, the subunits (fused and non-fused) may be expressed on the same plasmid or on different plasmids or on one or more of the organism's chromosome. Further, in any embodiment herein, the organism, cell, or GMO which can include the subunit-fused ACCase can be capable of reducing malonyl-COA. Further, the organism, cell, or GMO can comprise a polynucleotide encoding a protein that reduces or is capable of reducing malonly-CoA. In any embodiment herein, the protein that reduces or is capable of reducing malonyl-CoA can be NADPH dependent, NADH dependent, or a combination of these.

In any embodiment herein, the organism or GMO can produce 3-HP, and can comprise an exogenous nucleic acid molecule encoding a protein that is or can be comprised in an ACCase, wherein the encoded protein comprises at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, or at least 30 contiguous amino acid residues from any one of SEQ ID NOs: 1, 3, 5, 7, 9, or 11.

In any embodiment herein, the organism or GMO can produce 3-HP, and can comprise an exogenous nucleic acid molecule encoding a protein that is or can be comprised in an ACCase complex, wherein the encoded protein comprises at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, or at least 30 contiguous amino acid residues from any one of SEQ ID NOs: 1, 3, 5, 7, 9, or 11.

In any embodiment herein, the organism or GMO can produce 3-HP, and can comprise an exogenous nucleic acid molecule encoding a protein that is or can be comprised in an ACCase domain, wherein the encoded protein comprises at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, or at least 30 contiguous amino acid residues from any one of SEQ ID NOs: 1, 3, 5, 7, 9, or 11.

In any embodiment herein, the organism or GMO can produce 3-HP, and can comprise an exogenous nucleic acid molecule encoding a protein that is or can be comprised in an ACCase subunit, wherein the encoded protein comprises at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, or at least 30 contiguous amino acid residues from any one of SEQ ID NDs: 1, 3, 5, 7, 9, or 11.

In any embodiment herein, the organism or GMO can produce 3-HP, and can comprise an exogenous nucleic acid molecule comprising at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, or at least 30 contiguous polynucleotide residues from any one of SEQ ID NDs: 2, 4, 6, 8, or 10.

In any embodiment herein, the organism or GMO can produce 3-HP, and can comprise an exogenous nucleic acid molecule comprising at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, or at least 30 contiguous polynucleotide residues from any one of SEQ ID NDs: 2, 4, 6, 8, or 10, wherein the polynucleotide encodes a protein that is or can be comprised in an ACCase.

In any embodiment herein, the organism or GMO can produce 3-HP, and can comprise an exogenous nucleic acid molecule comprising at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, or at least 30 contiguous polynucleotide residues from any one of SEQ ID NDs: 2, 4, 6, 8, or 10, wherein the polynucleotide encodes a protein that is or can be comprised in an ACCase complex.

In any embodiment herein, the organism or GMO can produce 3-HP, and can comprise an exogenous nucleic acid molecule comprising at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, or at least 30 contiguous polynucleotide residues from any one of SEQ ID NOs: 2, 4, 6, 8, or 10, wherein the polynucleotide encodes a protein that is or can be comprised in an ACCase domain.

In any embodiment herein, the organism or GMO can produce 3-HP, and can comprise an exogenous nucleic acid molecule comprising at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, or at least 30 contiguous polynucleotide residues from any one of SEQ ID NDs: 2, 4, 6, 8, or 10, wherein the polynucleotide encodes a protein that is or can be comprised in an ACCase subunit.

In any embodiment herein, an accA-accD fused subunit can be introduced into an organism or GMO either alone or in combination with the accB-accC fused subunit, the accB gene, and/or the accC gene. In any embodiment herein, the organism or GMO can be a bacteria, *e.g., E. coli* or *Cupriavidus necator.*

### Composition stoichiometry

Composition stoichiometry can be, *e.g.,* the quantitative relationships among elements or parts that comprise a compound or thing *(e.g.,* such as an enzyme or enzyme complex, for example an ACCase or ACCase complex). Although stoichiometric terms are traditionally reserved for chemical compounds, theoretical consideration of stoichiometry are relevant when considering the optimal function of heterologous multi-subunit protein in a host cell.

A stoichiometric ratio of each of the four ACCase subunits relative to one another can be, for example, between or from: 0 and about 10, 0 and about 9, 0 and about 8, 0 and about 7, 0 and about 6, 0 and about 5, 0 and about 4, 0 and about 3, 0 and about 2, 0 and about 1, about 1 and about 10, about 2 and about 10, about 3 and about 10, about 4 and about 10, about 5 and about 10, about 6 and about 10, about 7 and about 10, about 8 and about 10, about 9 and about 10, or between or from about 0.5 to about 2, or about 7 to about 9. In an embodiment of the invention, the stoichiometric ratios of the protein subunits accA:accB:accC:accD are about: 1:2:1:1. In another embodimentof the invention, an organism is genetically modified to include an accA-accD fused subunit, an accB non-fused subunit, and an accC non-fused subunit, with the ratios or molar ratios of the accDA fusion:accB:accC being about 1:2:1, which can be close to the optimum for enzymatic activity.

In any embodiment herein, where an organism can be engineered to make 3-HP, in order to get optimal function in a host cell of a heterologous ACCase enzyme complex it can be important to engineer the stoichiometry of these subunits in such a way that provides maximal production of 3-HP such that the subunit can make a more stable enzyme complex when overexpressed in the cell.

In any embodiment herein can be provided for the controlled expression of the natural accA, accB, accC, and accD subunits of *E.coli* or having at least: 70%, 75%, 80%, 85%, 90%, 91%, 92%, 93%, 94%, 95%, 96%, 97%, 98%, or 99%, sequence homology to *E.coli* accA, accB, accC and accD. In any embodiment herein can be provided for the inducible expression of the natural accA, accB, accC, and accD subunits of *E.coli* or having at least 80% sequence homology to *E.coli* accA, accB, accC, and accD. In certain aspects the invention provides for the low, medium, high and/or inducible expression of the natural accA, accB, accC, and accD subunits of *E.coli* or having at least 80% sequence homology to *E.coli* accA, accB, accC and accD.

In any embodiment herein can be provided for the expression of the natural accC and accD subunits of *E.coli* or having at least 80% sequence homology to *E.coli* accA, accB, accC and accD in low, medium, high or inducible expression vectors. In any embodiment herein can be provided for the expression of the natural accB and accA subunits of *E.coli* or having at least 80% sequence homology to *E.coli* accA, accB, accC, and accD in low, medium, high or inducible expression vectors. In any embodiment herein can be provided for the expression of the natural accC and accD subunits with the accA subunit of *E.coli* or having at least 80% sequence homology to *E.coli* accA, accB, accC, and accD in low, medium, high or inducible expression vectors. In any embodiment herein can be provided for the expression of the natural accC and accD subunits with the accB subunit of *E.coli* or having at least about: 70%, 75%, 80%, 85%, 90%, 91%, 92%, 93%, 94%, 95%, 96%, 97%, 98%, or 99%, sequence homology to *E.coli* accA, accB, accC, and accD in low, medium, high or inducible expression vectors.

In any embodiment herein can be provided for the expression of a fusion of two, three, or all of the four ACCase subunits in one polypeptide in low, medium, high or inducible expression vectors. Such fusion may include any of the following combinations of the ACCase subunits: accA-accB, accA-accC, accA-accD, accB-accC, accB-accD, accC-accD, accA-accB-accC, accA-accB-accD, accA-accC-accD, accB-accC-accD, and accA-accB-accC-accD having at least 80% sequence homology to the same combinations of *E.coli* accA, accB, accC and accD or functional homologs thereof.

In any embodiment herein can be provided for an ACC complex in the stoichiometry of these subunits of the accCB and accDA in a 1:1, 1:2, 1:3, 1:4, 1:5, 1:6, 1:7, 1:8, 2:1, 2:3, 2:4, 2:5, 2:6, 2:7, 2:8, 3:1, 3:3, 3:4, 3:5, 3:6, 3:7, 3:8, 3:1, 3:3, 3:4, 3:5, 3:6, 3:7, 3:8, 4:1, 4:3, 4:4, 4:5, 4:6, 4:7, 4:8, 5:1, 5:3, 5:4, 5:5, 5:6, 5:7, 5:8, 6:1, 6:3, 6:4, 6:5, 6:6, 6:7, 6:8, 7:1, 7:3, 7:4, 7:5, 7:6, 7:7, 7:8, 8:1, 8:3, 8:4, 8:5, 8:6, 8:7, or 8:8 in low, medium, high or inducible expression vectors. In any embodiment herein can be provide for an ACC complex in the stoichiometry of these subunits of the accCB and accDA in about a 1:1, 1:2, 1:3, 1:4, 1:5, 1:6, 1:7, 1:8, 2:1, 2:3, 2:4, 2:5, 2:6, 2:7, 2:8, 3:1, 3:3, 3:4, 3:5, 3:6, 3:7, 3:8, 3:1, 3:3, 3:4, 3:5, 3:6, 3:7, 3:8, 4:1, 4:3, 4:4, 4:5, 4:6, 4:7, 4:8, 5:1, 5:3, 5:4, 5:5, 5:6, 5:7, 5:8, 6:1, 6:3, 6:4, 6:5, 6:6, 6:7, 6:8, 7:1, 7:3, 7:4, 7:5, 7:6, 7:7, 7:8, 8:1, 8:3, 8:4, 8:5, 8:6, 8:7, or 8:8 in low, medium, high or inducible expression vectors. In any embodiment herein can be provided for an ACC complex in the stoichiometry of these subunits of the accDA and accCB in a 1:1, 1:2, 1:3, 1:4, 1:5, 1:6, 1:7, 1:8, 2:1, 2:3, 2:4, 2:5, 2:6, 2:7, 2:8, 3:1, 3:3, 3:4, 3:5, 3:6, 3:7, 3:8, 3:1, 3:3, 3:4, 3:5, 3:6, 3:7, 3:8, 4:1, 4:3, 4:4, 4:5, 4:6, 4:7, 4:8, 5:1, 5:3, 5:4, 5:5, 5:6, 5:7, 5:8, 6:1, 6:3, 6:4, 6:5, 6:6, 6:7, 6:8, 7:1, 7:3, 7:4, 7:5, 7:6, 7:7, 7:8, 8:1, 8:3, 8:4, 8:5, 8:6, 8:7, or 8:8 in low, medium, high or inducible expression vectors. In any embodiment herein can be provided for an ACC complex in the stoichiometry of these subunits of the accDA and accCB in about a 1:1, 1:2, 1:3, 1:4, 1:5, 1:6, 1:7, 1:8, 2:1, 2:3, 2:4, 2:5, 2:6, 2:7, 2:8, 3:1, 3:3, 3:4, 3:5, 3:6, 3:7, 3:8, 3:1, 3:3, 3:4, 3:5, 3:6, 3:7, 3:8, 4:1, 4:3, 4:4, 4:5, 4:6, 4:7, 4:8, 5:1, 5:3, 5:4, 5:5, 5:6, 5:7, 5:8, 6:1, 6:3, 6:4, 6:5, 6:6, 6:7, 6:8, 7:1, 7:3, 7:4, 7:5, 7:6, 7:7, 7:8, 8:1, 8:3, 8:4, 8:5, 8:6, 8:7, or 8:8 in low, medium, high or inducible expression vectors.

In any embodiment herein can be provided for the stoichiometry of the accD-A subunits in a 1:1, 1:2, 1:3, 1:4, 1:5, 1:6, 1:7, 1:8, 2:1, 2:3, 2:4, 2:5, 2:6, 2:7, 2:8, 3:1, 3:3, 3:4, 3:5, 3:6, 3:7, 3:8, 3:1, 3:3, 3:4, 3:5, 3:6, 3:7, 3:8, 4:1, 4:3, 4:4, 4:5, 4:6, 4:7, 4:8, 5:1, 5:3, 5:4, 5:5, 5:6, 5:7, 5:8, 6:1, 6:3, 6:4, 6:5, 6:6, 6:7, 6:8, 7:1, 7:3, 7:4, 7:5, 7:6, 7:7, 7:8, 8:1, 8:3, 8:4, 8:5, 8:6, 8:7, or 8:8 in low, medium, high or inducible expression vectors. In any embodiment herein can be provided for the stoichiometry of the accD-A subunits in about a 1:1, 1:2, 1:3, 1:4, 1:5, 1:6, 1:7, 1:8, 2:1, 2:3, 2:4, 2:5, 2:6, 2:7, 2:8, 3:1, 3:3, 3:4, 3:5, 3:6, 3:7, 3:8, 3:1, 3:3, 3:4, 3:5, 3:6, 3:7, 3:8, 4:1, 4:3, 4:4, 4:5, 4:6, 4:7, 4:8, 5:1, 5:3, 5:4, 5:5, 5:6, 5:7, 5:8, 6:1, 6:3, 6:4, 6:5, 6:6, 6:7, 6:8, 7:1, 7:3, 7:4, 7:5, 7:6, 7:7, 7:8, 8:1, 8:3, 8:4, 8:5, 8:6, 8:7, or 8:8 in low, medium, high or inducible expression vectors. In any embodiment herein can be provided for the stoichiometry of the accC-B subunits in a 1:1, 1:2, 1:3, 1:4, 1:5, 1:6, 1:7, 1:8, 2:1, 2:3, 2:4, 2:5, 2:6, 2:7, 2:8, 3:1, 3:3, 3:4, 3:5, 3:6, 3:7, 3:8, 3:1, 3:3, 3:4, 3:5, 3:6, 3:7, 3:8, 4:1, 4:3, 4:4, 4:5, 4:6, 4:7, 4:8, 5:1, 5:3, 5:4, 5:5, 5:6, 5:7, 5:8, 6:1, 6:3, 6:4, 6:5, 6:6, 6:7, 6:8, 7:1, 7:3, 7:4, 7:5, 7:6, 7:7, 7:8, 8:1, 8:3, 8:4, 8:5, 8:6, 8:7, or 8:8 in low, medium, high or inducible expression vectors. In any embodiment herein can be provided for the stoichiometry of the accC-B subunits in about a 1:1, 1:2, 1:3, 1:4, 1:5, 1:6, 1:7, 1:8, 2:1, 2:3, 2:4, 2:5, 2:6, 2:7, 2:8, 3:1, 3:3, 3:4, 3:5, 3:6, 3:7, 3:8, 3:1, 3:3, 3:4, 3:5, 3:6, 3:7, 3:8, 4:1, 4:3, 4:4, 4:5, 4:6, 4:7, 4:8, 5:1, 5:3, 5:4, 5:5, 5:6, 5:7, 5:8, 6:1, 6:3, 6:4, 6:5, 6:6, 6:7, 6:8, 7:1, 7:3, 7:4, 7:5, 7:6, 7:7, 7:8, 8:1, 8:3, 8:4, 8:5, 8:6, 8:7, or 8:8 in low, medium, high or inducible expression vectors. In any embodiment herein can be provided for the stoichiometry of the accC-A subunits in a 1:1, 1:2, 1:3, 1:4, 1:5, 1:6, 1:7, 1:8, 2:1, 2:3, 2:4, 2:5, 2:6, 2:7, 2:8, 3:1, 3:3, 3:4, 3:5, 3:6, 3:7, 3:8, 3:1, 3:3, 3:4, 3:5, 3:6, 3:7, 3:8, 4:1, 4:3, 4:4, 4:5, 4:6, 4:7, 4:8, 5:1, 5:3, 5:4, 5:5, 5:6, 5:7, 5:8, 6:1, 6:3, 6:4, 6:5, 6:6, 6:7, 6:8, 7:1, 7:3, 7:4, 7:5, 7:6, 7:7, 7:8, 8:1, 8:3, 8:4, 8:5, 8:6, 8:7, or 8:8 in low, medium, high or inducible expression vectors. In any embodiment herein can be provided for the stoichiometry of the accC-A subunits in about a 1:1, 1:2, 1:3, 1:4, 1:5, 1:6, 1:7, 1:8, 2:1, 2:3, 2:4, 2:5, 2:6, 2:7, 2:8, 3:1, 3:3, 3:4, 3:5, 3:6, 3:7, 3:8, 3:1, 3:3, 3:4, 3:5, 3:6, 3:7, 3:8, 4:1, 4:3, 4:4, 4:5, 4:6, 4:7, 4:8, 5:1, 5:3, 5:4, 5:5, 5:6, 5:7, 5:8, 6:1, 6:3, 6:4, 6:5, 6:6, 6:7, 6:8, 7:1, 7:3, 7:4, 7:5, 7:6, 7:7, 7:8, 8:1, 8:3, 8:4, 8:5, 8:6, 8:7, or 8:8 in low, medium, high or inducible expression vectors. In any embodiment herein can be provided for the stoichiometry of the accC-B subunits in a 1:1, 1:2, 1:3, 1:4, 1:5, 1:6, 1:7, 1:8, 2:1, 2:3, 2:4, 2:5, 2:6, 2:7, 2:8, 3:1, 3:3, 3:4, 3:5, 3:6, 3:7, 3:8, 3:1, 3:3, 3:4, 3:5, 3:6, 3:7, 3:8, 4:1, 4:3, 4:4, 4:5, 4:6, 4:7, 4:8, 5:1, 5:3, 5:4, 5:5, 5:6, 5:7, 5:8, 6:1, 6:3, 6:4, 6:5, 6:6, 6:7, 6:8, 7:1, 7:3, 7:4, 7:5, 7:6, 7:7, 7:8, 8:1, 8:3, 8:4, 8:5, 8:6, 8:7, or 8:8 in low, medium, high or inducible expression vectors. In any embodiment herein can be provided for the stoichiometry of the accC-B subunits in about a 1:1, 1:2, 1:3, 1:4, 1:5, 1:6, 1:7, 1:8, 2:1, 2:3, 2:4, 2:5, 2:6, 2:7, 2:8, 3:1, 3:3, 3:4, 3:5, 3:6, 3:7, 3:8, 3:1, 3:3, 3:4, 3:5, 3:6, 3:7, 3:8, 4:1, 4:3, 4:4, 4:5, 4:6, 4:7, 4:8, 5:1, 5:3, 5:4, 5:5, 5:6, 5:7, 5:8, 6:1, 6:3, 6:4, 6:5, 6:6, 6:7, 6:8, 7:1, 7:3, 7:4, 7:5, 7:6, 7:7, 7:8, 8:1, 8:3, 8:4, 8:5, 8:6, 8:7, or 8:8 in low, medium, high or inducible expression vectors.

### Bioproduction Methods

In any inventive embodiment herein, the host cell can be genetically modified for increased malonyl-CoA flux by at least one heterologous ACCase complex, such as **Table 4** to further increase chemical bio-production in host cell.

### III. CONVERSION OF MALONYL-COA TO MALONATE SEMIALDEHYDE

One of the steps in the biosynthesis of 3-HP involves the conversion of malonyl-CoA (MCA) to malonate semialdehyde (MSA) and the conversion of malonate semialdehyde (MSA) to 3-HP (*see, e.g*., WO2011/038364). Inventive embodiments herein contemplate the use of novel enzymes and/or combinations of enzymes to catalyze the reaction in a microorganism from MCA to MSA, which results in enhanced cellular bioproduction of 3-HP in the host cell.

In some embodiments, the disclosure provides novel enzyme compositions or co-expression of a combinations of enzyme compositions to catalyze the conversion of malonyl-CoA to 3-HP. A general overview of the enzymes and the relevant reaction pathways methods are shown in **FIG.1****.** Any and all combinations of the relevant enzymes shown in **FIG.1** are contemplated in thisdisclosure.

In one embodiment, malonyl-CoA is converted to malonate semialdehyde by a malonyl-CoA reductase and malonate semialdehyde is converted to 3-HP through separate or both alternative pathways.

In another embodiment, malonyl-CoA is converted to malonate semialdehyde by a monofunctional malonyl-CoA reductase that catalyzes the malonyl-CoA conversion, but does not catalyze the malonate semialdehyde conversion.

In one embodiment, the microorganism herein comprises a monofunctional malonyl-CoA reductase derived from, *e.g., Sulfolobus tokodaii* (stMCR) (SEQ ID NOs. 15 and 16); a functional homolog of stMCR; an enzyme with at least 70%, 75%, 80%, 85%, 90%, 91%, 92%, 93%, 94%, 95%, 96%, 97%, 98%, or 99%, sequence homology/identity thereto.

In some embodiments, the microorganism herein comprises a bi-functional malonyl-CoA reductase having at least two protein fragments, with one fragment having malonyl-CoA reductase activity and the other fragment having malonate semialdehyde dehydrogenase activity derived from, *e.g., Chloroflexus aurantiacus* (caMCR).

### MCR-Dehydrogenase Enzymes for Conversion of 3-HP ions

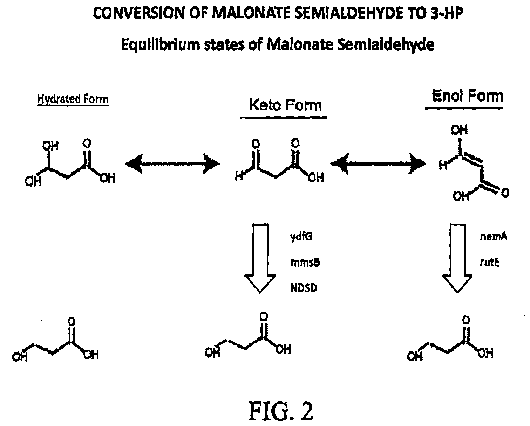

Following the conversion of the malonyl-CoA to malonate semialdehyde, the malonate semialdehyde is converted to 3-HP through separate or both of pathways. Malonate semialdehyde may exist in at least three states; the keto form, the enol form, and hydrate form, as shown in **FIG. 2****.** Malonate semialdehyde in the enol form, which will stabilize this form when compared to other aldehydes where the enol form is highly unfavored in the equilibrium among the three forms.

The malonate semialdehyde keto form can be converted to 3-HP utilizing a 3-hydroxy acid dehydrogenase enzyme (*e.g., ydfG*; SEQ ID NOs. 21 and 22), a 3-hydroxyisobutyrate dehydrogenase enzyme (*e.g., Pseudomonas aeruginosa mmsB;* SEQ ID NOs. 23 and 24), and/or NAD+-dependent serine dehydrogenase (*e.g., Pseudomonas NDSD*; SEQ ID NOs. 25 and 26). In an embodiment, *Pseudomonas mmsB, Pseudomonas NDSD,* and/or *E. coli ydfG* are used. The gene, *ydfG* from *E. coli* can be largely NADPH dependent, whereas *mmsB* and *NDSD* from *Pseudomonas* can utilize either NADPH or NADH.

The malonate semialdehyde enol form can be converted to 3-HP utilizing an N-ethylmaleimide reductase (*e.g., nemA*; SEQ ID NOs. 17 and 18), and/or a malonic semialdehyde reductase (*e.g., rutE*, SEQ ID NOs. 19 and 20) from *E. coli.* These enzymes do not directly utilize NADPH or NADH. Instead, these enzymes utilize a flavin mononucleotide that is cycled between oxidized and reduced states by NADPH or NADH. The enol pathway also has advantages over the keto pathway in that the equilibrium between the malonate semialdehyde enol form and 3-HP significantly favors the formation of 3-HP. Thus, the reaction may be considered less reversible, and/or essentially irreversible (*e.g*., drives the reaction toward the formation of 3-HP).

The malonate semialdehyde hydrated form may also be converted to 3-HP by either 3-HP dehydrogenase or malonate semialdehyde reductase enzymes, although the hydrated form is more likely to be converted to the enol form as the equilibrium continuously re-adjusts.

In one embodiment, the microorganism comprises a polynucleotide encoding: (1) a monofunctional malonyl-CoA reductase gene capable of catalyzing the conversion of malonyl-CoA to malonate semialdehyde; and (2) one or more genes encoding for at least one enzymes selected from the group consisting of: *ydfG, mmsB, NDSD, rutE*, and *nemA* or a functional homolog or a homolog with at least 70%, 75%, 80%, 85%, 90%, 91%, 92%, 93%, 94%, 95%, 96%, 97%, 98%, or 99%, sequence homology/identity .

In accordance with another aspect of the disclosure, there is provided an organism that is genetically modified to make 3-HP, wherein the genetic modification includes a polynucleotide encoding: (1) a monofunctional malonyl-CoA reductase gene capable of catalyzing the conversion of malonyl-CoA to malonate semialdehyde; (2) one or more genes encoding one or more enzymes capable of converting malonate semialdehyde keto form to 3-HP; and/or (3) one or more genes encoding one or more enzymes capable of converting either the malonate semialdehyde enol form or the malonate semialdehyde hydrated form to 3-HP.

In certain aspects, the disclosure provides a monofunctional malonyl-CoA reductase enzyme fused to a dehydrogenase enzyme that is either: (a) NADPH-dependent, (b) NADH-independent, (c) has the capability of using NADPH, (d) flavin-dependent, (e), flavin-independent, (f) has the capability of using flavin, (g) less susceptible to 3-HP inhibition at high concentration, and/or (h) catalyzes a reaction pathway to 3-HP that is substantially irreversible.

In certain aspects the disclosure also provides a monofunctional malonyl-CoA reductase enzyme fused to a dehydrogenase enzyme that is NADPH-dependent or has the capability of using NADPH.

Suitable 3-HP dehydrogenase enzymes that are largely NADH-dependent that can be used with the claimed invention include, but are not limited to, *mmsB* or *NDSD.* Suitable malonate reductase enzymes that are flavin-dependent include, but are not limited to, *rutE* and *nemA.* Suitable 3-HP dehydrogenase enzymes that are less susceptible 3-HP inhibition at high concentration that can be used with the claimed invention include, but are not limited to, *ydfG* and *NDSD.* Suitable 3-HP dehydrogenase or malonate semialdehyde dehydrogenase enzymes that catalyze a reaction pathway to 3-HP that is substantially irreversible are *rutE* and *nemA.*

In certain aspects the disclosure provides a monofunctional malonyl-CoA reductase enzyme fused to one or more dehydrogenase enzymes. Malonate semialdehyde, which is the intermediate product in the conversion of malonyl-CoA to 3-HP can be very reactive. Therefore, it can be advantageous to have a reaction pathway wherein the residence time of malonate semialdehyde within the cell is minimized, *e.g*., its conversion to 3-HP occurs quickly. By fusing the malonyl-CoA reductase with the malonate semialdehyde dehydrogenase to create a multi-domain protein (*e.g*., two domain protein) and having the MCR and dehydrogenase domains adjacent in the sequence, the the malonate semialdehyde can be quickly converted to 3-HP.

In certain aspects, the disclosure provides a first monofunctional malonyl-CoA reductase enzyme fused to a first dehydrogenase enzyme of one type and second monofunctional malonyl-CoA reductase enzyme fused to a dehydrogenase enzyme of a different type than the first dehydrogenase enzyme. Suitable different dehydrogenase enzymes include, but are not limited to, enzymes that use as a substrate, different forms of malonate semialdehyde.

In some embodiments, the disclosure provides for microorganisms comprising a genetic modification that include, but are not limited to, a malonyl-CoA reductase from S. *tokadaii* fused to *ydfG, mmsB, NDSD, rutE*, and/or *nemA* (or some combination thereof). The fused enzyme may include any of the following configurations: *mcr* - *ydfG, mcr* - *mmsB, mcr - NDSD, mcr* - *rutE*, *mcr* - *nemA, mcr* - *ydfG* - *mmsB, mcr* - *ydfG* - *NDSD, mcr* - *ydfG* - *rutE*, *mcr* - *ydfG* - *nemA, mcr* - *mmsB* - *ydfG, mcr* - *mmsB* - *NDSD, mcr* - *mmsB* - *rutE*, *mcr* - *mmsB* - *nemA, mcr* - *NDSD* - *ydfG, mcr* - *NDSD* - *mmsB, mcr* - *NDSD* - *rutE*, *mcr* - *NDSD* - *nemA, mcr* - *rutE - ydfG, mcr* - *rutE* - *mmsB, mcr* - *rutE* - *NDSD, mcr* - *rutE* - *nemA, mcr* - *nemA* - *ydfG, mcr* - *nemA - mmsB, mcr* - *nemA* - *NDSD, mcr* - *nemA* - *rutE*, functional homologs, and/or homologs with 70%, 75%, 80%, 85%, 90%, 91%, 92%, 93%, 94%, 95%, 96%, 97%, 98%, or 99%, sequence homology/identity.

In certain aspects the disclosure provides for microorganisms comprising a genetic modification that include, but are not limited to, malonyl-CoA reductase from C. *aggregans* fused to *ydfG, mmsB, NDSD, rutE*, and/or *nemA* (or some combination thereof). The fused enzyme may include any of the following configurations: *mcr* - *ydfG, mcr* - *mmsB, mcr* - *NDSD, mcr* - *rutE*, *mcr* - nemA, mcr - ydfG - mmsB, mcr - *ydfG* - *NDSD, mcr* - *ydfG* - *rutE*, *mcr* - *ydfG - nemA, mcr* - *mmsB* - *ydfG, mcr* - *mmsB* - *NDSD, mcr* - *mmsB* - *rutE*, *mcr* - *mmsB* - *nemA, mcr* - *NDSD* - *ydfG, mcr* - *NDSD* - *mmsB, mcr* - *NDSD* - *rutE*, *mcr* - *NDSD* - *nemA, mcr* - *rutE* - *ydfG, mcr* - *rutE* - *mmsB, mcr* - *rutE* - *NDSD, mcr* - *rutE* - *nemA, mcr* - *nemA* - *ydfG, mcr* - *nemA - mmsB, mcr* - *nemA* - *NDSD, mcr* - *nemA* - *rutE*, functional homologs, and/or homologs with 70%, 75%, 80%, 85%, 90%, 91%, 92%, 93%, 94%, 95%, 96%, 97%, 98%, or 99%, sequence homology/identity thereof.

In certain aspects the disclosure provides for microorganisms comprising a genetic modification that include, but are not limited to, a malonyl-CoA reductase from *O*. *trichoides* fused to *ydfG, mmsB, NDSD, rutE*, and/or *nemA* (or some combination thereof). The fused enzyme may include, but are not limited to, any of the following configurations: *mcr* - *ydfG, mcr - mmsB, mcr* - *NDSD, mcr* - *rutE*, *mcr* - *nemA, mcr* - *ydfG* - *mmsB, mcr* - *ydfG* - *NDSD, mcr* - *ydfG* - *rutE*, *mcr* - *ydfG* - *nemA, mcr* - *mmsB* - *ydfG, mcr* - *mmsB* - *NDSD, mcr* - *mmsB* - *rutE*, *mcr* - *mmsB* - *nemA, mcr* - *NDSD* - *ydfG, mcr* - *NDSD* - *mmsB, mcr* - *NDSD* - *rutE*, *mcr* - *NDSD - nemA, mcr* - *rutE* - *ydfG, mcr* - *rutE* - *mmsB, mcr* - *rutE* - *NDSD, mcr* - *rutE* - *nemA, mcr* - *nemA* - *ydfG, mcr* - *nemA* - *mmsB, mcr* - *nemA* - *NDSD, mcr* - *nemA* - *rutE*, functional homologs, and/or homologs with 70%, 75%, 80%, 85%, 90%, 91%, 92%, 93%, 94%, 95%, 96%, 97%, 98%, or 99%, sequence homology/identity.

### Enhanced Mutated Monofunctional MCR for Bioproduction

In certain aspects, the disclosure provides for microorganisms comprising a genetic modification having a mutated form of *stMCR* that has enhanced activity at about 0 °C to about 10°C, at about 8°C to about 15°C, at about 12°C to about 21°C, at 20°C to about 44°C, at about 30°C to about 37°C, at about 32°C to about 38°C, at about 35°C to about 42°C, at about 40°C to about 50°C, at about 50°C to about 60°C, and/or at about 59°C to less than equal to about 72°C. Such mutated forms may be designed based on the crystal structure for *stMCR; see* Demmer et al., J. Biol. Chem. 288:6363-6370, 2013.

The carboxylase domains of the malonyl-CoA reductase derived from *Chloroflexus aggregans* and/or *Oscillochloris trichoides* can be enhanced by mutations in the carboxylase binding domain to provide increased 3-HP production over the natural occurring enzyme.

The carboxylase activity of the malonyl-CoA reductase derived from *Chloroflexus aurantiacus* can be enhanced to increase activity. In certain aspects the disclosure provides for a mutated form of *Chloroflexus aurantiacus* malonyl-CoA reductase having a mutated carboxylase domain to provide increased 3-HP production over the natural occurring enzyme.

In certain aspects the disclosure provides for microorganisms comprising a genetic modification that include carboxylase domains of the malonyl-CoA reductase derived from C. *aggregans* fused to *ydfG, mmsB, NDSD, rutE*, and/or *nemA* (or some combination thereof). It is contemplated that the any of the enhanced MCR by mutation, as provide above, may be fused in any of the following configurations including, but not limited to, *mcr* - *ydfG, mcr* - *mmsB, mcr* - *NDSD, mcr* - *rutE*, *mcr* - *nemA, mcr* - *ydfG* - *mmsB, mcr* - *ydfG* - *NDSD, mcr* - *ydfG* - *rutE*, *mcr* - *ydfG* - *nemA, mcr* - *mmsB* - *ydfG, mcr* - *mmsB* - *NDSD, mcr* - *mmsB* - *rutE*, *mcr* - *mmsB* - *nemA, mcr* - *NDSD* - *ydfG, mcr* - *NDSD* - *mmsB, mcr* - *NDSD* - *rutE*, *mcr* - *NDSD* - *nemA, mcr* - *rutE - ydfG, mcr* - *rutE* - *mmsB, mcr* - *rutE* - *NDSD, mcr* - *rutE* - *nemA, mcr* - *nemA* - ydfG, *mcr* - *nemA* - *mmsB,* mcr - *nemA* - *NDSD, mcr* - *nemA* - *rutE*, functional homologs, and/or homologs with 70%, 75%, 80%, 85%, 90%, 91%, 92%, 93%, 94%, 95%, 96%, 97%, 98%, or 99%, sequence homology/identity.

### IV. SALT-TOLERANT ENZYMES

The growth of engineered microorganisms for enhanced production of a chemical product, such as *E. coli* can be inhibited by high salt concentrations, *e.g.,* when the chemical product accumulates when the organism produces large amounts of the chemical product.

Dose-dependent studies with increasing amounts of NaCl and Na-3-HP show that salt can have inhibitory effects on ACCase activity which can be essential to fatty acid biosynthesis of membranes required for growth and propagation and for the production of 3-HP (see **EXAMPLE 1)**. Thus, the use of salt-tolerant enzymes in 3-HP production should increase 3-HP production in a host cell.

### A. Enzymes from Halophilic Organisms

Halophiles are characterized as organisms with a great affinity for salt. In some embodiments, a halophilic organism can require at least 0.01M, 0.05M, 0.1M, 0.2M, 0.3M, 0.4M, 0.5M, 0.6M, 0.7M, 0.8M, 0.9M, 1.0M, 1.25M, or 1.5M concentrations of salt (NaCl) for growth. Halophiles can live in hypersaline environments that are generally defined occurring to their salt concentration of their habitats. Halophilic organisms that are defined as having "slight salt affinity" can grow optimally at 2-5% NaCl, moderate halophiles can grow optimally at 5-20% NaCl, and extreme halophiles can grow optimally at 20-30% NaCl.

In an embodiment, a genetically engineered microorganism can be made to express homologous enzymes of a specific halophile, for example, from a moderate halophile or an extreme halophile.

In an embodiment, microorganisms can comprise a genetic modification that includes or codes for enzymes from slight halophile organisms. In an embodiment, microorganisms can comprise a genetic modification that includes or codes for enzymes from moderate halophile organisms. In an embodiment, microorganisms can comprise a genetic modification that includes or codes for enzymes from extreme halophile organisms.

Homology with genes may be determined by analysis with BLASTN version 2.0 provided through the NCBI website. Homology with proteins may be determined by analysis with BLASTP version 2.2.2 provided through the NCBI website. These programs can align disclosed fragments being compared and can determine the extent of identity or similarity between them.

To date there are many sequenced halophilic organisms that can be used with the claimed disclosure. Examples of some sequenced halophilic organisms include, but are not limited to, *Chromohalobacter salexigens, Flexistipes sinusarabici strain (MAS10T), Halobacterium sp. NRC-1, Haloarcula marismortui, Natronomonas pharaonis, Haloquadratum walsbyi, Haloferax volcanii, Halorubrum lacusprofundi, Halobacterium sp. R-1, Halomicrobium mukohataei, Halorhabdus utahensis, Halogeometricum borinquense, Haloterrigena turkmenica, Natronobacterium gregoryi SP2, Halalkalicoccus jeotgali, Natrialba magadii, Haloarcula hispanica, Halopiger xanaduensis, Halophilic archaeon DL31, Haloferax mediterranei, Halovivax ruber, Natronococcus gregoryi,* and *Natronococcus occultus.*

Examples of suitable moderate halophilic organisms in which homologous enzymes of the disclosurecan be derived from include, but are not limited to, eukaryotes such as crustaceans (*e.g., Artemia salina*)*,* insects (*e.g., Ephydra hians*), certain plants from the genera *Salicornia spp,* algae (*e.g., Dunaliella viridis*), fungi, and protozoa (*e.g., Fabrea salina*)*,* phototrophic organisms, such as planktonic and microbial mat-formers cyanobacteria as well as other anaerobic red and green sulphur bacteria from the genera *Ectothiorhodospira spp.*) and non-sulphur bacteria from the genera *Chromatium spp*.; gram-negative anaerobic bacteria, for example from the genera *Haloanaerobacter spp.* some of which are methanogenic, for example from the genera *Methanohalophilus* spp. and either aerobic or facultative such as species from the genera *Halomonas, Chromohalobacter, Salinovibrio, Pseudomonas,* for example (*e.g., Halomonase elongate*); gram-positive bacteria from genera such as *Halobacillus, Bacillus, Marinococcus,* etc. as well as some actinomycetes, for example, *Actinopolyspora halophila.*

### Genomic and Proteomic Hallmarks of Halophilic Organisms

Comparative genomic and proteomic studies of halophiles and non-halophiles can reveal some common trends in the genomes and proteomes of halophiles. At the protein level, halophilic organisms can be characterized by low hydrophobicity, over-representation of acidic residues, especially Asp, under-representation of Cys, lower propensities for helix formation and higher propensities for coil structure.

At the DNA level, halophilic organisms can be characterized by the dinucleotide abundant profiles of halophilic genomes that bear some common characteristics, which are distinct from non-halophiles, and hence may be regarded as specific genomic signatures for salt-adaptation. The synonymous codon usage in halophiles may also exhibit similar patterns regardless of their long-term evolutionary history.

In one embodiment, microorganisms can be synthesized to comprise proteins that are modified for salt tolerance such that they have low hydrophobicity, over-representation of acidic residues, especially Asp, under-representation of Cys, lower propensities for helix formation and higher propensities for coil structure.

Suitable salt-tolerant enzymes can include enzymes from salt-tolerant organisms. Salt-tolerant organisms (such as, for example, halophiles) can include any living organisms that are adapted to living in conditions of high salinity. Suitable salt-tolerant enzymes can include enzymes from salt-tolerant organisms that are homologs of the following enzymes: Sucrose-6-phosphate hydrolase (cscA from *E. coli),* glucose-6-phosphate isomerase (pgi from *E. coli),* fructokinase (cscK *from E. coli),* fructose-1,6-bisphosphatase (yggF *from E. coli),* fructose 1,6-bisphosphatase (ybhA *from E. coli),* fructose 1,6-bisphosphatase II (glpX *from E. coli),* fructose-1,6-bisphosphatase monomer (fbp *from E. coli),* 6-phosphofructokinase-1 monomer (pfkA from *E. coli),* 6-phosphofructokinase-2 monomer (pfkB *from E. coli),* fructose bisphosphate aldolase monomer (fbaB *from E. coli),* fructose bisphosphate aldolase monomer (fbaA *from E. coli),* triose phosphate isomerase monomer (tpiA), glyceraldehyde 3-phosphate dehydrogenase-A monomer (gapA *from E. coli),* phosphoglycerate kinase (pgk), 2,3-bisphosphoglycerate-independent phosphoglycerate mutase (gpmM from *E. coli),* 2,3-bisphosphoglycerate-dependent or tdcE (from *E. coli),* phosphoglycerate mutase (gpmA), enolase (eno *from E. coli),* phosphoenolpyruvate carboxylase (ppc from *E. coli),* malate dehydrogenase (mdh), fumarase A (fum from *E. coli),* fumarase B (fumB), fumarase C (fumC from *E. coli),* phosphoenolpyruvate synthetase (ppsA from *E. coli),* pyruvate kinase I monomer (pykF from *E. coli),* pyruvate kinase II monomer (pykA from *E. coli),* fumarate reductase (frdABCD from *E. coli),* lipoamide dehydrogenase (lpd from *E. coli),* pyruvate dehydrogenase (aceE from *E. coli),* pyruvate dehydrogenase (aceF from *E. coli),* pyruvate formate-lyase (pflB from *E. coli),* acetyl-CoA carboxylase (accABCD from *E. coli),* malonyl CoA reductase (mcr), 3HP dehydrogenase (mmsB, NDSD, or ydfG), malonate semialdehyde reductase (nemA, rutE from *E. coli),* or a combination thereof.

Suitable salt-tolerant enzyme homologs that can be used with the claimed disclosurecan have at least or at least about 99%, 98%, 97%, 96%, 95%, 94%, 93%, 92%, 91%, 90%, 89%, 88%, 87%, 86%, 85%, 84%, 83%, 82%, 81%, 80%, 79%, 78%, 77%, 76%, 75%, 74%, 73%, 72%, 71%, 70% overall amino acid or nucleotide identity and/or homology to the above enzymes. Suitable salt-tolerant enzyme homologs that can be used with the claimed disclosurecan have at least or at least about 99%, 98%, 97%, 96%, 95%, 94%, 93%, 92%, 91%, 90%, 89%, 88%, 87%, 86%, 85%, 84%, 83%, 82%, 81%, 80%, 79%, 78%, 77%, 76%, 75%, 74%, 73%, 72%, 71%, 70% amino acid or nucleotide identity and/or homology to the essential protein function domains of the enzymes above. Suitable salt-tolerant enzyme homologs that can be used with the claimed disclosurecan have at least or at least 99%, 98%, 97%, 96%, 95%, 94%, 93%, 92%, 91%, 90%, 89%, 88%, 87%, 86%, 85%, 84%, 83%, 82%, 81%, 80%, 79%, 78%, 77%, 76%, 75%, 74%, 73%, 72%, 71%, 70% overall amino acid or nucleotide identity and/or homology to the essential binding amino acids within an essential protein function domain of the enzymes above.

In an embodiment, suitable salt-tolerant enzyme homologs can be enzymes from one of the following organisms: *Halomonas elongata, Salinibacter rubu*r*,* or *Halobacterium* species (Archaea).

In an embodiment, there can be provided a non-salt-tolerant organism that can be genetically modified to make 3-HP, wherein the genetic modification can include introducing into the organism a polynucleotide encoding for an acetyl-CoA carboxylase from a salt-tolerant organism. In an embodiment, the acetyl-CoA carboxylase subunits accA, accB, accC and accD can be from *Halomonas elongata.*

### V. CHEMICAL TRANSPORTER

In one aspect of this disclosure, any of the microorganisms may be genetically modified to introduce a nucleic acid sequence encoding a polypeptide that: (1) facilitates the exportation of the chemical of interest or the export of an inhibitory chemical from the cell; and/or (2) facilitates the importation into the cell, a reactant, precursor, and/or metabolite used in the organism's production pathway for producing the chemical of interest.

### 3-HP Exporter

In one aspect of this disclosure, 3-HP can be produced using a genetically modified *E. coli* organism. Thus, the present disclosurecontemplates a host cell genetically modified to express or increase expression of an exporter that can function to transfer 3HP from the cellular environment extracellularly.

Bacterial cells, such as *E. coli,* have at least five different types of exporters: the major facilitator superfamily (MFS); the ATP-binding cassette superfamily (ABC); the small multidrug resistance family (SMR); the resistance-nodulation-cell division superfamily (RND); and the multi antimicrobial extrusion protein family (MATE). In addition, amino acid exporters, which are common to almost all host cells, may be made to export 3-HP. Additionally, solvent tolerant transporters, for example, bromoacetate, butanol, isobutanol, and the like, may be used to export 3-HP.

In certain aspects the disclosureprovides a host cell with a recombinant exporter wherein the exporter is an MFS exporter, ABC exporter, SMR exporter, RND exporter, MATE exporter, amino acid exporter, solvent tolerance transporter, or a combination thereof.

Suitable exporters that can be used include, but are not limited to, *aerD, bcr, cusA, dedA, eamA, eamB, eamH, emaA, emaB, emrB, emrD, emrKY, emrY, garP, gudP, hsrA, leuE, mdlB, mdtD, mdtG, mdtL,* mdtM, *mhpT, rhtA, rhtB, rhtC, thtB, yahN, yajR, ybbP, ybiF, ybjJ, ycaP, ydcO, yddG, ydeD, ydgE, yddG, ydhC, ydhP, ydiN, ydiM, ydjE, ydjI, ydjK, yeaS, yedA, yeeO, yegH, yggA, yfcJ, yfiK, yhjE, yidE, yigK, yigJ, yijE, yjiI, yjiJ, yjiO, ykgH, ypjD, ytfF, ytfL,* or functional homolog or homolog having at least or at least about 99%, 98%, 97%, 96%, 95%, 94%, 93%, 92%, 91%, 90%, 89%, 88%, 87%, 86%, 85%, 84%, 83%, 82%, 81%, 80%, 79%, 78%, 77%, 76%, 75%, 74%, 73%, 72%, 71%, 70% sequence identity and/or homology. Other potential transporter proteins may be identified using topology analysis as illustrated in [Daley et al., Science 308: 1321-1323, 2005].

In certain aspects the disclosurecan provide the exporter to be improved for binding to 3-HP. In certain aspects the disclosurecan provide the exporters named to be further enhance by genetic modification of the predicted cytoplasmic domain to increase 3-HP binding. In certain aspects the disclosurecan provide the exporter to be improved for binding to 3-HP. In certain aspects the invention can provide the exporters named to be further enhance by genetic modification of the predicted transmembrane binding domain to increase 3-HP binding or incorporation into the host cell membrane.

Suitable exporter homologs that can be used with the claimed disclosurecan have at least or about at least 99%, 98%, 97%, 96%, 95%, 94%, 93%, 92%, 91%, 90%, 89%, 88%, 87%, 86%, 85%, 84%, 83%, 82%, 81%, 80%, 79%, 78%, 77%, 76%, 75%, 74%, 73%, 72%, 71%, 70% overall amino acid or nucleotide identity and/or homology to the previously mentioned exporters. Suitable exporter homologs that can be used with the claimed disclosurecan have at least or at least about 99%, 98%, 97%, 96%, 95%, 94%, 93%, 92%, 91%, 90%, 89%, 88%, 87%, 86%, 85%, 84%, 83%, 82%, 81%, 80%, 79%, 78%, 77%, 76%, 75%, 74%, 73%, 72%, 71%, 70% amino acid or nucleotide identity and/or homology to the essential protein function domains of the exporters above. Suitable exporter homologs that can be used with the claimed disclosurecan have at least or at least about 99%, 98%, 97%, 96%, 95%, 94%, 93%, 92%, 91%, 90%, 89%, 88%, 87%, 86%, 85%, 84%, 83%, 82%, 81%, 80%, 79%, 78%, 77%, 76%, 75%, 74%, 73%, 72%, 71%, 70% amino acid or nucleotide identity and/or homology to the essential binding amino acids within an essential exporter domain of the enzymes above.

In certain aspects the disclosurecan provide for at least one of the exporters provided herein to be expressed in a host cell to increase the chemical production of 3-HP. In certain aspects the disclosurecan provide for at least one of the exporters provided herein to be expressed in a host cell and with a genetic modification of *tig* to increase the chemical production of 3-HP.

In certain aspects the disclosurecan provide for one exporter to be further modified by one or more genetic modulates so that the expression level and timing of expression of the exporter can be controlled in the host cell. In certain aspects the invention can provide for one exporter to be further modified by an inducible promoter, RBS, high, multicopy plasmid or combination thereof.

In certain aspects the disclosurecan provide exporters to be expressed in a host cell in an equal ratio. In certain aspects the disclosurecan provide exporters to be expressed in a host cell in a 1:2 ratio. In certain aspects the invention can provide exporters to be expressed in a host cell in a 1:3 ratio. In certain aspects the disclosurecan provide exporters to be expressed in a host cell in a 1:4 ratio. In certain aspects the disclosurecan provide exporters to be expressed in a host cell in a 2:3 ratio.

In certain aspects the disclosurecan provide for the exporter to maintain the host cell at pH 7.0-7.4 during the continuous production phase. In certain aspects the disclosureprovides for an exporter and a base importer, wherein the cell can maintain a pH 7.0-7.4 during the continuous production phase. In certain aspects the disclosurecan provide for the exporter to maintain the host cell at pH 3.0 to pH 4.0, pH 4.0 to pH 5.0, pH 5.0 to pH 6.0, pH 6.0 to pH 7.0, pH 7.0 to pH 8.0, pH 8.0 to pH 9.0, pH 9.0 to pH 10.0, and/or pH 7.0-7.3 during the continuous production phase. In certain aspects the disclosureprovides for an exporter and base importer, wherein the cell can maintain a pH 3.0 to pH 4.0, pH 4.0 to pH 5.0, pH 5.0 to pH 6.0, pH 6.0 to pH 7.0, pH 7.0 to pH 8.0, pH 8.0 to pH 9.0, pH 9.0 to pH 10.0, and/or pH 7.0-7.3 during the continuous production phase.

In some embodiments, additional modifications to the host cell may be made to further enhance the transporter's function. In particular, deletion of the tig gene from the genome of the host cell may enhance expression and total activity of integral membrane proteins such as exporters and importers.

### Bicarbonate Importer

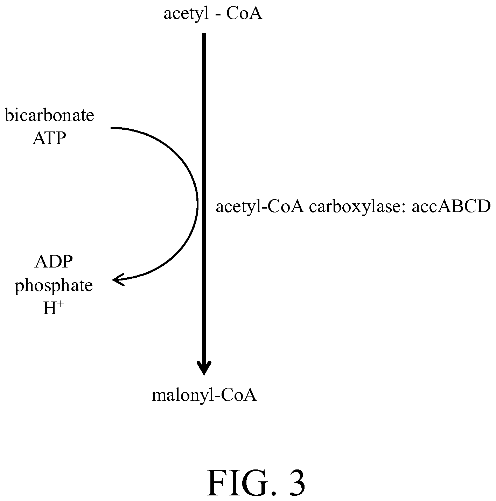

One of the steps in the conversion of biomass to 3-HP can be the conversion of acetyl-CoA to malonyl-CoA, which is illustrated in **FIG. 3****.**

As shown in **FIG. 3**, this reaction can be catalyzed by the acetyl-CoA carboxylase, and bicarbonate is a reactant that can drive the reaction. One of the primary sources of bicarbonate to drive this reaction can be carbon dioxide within the cell. Carbon dioxide can readily diffuse through a cell's membrane, and a natural equilibrium will be reached between the intracellular and extracellular carbon dioxide. As a cell produces carbon dioxide it can migrate through the cell, and since it tends not to be very soluble in the media, it may bubble out of the system and more intracellular carbon dioxide may migrate out of the cell to maintain the equilibrium. This process can impede the production of 3-HP since bicarbonate (which is in equilibrium with the dissolved carbon dioxide in the form of carbonic acid) can be needed to drive the acetyl-CoA → malonyl-CoA reaction, and the intracellular carbon dioxide can be the primary source for intracellular bicarbonate.

In some embodiments , an organism can be provided that can include a heterologous gene encoding for a polypeptide that can act as a carbon dioxide importer (*e.g*., it enhances the importation of carbon dioxide into the cell or inhibits the exportation of carbon dioxide from the cell), which can result in increased intracellular carbon dioxide. Use of a CO₂ importer can mitigate against the natural outflow of carbon dioxide.

In some embodiments, there can be provided an organism that can be genetically modified, wherein the genetic modification can include a polynucleotide encoding a polypeptide that is capable of importing extracellular carbon dioxide from the media to within the cell membrane or inhibiting the exportation of intracellular carbon dioxide from within the cell membrane to the media. In some embodiments, a microorganism can be genetically modified to encode one or more of the following heterologous genes: *bicA* from *Synechococcus* species, *ychM* gene product of *E. coli, yidE* gene product of *E. coli,* any of the bicarbonate transporters as described in [Felce and Saier, J. Mol. Microbiol. Biotechnol. 8: 169-176, 2004] or any amino acid sequences homologous thereof (*e.g.,* at least or at least about 99%, 98%, 97%, 96%, 95%, 94%, 93%, 92%, 91%, 90%, 89%, 88%, 87%, 86%, 85%, 84%, 83%, 82%, 81%, 80%, 79%, 78%, 77%, 76%, 75%, 74%, 73%, 72%, 71%, 70% homologous and/or identical to the amino acid or nucleotide sequences of the CO2 importer/exporters described herein].

### Bioproduction Methods

In some embodiments, the host cell can be genetically modified by at least one heterologous gene and/or salt tolerant heterologous gene of **FIG. 1** or **Table 1** and at least one 3-HP exporter provided herein to further increase chemical bioproduction in a host cell.

In some embodiments, the host cell can be genetically modified with a heterologous gene for increased malonyl-CoA flux, 3-HP export, and to increase chemical bio-production. In some embodiments, the host cell can be genetically modified to increase malonyl-CoA flux, 3-HP export to increase chemical bioproduction.

### VI. MULTI- PHASE FERMENTATION

In accordance with another aspect of the present disclosure, there is provided a method of producing a chemical product from a carbon source through a bioproduction process that comprises a controlled multi-phase production process. The multi-phase production process includes an *e.g.,* an initiation phase and a production phase, wherein the production phase and/or process can be controlled by genetic modifications to the organism producing the chemical product and/or can be controlled by changes made to the cell environment.

In one aspect, the bioproduction process may include two or more of the following phases: (1) growth phase; (2) induction phase; and (3) production phase. During the growth phase, the organism replicates itself and the biocatalyst needed to produce the chemical product is built up. During the induction phase, expression of key enzymes critical to the production of the chemical is induced and the enzymes accumulate within the biocatalyst to carry out the reactions required to produce the product. During the production phase organism produces the desired chemical product.

The initiation and/or completion of the growth, induction and/or production phases can be controlled. In accordance with the present disclosure, the growth phase can be dependent on the presence of a critical external reactant that will initiate growth. The initiation and completion of the growth phase can be controlled by the addition and amount of the initiating reactant added to the reaction vessel.

In accordance with certain embodiments, the chemical product can be 3-HP and the production organism can be *E. coli* or yeast. The critical external reactant may be phosphate, which may be needed for replication of *E. coli* cells. In one embodiment, the growth phase can be initiated by the addition of phosphate to a reaction vessel (together with a carbon source such as sugar and the *E. coli* cells), and the duration of the growth phase can be controlled by the amount of phosphate added to the system.

The induction phase can be controlled by genetic modifications to the producing organism. The key enzymes triggered during this phase can be engineered into the organism using promoters that can be sensitive to (*e.g*., activated by) the depletion of the initiating reactant. As a result, once the initiating reactant is depleted, the growth phase ends, the key enzymes are activated and the induction phase begins.

In another embodiment, the induction phase can be controlled by key genes that encode for enzymes in the biosynthetic pathway for the product into the production organism using promoters that are activated by phosphate depletion. In one embodiment where the chemical product is 3-HP and the production organism is *E. coli,* the key genetic modifications may include one or more of the following: mcr, mmsB, ydfG, rutE, nemA and NDSD; genes that encode individual or fused subunits of ACCase, such as accA, accB, accC, accD, accDA fusion, and accCB fusion, and the promoters may include one or more of the promoters that direct expression of the following *E. coli* genes: amn, tktB, xasA, yibD, ytfK, pstS, phoH, phnC, or other phosphate-regulated genes as described in Baek and Lee, FEMS Microbiol Lett 264: 104-109, 2006. In accordance with this embodiment, once the phosphate is depleted, expression of the key enzymes can be activated by their promoters and the induction phase begins.

The production phase may also be controlled by genetic modifications. For example, the organism can be engineered to included mutated forms of enzymes critical to the initiation of production of the chemical product. These initiation enzymes may facilitate initiation of production either by: (1) becoming active and serving a key function in the production pathway; and/or (2) becoming inactive and/or disrupted thereby turning off a branch pathway or other competitive pathway that prevents or limits the production pathway leading to the chemical product. In accordance with an embodiment, initiation enzymes can be mutated to form temperature sensitive variants of the enzymes that can be either activated by or deactivated at certain temperatures. As a result, the production phase can be initiated by changing the changing the temperature within the reaction vessel.

In one embodiment, the production phase can be controlled by genetically modifying the microorganism with a heterologous nucleotide sequence encoding one or more of the following temperature sensitive enzymes: fabI^{ts} (SEQ ID NO. 27), fabB^{ts} (SEQ ID NO.28) and fabD^{ts} (SEQ ID NO. 29). These enzymes can be deactivated or shut-off or altered to have decreased activity at the desired temperature for production of the chemical product. These enzymes can play a key role shuttling carbon atoms into the fatty acid synthesis pathway. Although fatty acid synthesis pathway can be critical during the growth phase, it can inhibit production of the chemical product. Once the reaction vessel temperature is changed, the temperature sensitive enzymes are deactivated and the fatty acid synthesis pathway shuts down thereby allowing the production pathway of the chemical product to ramp up.

In accordance with the present disclosure, the growth phase can last be between 10 to 40 hours, or about 15 to about 35 hours, or about 20 to about 30 hours. The induction phase may be for about 1 to about 6 hours, about 1 to about 5 hours, or about 2 to about 4 hours. The production phase may be greater than about: 5, 10, 15, 20, 25, 30, 35, 40, 45, 50, 55, 60, 65, 70, 75, 80, 85, 90, 95, or 100 hours depending on the amount of chemical product that is desired.

In accordance with the present disclosure, the growth phase and induction phase can be conducted at a temperature of about 25°C to about 35°C, about 28°C to about 32°C, or about 30 °C. The production phase can be conducted at a temperature of about 35°C to about 45°C, about 35°C to about 40°C, or about 36°C to about 38 °C. The production phase temperature can be higher than the induction phase temperature, and the increase in temperature that initiates the production phase can occur over a period of about 1 to about 5 hours, about 1 to about 3 hours, about 2 hours, or about 1 hour.

In accordance with the present disclosure, there is provided a method of producing a chemical product from a renewable carbon source through a bioproduction process comprising:
(1) constructing a genetically modified organism capable of converting said renewable carbon source to said chemical product, wherein said genetically modified organism requires inorganic phosphate for growth and comprises: (a) at least one heterologous gene whose expression is regulated by a promoter sensitive to inorganic phosphate levels within a culture system, wherein said gene provides a critical function in converting said carbon source to said chemical product and is not required for the genetically modified organism to replicate; and (b) a gene encoding a temperature-sensitive enzyme;
(2) forming a culture system comprising said carbon source in an aqueous medium and said genetically modified microorganism;
(3) maintaining the culture system under conditions that allow the genetically modified microorganism to replicate comprising maintaining a sufficient level of inorganic phosphate within said culture system;
(4) allowing the inorganic phosphate to deplete thereby triggering the expression of the gene regulated by a promoter sensitive to inorganic phosphate levels; and
(5) changing the temperature of the culture system thereby activating or deactivating said temperature-sensitive enzyme and initiating the production of said chemical product.

In accordance with the present disclosure, there is provided a method of producing 3-hydropropionic acid (3-HP) from a renewable carbon source, comprising:
(1) constructing a genetically modified organism capable of converting said renewable carbon source to 3-HP, wherein said genetically modified organism requires inorganic phosphate for growth and comprises: (a) at least one heterologous gene whose expression is regulated by a promoter sensitive to inorganic phosphate levels within a culture system, wherein said gene is selected from the group consisting of mcr, mmsB, ydfG, rutE, nemA, NDSD, accA, accB, accC, accD, accDA fusion, and accCB fusion; and (b) a gene encoding a temperature-sensitive enzyme selected from the group consisting of fabI, fabB, fabD, and combinations thereof;
(2) forming a culture system comprising said carbon source in an aqueous medium, phosphate and said genetically modified microorganism, and thereby initiating a growth phase during which the genetically modified microorganism replicates;
(3) maintaining a sufficient level of inorganic phosphate within said culture system until the desired level of cell growth is achieved;
(4) allowing the inorganic phosphate to deplete thereby initiating an induction phase which begins the expression of said gene regulated by a promoter sensitive to inorganic phosphate levels; and
(5) changing the temperature of the culture system thereby activating or deactivating said temperature-sensitive enzyme and initiating a growth phase during which said genetically modified microorganism produces 3-HP.

### Fermentation Conditions

Depending on the host cell fermentation may be performed under aerobic, microaerobic, or anaerobic conditions, with or without agitation. The operation of culture systems to achieve aerobic, microaerobic and anaerobic conditions are well known to those of ordinary skill in the art.

Suitable pH ranges for fermentation depend on the multiple factors such as the host cell. In some applications of the invention fermentation can occur between various pH ranges for example, pH 3.0 to pH 4.0, pH 4.0 to pH 5.0, pH 5.0 to pH 6.0, pH 6.0 to pH 7.0, pH 7.0 to pH 8.0, pH 8.0 to pH 9.0, or pH 9.0 to pH 10.0. However, the actual pH conditions for a particular application are not meant to be limited by these ranges and can be between the expressed pH ranges if it provides more optimal production of the fermentation process, such as increased 3-HP production.

### VII. GENES AND PROTEINS FOR THE BIOPRODUCTION OF CHEMICALS

An overview of the engineered pathways provided by the disclosurein a host cell is shown in **FIG.1****.** Various combinations of the pathways shown can be carried out by various combinations of genetic modifications to key enzymes either in the intrinsic pathways or supplied through the transformation of a heterologous gene.

In some applications of the genetically modified microorganism of the invention may comprise a single genetic modification, or one or more genetic modifications. Various types of genetic modifications that can be used with the disclosure are disclosed herein.

In some embodiments the genetic modified organism of the invention can comprise a genetic modification to the following gene/proteins or a homolog with at least 70%, 75%, 80%, 85%, 90%, 91%, 92%, 93%, 94%, 95%, 96%, 97%, 98%, or 99%, sequence homolog/identity to or a functional homolog of: bifunctional malonyl-CoA reductase (MCR from *Chloroflexus aurantiacus*), monofunctional malonyl-CoA reductase (caMCR from *Chloroflexus aurantiacus*), malonyl-CoA reductase (stMCR from *Sulfolobus tokodaii.*), Enzyme: malonyl-CoA reductase (cgMCR from *Chloroflexus aggregans*), Enzyme: malonyl-CoA reductase (otMCR from *Oscillochloris trichoides),* Polypeptide: host restriction; endonuclease R (hsdR *from E. coli),* lactose metabolism (lac from *E. coli),* L-rhamnulose kinase (rhaB from *E. coli),* rhamnulose-1-phosphate aldolase (rhaD *from E. coli),* Enzyme: β-galactosidase (lacZ *from E. coli),* L-ribulose 5-phosphate 4-epimerase (araD *from E. coli),* L-ribulokinase (araB *from E. coli),* Enzyme: D-lactate dehydrogenase - fermentative (ldhA from *E. coli),* enzyme: pyruvate formate-lyase (pflB *from E. coli),* Enzyme: phosphate acetyltransferase / phosphate propionyltransferase (pta from *E. coli),* Enzyme: pyruvate oxidase (poxB *from E. coli),* Enzyme: methylglyoxal synthase (mgsA from *E. coli),* enzyme: Acetate kinase (ackA from *E. coli),* enzymes: phosphotransacetylase-acetate kinase (pta-ack from *E. coli),* Enzyme: enoyl-[acyl-carrier-protein] reductase (fabI from *E. coli),* Protein: zeocin binding protein (zeoR from *Streptoalloteichus Hindustanus*), Enzyme: carboxytransferase moiety of acetyl-CoA carboxylase (accAD from *E. coli),* Enzyme: triose phosphate isomerase (tpiA *from E. coli),* Enzyme: biotoin carboxylase moiety of acetyl-CoA carboxylase (accBC *from E. coli),* Enzyme: transhydrogenase (pntAB *from E. coli),* Polypeptide: LacI DNA-binding transcriptional repressor (lacI from *E. coli),* Enzyme: β-ketoacyl-ACP synthases I (fabB *from E. coli),* Enzyme: β-ketoacyl-ACP synthases II (fabF *from E. coli),* Enzyme: malonyl-CoA-ACP transacylase (fabD *from E. coli),* Enzyme: pantothenate kinase (coaA from *E. coli*)*,* Enzyme: pyruvate dehydrogenase complex (aceEF from *E. coli*)*,* Enzyme: 3-hydroxyisobutyrate/3-HP dehydrogenase (mmsB from *Pseudomonas aeruginosa*)*,* Enzyme: lipoamide dehydrogenase (lpd from *E. coli),* Enzyme: γ-glutamyl-γ-aminobutyraldehyde dehydrogenase (puuC from *E. coli),* Enzyme: malate synthase A (aceB from *E. coli),* Enzyme: isocitrate lyase (aceA from *E. coli),* Enzyme: isocitrate dehydrogenase phosphatase/kinase (aceK from *E. coli),* Enzyme: 3-hydroxy acid dehydrogenase (ydfG from *E. coli),* Enzyme: acetyl CoA carboxylase (accADBC from *E. coli),* Polypeptide: predicted transcriptional regulator (yieP from *E. coli),* Blastocyin resistance gene (BSD from *Schizosaccharomyces pombe*)*,* Enzyme: pyridine nucleotide transhydrogenase (udha from *E. coli),* Protein: Cra DNA-binding transcriptional dual regulator (fruR from *E. coli),* (SCB from *E. coli),* enzyme: aldehyde dehydrogenase B (aldB from *E. coli),* Enzyme: carbonic anhydrase (cynT from *E. coli),* Enzyme: cyanase (cynS *from E. coli),* DNA gyrase toxin-antitoxin system (ccdAB *from E. coli),* Enzyme: phosphoglycerate mutase (pgi from *E. coli),* ArcA transcriptional dual regulator or Aerobic respiration control (arcA *from E. coli),* Enzyme: 6-phosphofructokinase (pfk *from E. coli),* Enzyme: glyceraldehyde 3-phosphate dehydrogenase-A complex (gapA *from E. coli),* aldehyde dehydrogenase A (alda from *E. coli),* Enzyme: glutamate dehydrogenase (gdhA *from E. coli),* Enzyme: NADH-dependent serine dehydrogenase (NDSD from *Pseudomonas aeruginosa*)*,* Protein: threonine/homoserine efflux transporter (rhtA from *E. coli),* Enzyme: glyceraldehyde 3-phosphate dehydrogenase (gapN *from E. coli),* Phosphotransferase system (pts from *E. coli),* Enzyme: 6-phosphofructokinase II (pfkB from *E. coli),* Enzyme: methylmalonate-semialdehyde dehydrogenase (mmsA from *Pseudomonas aeruginosa*), Oxaloacetate:beta-alanine aminotransferase (OAT-1 from *Bacillus cereus*), Enzyme: aspartate 1-decarboxylase (panD *from E. coli),* Gene that confers resistance to valine (ValR from *E. coli),* Enzyme: glucokinase (glk *from E. coli),* Polypeptide: 30 S ribosomal sununit protein S12 (rpsL from *E. coli),* Polypeptide: CynR DNA-binding transcriptional repressor (cynR from *E. coli),* Transporter: galactose:H+ symporter (galP *from E. coli),* aspartate aminotransferase (aspC from *E. coli),* Enzyme: alpha-ketoglutarate reductase (serA from *E. coli),* Enzyme: 6-phosphofructokinase I (pfkA from *E. coli),* Enzyme: phosphoenolpyruvate carboxylase (ppc *from E. coli),* Enzyme: succinate-semialdehyde dehydrogenase (NADP+) (gabD *from E. coli),* Enzyme: pyruvate kinase (pyk *from E. coli),* Enzyme: oxaloacetate 4-decarboxylase (OAD from *Leuconostoc mesenteroides*)*,* Enzyme: trigger factor; a molecular chaperone involved in cell division (tig *from E. coli),* Transcription Unit (ptsHIcrr from *E. coli),* Enzyme: acetyl-CoA acetaldehyde dehydrogenase/alcohol dehydrogenase (adhE from *E. coli),* Enzyme: fattyacyl thioesterase I (tesA from *E. coli*), Enzyme: guanosine 3'-diphosphate 5'-triphosphate 3'-diphosphatase (spoT from *E. coli),* combination of genes encoding accABCD subunits (from *E. coli* and *Halomonas elongata*), pol (from *E. coli),* Enzyme: GDP pyrophosphokinase / GTP pyrophosphokinase (relA from *E. coli),* [Enzyme Name] (me *from E. coli),* Enzyme: citrate synthase (gltA *from E. coli),* Polypeptide: DNA gyrase, subunit A (gyrA *from E. coli),* Enzyme: multifunctional 2-keto-3-deoxygluconate 6-phosphate aldolase and 2-keto-4-hydroxyglutarate aldolase and oxaloacetate decarboxylase (eda from *E. coli),* thiamin biosynthesis (thi from *E. coli),* Polypeptide: acetolactate synthase II (ilvG *from E. coli),* acetyl CoA carboxylase (accDACB *from E. coli),* Citrate synthase (ArCS from *Arthrobacter aurescens*), Acetyl-CoA carboxylase from Corynebacter glutamicum (CgACC from *Corynebacter glutamicum*), Polypeptide: ferrichrome / phage / antibiotic outer membrane porin FhuA (fhuA *from E. coli),* Transporter: phosphate:H+ symporter PitA (pitA from *E. coli),* Transporter: uracil:H+ symporter (uraA from *E. coli),* Enzyme: uracil phosphoribosyltransferase (upp from *E. coli),* Enzyme: acylphosphatase (yccX from *E. coli),* acetyl-CoA synthetase (acsA *from E. coli),* Polypeptide: restriction of methylated adenine (mrr from *E. coli),* Protein: TrpR transcriptional repressor (trpR *from E. coli),* Enzymes: glutamate 5-semialdehyde dehydrogenase / gamma-glutamyl kinase (proAB from *E. coli),* methylcytosine restriction system (mcrBC from *E. coli),* Protein: citrate lyase, citrate-ACP transferase component (citF from *E. coli),* Enzyme: thioesterase II (tesB from *E. coli),* Enzyme: DNA-specific endonuclease I (endA *from E. coli),* Enzyme: phosphate acetyltransferase (eutD from *E. coli),* Enzyme: propionate kinase (tdcD from *E. coli),* tRNA: tRNA glnV (supE from *E. coli*)*,* Enzyme: DNA-binding, ATP-dependent protease La (Ion from *E. coli),* Polypeptide: DNA strand exchange and recombination protein with protease and nuclease activity (recA from *E. coli),* Transcription Unit: restriction endonulease component of EcoKI restriction-modification system (hsdRMS from *E. coli),* Enzyme: restriction of DNA at 5-methylcytosine residues (mcrA from *E. coli)* araD (from *E. coli),* araB (from *E. coli),* rhaD (from *E. coli),* rhaB (from *E. coli),* ack (from *E. coli),* fruR (from *E. coli),* gapA (from *E. coli),* lacI (from *E. coli),* lacZ (from *E. coli),* ldhA (from *E. coli),* mgsA (from *E. coli),* pfkA (from *E. coli),* pflB (from *E. coli),* pgi (from *E. coli),* poxB (from *E. coli),* pta-ack (from *E. coli),* ptsH (from *E. coli),* glut1 (from *E. coli)* and/ or ack (from *E. coli)* or any combination thereof.

The use of genetic modifications in genetic elements, genes, proteins or the use of compounds, such as siRNA technology, anti-sense technology, and small molecule inhibitors supplied to the host cell that modulate the expression of gene and proteins provided by the present disclosure are also contemplated.

In some embodiments the genetic modified organism of the disclosureuses genetic elements such as siRNA or equivalent (including but not limited to shRNA, RNAi, miRNA, etc.), genes, proteins or compounds supplied to the host cell to modulate one or more of the following: bifunctional malonyl-CoA reductase (MCR from *Chloroflexus aurantiacus*), monofunctional malonyl-CoA reductase (caMCR from *Chloroflexus aurantiacus*),malonyl-CoA reductase (stMCR from *Sulfolobus tokodaii*.), Enzyme: malonyl-CoA reductase (cgMCR from *Chloroflexus aggregans*), Enzyme: malonyl-CoA reductase (otMCR from *Oscillochloris trichoides),* Polypeptide: host restriction; endonuclease R (hsdR from *E. coli),* lactose metabolism (lac from *E. coli),* L-rhamnulose kinase (rhaB from *E. coli),* rhamnulose-1-phosphate aldolase (rhaD *from E. coli),* Enzyme: β-galactosidase (lacZ *from E. coli),* L-ribulose 5-phosphate 4-epimerase (araD *from E. coli),* L-ribulokinase (araB *from E. coli),* Enzyme: D-lactate dehydrogenase - fermentative (ldhA from *E. coli),* enzyme: pyruvate formate-lyase (pflB *from E. coli),* Enzyme: phosphate acetyltransferase / phosphate propionyltransferase (pta from *E. coli),* Enzyme: pyruvate oxidase (poxB *from E. coli),* Enzyme: methylglyoxal synthase (mgsA from *E. coli),* enzyme: Acetate kinase (ackA from *E. coli),* enzymes: phosphotransacetylase-acetate kinase (pta-ack from *E. coli),* Enzyme: enoyl-[acyl-carrier-protein] reductase (fabI from *E. coli),* Protein: zeocin binding protein (zeoR from *Streptoalloteichus Hindustanus*), Enzyme: carboxytransferase moiety of acetyl-CoA carboxylase (accAD from *E. coli),* Enzyme: triose phosphate isomerase (tpiA *from E. coli),* Enzyme: biotoin carboxylase moiety of acetyl-CoA carboxylase (accBC *from E. coli),* Enzyme: transhydrogenase (pntAB from *E. coli),* Polypeptide: LacI DNA-binding transcriptional repressor (lacI *from E. coli),* Enzyme: β-ketoacyl-ACP synthases I (fabB *from E. coli),* Enzyme: β-ketoacyl-ACP synthases II (fabF *from E. coli),* Enzyme: malonyl-CoA-ACP transacylase (fabD *from E. coli),* Enzyme: pantothenate kinase (coaA from *E. coli),* Enzyme: pyruvate dehydrogenase complex (aceEF *from E. coli),* Enzyme: 3-hydroxyisobutyrate/3-HP dehydrogenase (mmsB from *Pseudomonas aeruginosa*), Enzyme: lipoamide dehydrogenase (lpd from *E. coli),* Enzyme: γ-glutamyl-γ-aminobutyraldehyde dehydrogenase (puuC from *E. coli),* Enzyme: malate synthase A (aceB from *E. coli*), Enzyme: isocitrate lyase (aceA from *E. coli*), Enzyme: isocitrate dehydrogenase phosphatase/kinase (aceK from *E. coli),* Enzyme: 3-hydroxy acid dehydrogenase (ydfG from *E. coli),* Enzyme: acetyl CoA carboxylase (accADBC from *E. coli),* Polypeptide: predicted transcriptional regulator (yieP from *E. coli),* Blastocyin resistance gene (BSD from *Schizosaccharomyces pombe*), Enzyme: pyridine nucleotide transhydrogenase (udha from *E. coli),* Protein: Cra DNA-binding transcriptional dual regulator (fruR from *E. coli),* (SCB from *E. coli),* enzyme: aldehyde dehydrogenase B (aldB from *E. coli*), Enzyme: carbonic anhydrase (cynT from *E. coli),* Enzyme: cyanase (cynS *from E. coli),* DNA gyrase toxin-antitoxin system (ccdAB *from E. coli),* Enzyme: phosphoglycerate mutase (pgi from *E. coli*), ArcA transcriptional dual regulator or Aerobic respiration control (arcA *from E. coli),* Enzyme: 6-phosphofructokinase (pfk *from E. coli),* Enzyme: glyceraldehyde 3-phosphate dehydrogenase-A complex (gapA *from E. coli),* aldehyde dehydrogenase A (alda from *E. coli),* Enzyme: glutamate dehydrogenase (gdhA *from E. coli),* Enzyme: NADH-dependent serine dehydrogenase (NDSD from *Pseudomonas aeruginosa*), Protein: threonine/homoserine efflux transporter (rhtA from *E. coli),* Enzyme: glyceraldehyde 3-phosphate dehydrogenase (gapN from *E. coli),* Phosphotransferase system (pts from *E. coli),* Enzyme: 6-phosphofructokinase II (pfkB from *E. coli),* Enzyme: methylmalonate-semialdehyde dehydrogenase (mmsA from *Pseudomonas aeruginosa*), Oxaloacetate:beta-alanine aminotransferase (OAT-1 from *Bacillus cereus*), Enzyme: aspartate 1-decarboxylase (panD *from E. coli),* Gene that confers resistance to valine (ValR from *E. coli),* Enzyme: glucokinase (glk *from E. coli),* Polypeptide: 30 S ribosomal sununit protein S12 (rpsL from *E. coli),* Polypeptide: CynR DNA-binding transcriptional repressor (cynR from *E. coli),* Transporter: galactose:H+ symporter (galP *from E. coli),* aspartate aminotransferase (aspC from *E. coli),* Enzyme: alpha-ketoglutarate reductase (serA from *E. coli),* Enzyme: 6-phosphofructokinase I (pfkA from *E. coli*), Enzyme: phosphoenolpyruvate carboxylase (ppc *from E. coli),* Enzyme: succinate-semialdehyde dehydrogenase (NADP+) (gabD *from E. coli),* Enzyme: pyruvate kinase (pyk *from E. coli),* Enzyme: oxaloacetate 4-decarboxylase (OAD from *Leuconostoc mesenteroides*), Enzyme: trigger factor; a molecular chaperone involved in cell division (tig *from E. coli),* Transcription Unit (ptsHIcrr from *E. coli),* Enzyme: acetyl-CoA acetaldehyde dehydrogenase/alcohol dehydrogenase (adhE from *E. coli),* Enzyme: fattyacyl thioesterase I (tesA from *E. coli*), Enzyme: guanosine 3'-diphosphate 5'-triphosphate 3'-diphosphatase (spoT from *E. coli),* combination of genes encoding accABCD subunits (from *E. coli* and *Halomonas elongata*), pol (from *E. coli),* Enzyme: GDP pyrophosphokinase / GTP pyrophosphokinase (relA from *E. coli),* [Enzyme Name] (me *from E. coli),* Enzyme: citrate synthase (gltA *from E. coli),* Polypeptide: DNA gyrase, subunit A (gyrA *from E. coli),* Enzyme: multifunctional 2-keto-3-deoxygluconate 6-phosphate aldolase and 2-keto-4-hydroxyglutarate aldolase and oxaloacetate decarboxylase (eda from *E. coli),* thiamin biosynthesis (thi from *E. coli),* Polypeptide: acetolactate synthase II (ilvG *from E. coli),* acetyl CoA carboxylase (accDACB *from E. coli),* Citrate synthase (ArCS from *Arthrobacter aurescens*), Acetyl-CoA carboxylase from Corynebacter glutamicum (CgACC from *Corynebacter glutamicum*), Polypeptide: ferrichrome / phage / antibiotic outer membrane porin FhuA (fhuA *from E. coli),* Transporter: phosphate:H+ symporter PitA (pitA from *E. coli),* Transporter: uracil:H+ symporter (uraA from *E. coli),* Enzyme: uracil phosphoribosyltransferase (upp from *E. coli),* Enzyme: acylphosphatase (yccX from *E. coli*), acetyl-CoA synthetase (acsA *from E. coli),* Polypeptide: restriction of methylated adenine (mrr from *E. coli),* Protein: TrpR transcriptional repressor (trpR *from E. coli),* Enzymes: glutamate 5-semialdehyde dehydrogenase / gamma-glutamyl kinase (proAB from *E. coli),* methylcytosine restriction system (mcrBC from *E. coli),* Protein: citrate lyase, citrate-ACP transferase component (citF from *E. coli),* Enzyme: thioesterase II (tesB from *E. coli),* Enzyme: DNA-specific endonuclease I (endA *from E. coli),* Enzyme: phosphate acetyltransferase (eutD from *E. coli*), Enzyme: propionate kinase (tdcD from *E. coli),* tRNA: tRNA glnV (supE from *E. coli*), Enzyme: DNA-binding, ATP-dependent protease La (Ion from *E. coli),* Polypeptide: DNA strand exchange and recombination protein with protease and nuclease activity (recA from *E. coli),* Transcription Unit: restriction endonulease component of EcoKI restriction-modification system (hsdRMS from *E. coli),* Enzyme: restriction of DNA at 5-methylcytosine residues (mcrA from *E. coli).* In some embodiments the genetic modifications listed above are modified further with the genetic modules provided herein.

In some embodiments the genetic modification of the genes, proteins and enzymes of the disclosurecan be for the method of bioproduction of various chemicals which can be used to make various consumer products, including but not limited to those described herein.

In some embodiments the genetic modification of the genes, proteins and enzymes of the disclosurecan be for the bioproduction of 1,4-butanediol (1,4-BDO). *See, e.g.,* U.S. Pub. No. 20110190513. In some embodiments the genetic modification of the genes, proteins and enzymes of the disclosurecan be for the bioproduction of butanol. *See, e.g.,* U.S. App. No. 13/057,359. In some embodiments, the genetic modification of the genes, proteins and enzymes of the disclosurecan be for the bioproduction of isobutanol. *See, e.g.,* U.S. App. No. 13/057,359.

In some embodiments the genetic modification of the genes, proteins and enzymes of the disclosurecan be for the bioproduction of 3-HP such and its aldehyde metabolites. *See, e.g.,* U.S. App.No. 13/062,917.

In some embodiments the genetic modification of the genes, proteins and enzymes of the disclosurecan be for the bioproduction of polyketide chemical products. *See, e.g.,* U.S. App. No. 13/575,581.

In some embodiments the genetic modification of the genes, proteins and enzymes of the disclosurecan be for the bioproduction of fatty acid methyl esters. *See e.g.,* U.S. Pub. No. 20110124063. In some embodiments the genetic modification of the genes, proteins and enzymes of the invention can be for the bioproduction of C4-C18 fatty acids. *See, e.g.,* U.S. App No. 61/682,127.

### Genetic Modifications

Various methods to achieve such genetic modification in a host strain are known to one skilled in the art. Example of genetic modifications that can be used by the claimed disclosureinclude, but are not limited to, increasing expression of an endogenous genetic element; increasing expression of an exogenous genetic element; decreasing functionality of a repressor gene; increasing functionality of a repressor gene; increasing functionality of an activator gene; decreasing functionality of an activator gene; introducing a genetic change or element integrated in the host genome, introducing a heterologous genetic element permanently, by integration into the genome or transiently by transformation with plasmid; increasing copy number of a nucleic acid sequence encoding a polypeptide catalyzing an enzymatic conversion step; mutating a genetic element to provide a mutated protein to increase specific enzymatic activity; mutating a genetic element to provide a mutated protein to decrease specific enzymatic activity; over-expressing of a gene; reducing the expression of a gene; knocking out or deleting a gene; altering or modifying feedback inhibition; providing an enzyme variant comprising one or more of an impaired binding sites or active sites; increasing functionality of a siRNA or equivalent, decreasing functionality of a siRNA or equivalent, increasing functionality of an antisense molecule, decreasing functionality of an antisense molecule, addition of genetic modules such as RBS, '3 UTR elements to increase mRNA stability or translation; deletion of genetic modules such as RBS, '3 UTR elements to decrease mRNA stability or translation; addition or modification of genetic modules such as '5 UTR elements to increase transcription; deletion or modification of genetic modules such as '5 UTR and elements to increase transcription. In addition other genetic modules, provide herein, such a multicopy plasmids and various promoters can be used to further modify of the genetic modifications provide herein. Additionally, as known to those of ordinarily skill in the art compounds such as siRNA technology, anti-sense technology, and small molecule in inhibitors can be used to alter gene expression in the same manner as a genetic modification.

Screening methods, such as SCALE in combination with random mutagenesis may be practiced to provide genetic modifications that provide a benefit to increased production of 3-HP in a host cell. Examples of random mutagenesis can include insertions, deletions and substitutions of one or more nucleic acids in a nucleic acid of interest. In various embodiments, a genetic modification results in improved enzymatic specific activity and/or turnover number of an enzyme. Without being limited in any way, changes may be measured by one or more of the following: KM; Kcat, Kavidity, gene expression level, protein expression level, level of a product known to be produced by the enzyme, 3-HP tolerance, or by 3-HP production or by any other known means.

### Host Cells

In some applications of the disclosurethe host cell can be a gram-negative bacterium. In some applications of the disclosurethe host cell can be from *Zymomonas, Escherichia, Pseudomonas, Alcaligenes, or Klebsiella.* In some applications of the disclosurethe host cell can be *Escherichia coli, Cupriavidus necator, Oligotropha carboxidovorans,* or *Pseudomonas putida.* In some applications of the disclosurethe host cell is one or more *E. coli* strains.

In some applications of the disclosurethe host cell can be a gram-positive bacterium. In some applications of the disclosurethe host cell can be from *Clostridium, Salmonella, Rhodococcus, Bacillus, Lactobacillus, Enterococcus, Paenibacillus, Arthrobacter, Corynebacterium,* or *Brevibacterium.* In some applications of the disclosurethe host cell is *Bacillus licheniformis, Paenibacillus macerans, Rhodococcus erythropolis, Lactobacillus plantarum, Enterococcus faecium, Enterococcus gallinarium, Enterococcus faecalis,* or *Bacillus subtilis.* In some applications of the disclosurethe host cell is *B. subtilis* strain.

In some applications of the disclosurethe host cell can be yeast. In some applications of the disclosurethe host cell can be from *Pichia, Candida, Hansenula or Saccharomyces.* In some applications of the disclosurethe host cell is *Saccharomyces cerevisiae.* In some applications of the disclosurethe host cell is *Saccharomyces pombe.*

In some applications of the disclosurethe host cell can be an alga. In some applications of the disclosurethe host cell can be a halophile. In some applications of the disclosurethe host cell can be an alga. In some applications of the invention the host cell is a chemolithotrophic bacterium.

In some applications of the disclosurethe host cell can be comprised of multiple host cell types. In some applications of the disclosurethe host cell is comprised of one host cell type. In some applications of the disclosurethe host cell is comprised of one more species or strain of a host cell type.

### VIII. DOWNSTREAM CONSUMER PRODUCTS CHEMICALS

3-HP purified according to the methods provided in this disclosure may be converted to various other products having industrial uses including, but not limited to, acrylamide, acrylic acid, esters of acrylic acid, 1,3-propanediol, and other chemicals, collectively referred to as "downstream chemical products" or "downstream products." In some instances, the conversion can be associated with a separation and/or purification process. These downstream chemical products can be useful for producing a variety of consumer products which are described in more detail below. The methods of the present invention can include steps to produce downstream products of 3-HP.

As a C3 building block, 3-HP can offer the potential for a variety of chemical conversions into commercially important intermediates, industrial end products, and consumer products. For example, 3-HP may be converted to acrylic acid, acrylates (*e.g*., acrylic acid salts and esters), 1,3-propanediol, malonic acid, ethyl-3-hydroxypropionate, ethyl ethoxy propionate, propiolactone, acrylamide, or acrylonitrile.

Additionally, 3-HP may be oligomerized or polymerized to form poly(3-hydroxypropionate) homopolymers, or co-polymerized with one or more other monomers to form various co-polymers. Because 3-HP has a single stereoisomer, polymerization of 3-HP may not be complicated by the stereo-specificity of monomers during chain growth. This can be contrasted with (S)-2-hydroxypropanoic acid (also known as lactic acid), which has two (D, L) stereoisomers that should be considered during its polymerizations.

As will be further described, 3-HP can be converted into derivatives starting (i) substantially as the protonated form of 3-hydroxypropionic acid; (ii) substantially as the deprotonated form, 3-hydroxypropionate; or (iii) as mixtures of the protonated and deprotonated forms. Generally, the fraction of 3-HP present as the acid versus the salt can depend on the pH, the presence of other ionic species in solution, temperature (which can change the equilibrium constant relating the acid and salt forms), and, to some extent, pressure. Many chemical conversions may be carried out from either of the 3-HP forms, and overall process economics can likely dictate the form of 3-HP for downstream conversion.

Acrylic acid obtained from 3-HP purified by the methods described in this disclosure may be further converted to various polymers. For example, the free-radical polymerization of acrylic acid can take place by polymerization methods known to the skilled worker and can be carried out, for example, in an emulsion or suspension in aqueous solution or another solvent. Initiators, such as but not limited to organic peroxides, can be often added to aid in the polymerization. Among the classes of organic peroxides that may be used as initiators can be diacyls, peroxydicarbonates, monoperoxycarbonates, peroxyketals, peroxyesters, dialkyls, and hydroperoxides. Another class of initiators can be azo initiators, which may be used for acrylate polymerization as well as co-polymerization with other monomers. U.S. Patent Nos. 5,470,928; 5,510,307; 6,709,919; and 7,678,869 teach various approaches to polymerization using a number of initiators, including organic peroxides, azo compounds, and other chemical types.

Accordingly, it can be further possible for co-monomers, such as crosslinkers, to be present during the polymerization. The free-radical polymerization of the acrylic acid obtained from dehydration of 3-HP, as produced herein, in at least partly neutralized form and in the presence of crosslinkers can be practiced in certain embodiments. This polymerization may result in hydrogels which can then be comminuted, ground and, where appropriate, surface-modified, by known techniques.

An important commercial use of polyacrylic acid can be for superabsorbent polymers. *See e.g.,* Modern Superabsorbent Polymer Technology, Buchholz and Graham (Editors), WileyVCH, 1997. Superabsorbent polymers can be used as absorbents for water and aqueous solutions for diapers, adult incontinence products, feminine hygiene products, and similar consumer products. In such consumer products, superabsorbent materials can replace traditional absorbent materials such as cloth, cotton, paper wadding, and cellulose fiber. Superabsorbent polymers can absorb, and retain under a slight mechanical pressure, up to 25 times or more their weight in liquid. The swollen gel can hold the liquid in a solid, rubbery state and can prevent the liquid from leaking. Superabsorbent polymer particles can be surface-modified to produce a shell structure with the shell being more highly cross-linked than the rest of the particle. This technique can improve the balance of absorption, absorption under load, and resistance to gel-blocking. It is recognized that superabsorbent polymers can have uses in fields other than consumer products, including agriculture, horticulture, and medicine.

Superabsorbent polymers can be prepared from acrylic acid (such as acrylic acid derived from 3-HP provided herein) and a crosslinker, by solution or suspension polymerization. Exemplary methods include those provided in U.S. Patent Nos. 5,145,906; 5,350,799; 5,342,899; 4,857,610; 4,985,518; 4,708, 997; 5,180,798; 4,666,983; 4,734,478; and 5,331,059.

Among consumer products, a diaper, a feminine hygiene product, and an adult incontinence product can be made with superabsorbent polymer that itself can be made substantially from acrylic acid converted from 3-HP made in accordance with the present disclosure.

Diapers and other personal hygiene products may be produced that incorporate superabsorbent polymers that can be made from acrylic acid that can be made from 3-HP which can be produced and purified by the teachings of the present application. The following can provide general guidance for making a diaper that incorporates such superabsorbent polymer. The superabsorbent polymer first can be molded into an absorbent pad that may be vacuum formed, and in which other materials, such as a fibrous material (*e.g*., wood pulp) can be added. The absorbent pad then can be assembled with sheet(s) of fabric, generally a nonwoven fabric *(e.g.,* that can be made from one or more of nylon, polyester, polyethylene, and polypropylene plastics) to form diapers.

More particularly, in one non-limiting process, multiple pressurized nozzles, located above a conveyer belt, can spray superabsorbent polymer particles (*e.g*., about 400 micron size or larger), fibrous material, and/or a combination of these onto the conveyer belt at designated spaces/intervals. The conveyor belt can be perforated and under vacuum from below, so that the sprayed on materials can be pulled toward the belt surface to form a flat pad. In various embodiments, fibrous material can be applied first on the belt, followed by a mixture of fibrous material and the superabsorbent polymer particles, followed by fibrous material, so that the superabsorbent polymer can be concentrated in the middle of the pad. A leveling roller may be used toward the end of the belt path to yield pads of uniform thickness. Each pad thereafter may be further processed, such as to cut it to a proper shape for the diaper, or the pad may be in the form of a long roll sufficient for multiple diapers. Thereafter, the pad can be sandwiched between a top sheet and a bottom sheet of fabric (one generally being liquid pervious, the other liquid impervious), for example, on a conveyor belt, and these can be attached together, for example by gluing, heating or ultrasonic welding, and cut into diaper-sized units (if not previously so cut). Additional features may be provided, such as elastic components, strips of tape, etc., for fit and ease of wearing by a person.

The ratio of the fibrous material to polymer particles can affect performance characteristics. In some cases, this ratio can be between 75:25 and 90:10 (see *e.g.,* U.S. Patent No. 4,685,915). Other disposable absorbent articles may be constructed in a similar fashion, such as absorbent articles for adult incontinence, feminine hygiene (sanitary napkins), tampons, etc. (see, for example, U.S. Patent Nos. 5,009,653; 5,558,656; and 5,827,255.

Low molecular weight polyacrylic acid can have uses for water treatment, and as a flocculant and thickener for various applications including cosmetics and pharmaceutical preparations. For these applications, the polymer may be uncrosslinked or lightly cross-linked, depending on the specific application. The molecular weights can be typically from about 200 to about 1,000,000 g/mol. An example of the preparation of these low molecular weight polyacrylic acid polymers is described in U.S. Patent Nos. 3,904,685; 4,301,266; 2,798,053; and 5,093,472.

Acrylic acid may be co-polymerized with one or more other monomers selected from the group consisting of: acrylamide, 2-acrylamido-2-methylpropanesulfonic acid, N,N-dimethylacrylamide, N-isopropylacrylamide, methacrylic acid, and methacrylamide, to name a few. The relative reactivity of the monomers can affect the microstructure and thus the physical properties of the polymer. Co-monomers may be derived from 3-HP, or otherwise provided, to produce co-polymers. *See e.g.,* Ullmann's Encyclopedia of Industrial Chemistry, Polyacrylamides and Poly(Acrylic Acids), WileyVCH Verlag GmbH, Wienham (2005).

Acrylic acid can in principle be copolymerized with almost any free-radically polymerizable monomers including styrene, butadiene, acrylonitrile, acrylic esters, maleic acid, maleic anhydride, vinyl chloride, acrylamide, itaconic acid, and so on. End-use applications typically dictate the co-polymer composition, which can influences properties. Acrylic acid also may have a number of optional substitutions and, after such substitutions, may be used as a monomer for polymerization, or co-polymerization reactions. As a general rule, acrylic acid (or one of its co-polymerization monomers) may be substituted by any substituent that does not interfere with the polymerization process, such as alkyl, alkoxy, aryl, heteroaryl, benzyl, vinyl, allyl, hydroxy, epoxy, amide, ethers, esters, ketones, maleimides, succinimides, sulfoxides, glycidyl, and silyl (see *e.g.,* U.S. Patent No. 7,678,869). The following paragraphs provide a few non-limiting examples of copolymerization applications.

Paints that comprise polymers and copolymers of acrylic acid and its esters are in wide use as industrial and consumer products. Aspects of the technology for making such paints can be found in *e.g.,* U.S. Patent Nos. 3,687,885 and 3,891,591. Generally, acrylic acid and its esters may form homopolymers or copolymers among themselves or with other monomers, such as amides, methacrylates, acrylonitrile, vinyl, styrene and butadiene. A desired mixture of homopolymers and/or copolymers, referred to in the paint industry as "vehicle" (or "binder") can be added to an aqueous solution and agitated sufficiently to form an aqueous dispersion that can include sub-micrometer sized polymer particles. The paint can cure by coalescence of these vehicle particles as the water and any other solvent evaporate. Other additives to the aqueous dispersion may include pigment, filler *(e.g.,* calcium carbonate, aluminum silicate), solvent (*e.g.,* acetone, benzol, alcohols, etc., although these are not found in certain no VOC paints), thickener, and additional additives depending on the conditions, applications, intended surfaces, etc. In many paints, the weight percent of the vehicle portion may range from about nine to about 26 percent, but for other paints the weight percent may vary beyond this range.

Acrylic-based polymers can be used for many coatings in addition to paints. For example, for paper coating latexes, acrylic acid can be used from 0.1-5.0%, along with styrene and butadiene, to enhance binding to the paper and modify rheology, freeze-thaw stability and shear stability. *See e.g.,* U.S. Patent Nos. 3,875,101 and 3,872,037. Acrylate-based polymers can be used in many inks, particularly UV curable printing inks. For water treatment, acrylamide and/or hydroxy ethyl acrylate can be co-polymerized with acrylic acid to produce low molecular-weight linear polymers. *See e.g.,* U.S. Patent Nos. 4,431,547 and 4,029,577. Co-polymers of acrylic acid with maleic acid or itaconic acid can also be produced for water-treatment applications, as described in U.S. Patent No. 5,135,677. Sodium acrylate (the sodium salt of glacial acrylic acid) can be co-polymerized with acrylamide (which may be derived from acrylic acid via amidation chemistry) to make an anionic co-polymer that can be used as a flocculant in water treatment.

For thickening agents, a variety of co-monomers can be used, such as those described in U.S. Patent Nos. 4,268,641 and 3,915,921. U.S. Patent No. 5,135,677 describes a number of co-monomers that can be used with acrylic acid to produce water-soluble polymers.

In some cases, conversion to downstream products may be made enzymatically. For example, 3-HP may be converted to 3-HP-CoA, which then may be converted into polymerized 3-HP with an enzyme having polyhydroxy acid synthase activity (EC 2.3.1.-). Also, 1,3-propanediol can be made using polypeptides having oxidoreductase activity or reductase activity (*e.g.* , enzymes in the EC 1.1.1.- class of enzymes). Alternatively, when creating 1,3-propanediol from 3-HP, a combination of (1) a polypeptide having aldehyde dehydrogenase activity (*e.g*., an enzyme from the 1.1.1.34 class) and (2) a polypeptide having alcohol dehydrogenase activity (*e.g*., an enzyme from the 1.1.1.32 class) can be used. Polypeptides having lipase activity may be used to form esters. Enzymatic reactions such as these may be conducted *in vitro,* such as using cell-free extracts, or in *vivo.*

Thus, various embodiments of the invention, such as methods of making a chemical, can include conversion steps to any downstream products of microbially produced 3-HP, including but not limited to those chemicals described herein and known in the art. For example, in some cases, 3-HP can be produced and can be converted to polymerized-3-HP (poly-3-HP) or acrylic acid. In some cases, 3-HP or acrylic acid can be used to produce polyacrylic acid (polymerized acrylic acid, in various forms), methyl acrylate, acrylamide, acrylonitrile, propiolactone, ethyl 3-HP, malonic acid, 1,3-propanediol, ethyl acrylate, n-butyl acrylate, hydroxypropyl acrylate, hydroxyethyl acrylate, isobutyl acrylate, 2-ethylhexyl acrylate, and acrylic acid or an acrylic acid ester to which an alkyl or aryl addition may be made, and/or to which halogens, aromatic amines or amides, and aromatic hydrocarbons may be added.

Reactions that form downstream compounds such as acrylates or acrylamides can be conducted in conjunction with use of suitable stabilizing agents or inhibiting agents reducing the likelihood of polymer formation. *See,* for example, U.S. Publication No. 2007/0219390. Stabilizing agents and/or inhibiting agents include, but are not limited to, *e.g.,* phenolic compounds (*e.g*., dimethoxyphenol (DMP) or alkylated phenolic compounds such as di-tert-butyl phenol), quinones (*e.g*., t-butyl hydroquinone or the monomethyl ether of hydroquinone (MEHQ)), and/or metallic copper or copper salts *(e.g.,* copper sulfate, copper chloride, or copper acetate). Inhibitors and/or stabilizers can be used individually or in combinations as will be known by those of skill in the art.

In some cases, the one or more downstream compounds can be recovered at a molar yield of up to about 100 percent, or a molar yield in the range from about 70 percent to about 90 percent, or a molar yield in the range from about 80 percent to about 100 percent, or a molar yield in the range from about 90 percent to about 100 percent. Such yields may be the result of single-pass (batch or continuous) or iterative separation and purification steps in a particular process.

The methods of the invention can also be used to produce downstream compounds derived from 3-HP, such as but not limited to, polymerized-3-HP (poly-3-HP), acrylic acid, polyacrylic acid (polymerized acrylic acid, in various forms), copolymers of acrylic acid and acrylic esters, acrylamide, acrylonitrile, propiolactone, ethyl 3-HP, malonic acid, and 1,3-propanediol. Also, among esters that are formed are methyl acrylate, ethyl acrylate, n-butyl acrylate, hydroxypropyl acrylate, hydroxyethyl acrylate, isobutyl acrylate, and 2-ethylhexyl acrylate. These and/or other acrylic acid and/or other acrylate esters may be combined, including with other compounds, to form various known acrylic acid-based polymers. Numerous approaches may be employed for such downstream conversions, generally falling into enzymatic, catalytic (chemical conversion process using a catalyst), thermal, and combinations thereof (including some wherein a desired pressure is applied to accelerate a reaction). For example, without being limiting, acrylic acid may be made from 3-HP via a dehydration reaction, methyl acrylate may be made from 3-HP via dehydration and esterification, the latter to add a methyl group (such as using methanol), acrylamide may be made from 3-HP via dehydration and amidation reactions, acrylonitrile may be made via a dehydration reaction and forming a nitrile moiety, propiolactone may be made from 3-HP via a ring-forming internal esterification reaction, ethyl-3-HP may be made from 3-HP via esterification with ethanol, malonic acid may be made from 3-HP via an oxidation reaction, and 1,3-propanediol may be made from 3-HP via a reduction reaction. Additionally, it is appreciated that various derivatives of the derivatives of 3-HP and acrylic acid may be made, such as the various known polymers of acrylic acid and its derivatives. Production of such polymers can be considered within the scope of the present disclosure. Copolymers containing acrylic acid and/or esters have been widely used in the pharmaceutical formulation to achieve extended or sustained release of active ingredients, for example as coating material. Downstream compounds may also be converted to consumer products such as diapers, carpet, paint, and adhesives.

Another important product, acrylamide, has been used in a number of industrial applications. Acrylamide may be produced from 3-HP, for example, without being limiting, via an esterification-amidation-dehydration sequence. Refluxing an alcohol solution of 3-HP in the presence of an acid or Lewis acid catalyst described herein can lead to a 3-HP ester. Treatment of the 3-HP ester with either an ammonia gas or an ammonium ion could yield 3-HP amide. Finally, dehydration of the 3-HP amide with dehydration reagents described elsewhere in this disclosure could produce acrylamide. The steps mentioned herein may be rearranged to produce the same final product acrylamide. Polymerization of acrylamide can be achieved, for example, and without being limiting, by radical polymerization. Polyacrylamide polymers have been widely used as additives for treating municipal drinking water and waste water. In addition, they have found applications in gel electrophoresis, oil-drilling, papermaking, ore processing, and the manufacture of permanent press fabrics.

### IX. EXPRESSION SYSTEMS GENERAL CONCEPTS

The following general concepts can be applicable to the embodiments of the disclosuredescribed above.

### Multicopy Plasmids

The researcher is faced with a myriad of genetic module options when designing a plasmid for expression of a heterologous protein in a host cell. How to optimize an expression plasmid system often depends on the downstream use of the expressed protein.

Different cloning vectors or plasmids are maintained at different copy numbers, dependent on the replicon of the plasmid. Most general cloning plasmids can carry a DNA insert up to about 15 kb in size.

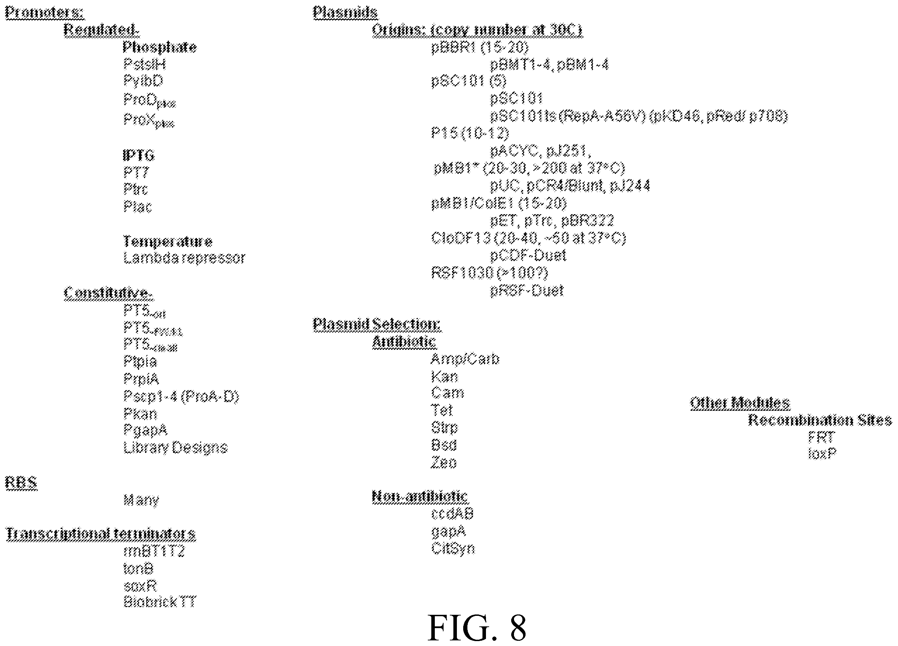

Multicopy plasmids can be used for the expression of recombinant genes in *Escherichia coli.* Examples of include multicopy plasmids include high-copy, medium-copy and low-copy plasmids **(see *e.g.,*** **FIG. 8****)**. The high copy number plasmid is generally desired for maximum gene expression. However, the metabolic burden effects can result from multiple plasmid copies could prove to be detrimental for maximum productivity in certain metabolic engineering applications by adding significant metabolic burden to the system.

The low-copyplasmids, for example, pBR322 is based on the original ColE1 replicon and thus has a copy number of about 15-20. The pACYC series of plasmids are based on the p15A replicon, which has a copy number of about 18-22, whereas pSC101 has even a lower copy number of about 5, and BACs are typically maintained at one copy per cell. Such low copy plasmids may be useful in metabolic engineering applications, particularly when one or more of the substrates used in the recombinant pathway are required for normal cellular metabolism and can be toxic to the cell at high levels.

However, the use of high-copy plasmids may be useful in enhanced cellular metabolism contexts. The mutant ColEl replicon, as found in the pUC series of plasmids produces a copy number of about 500-700 as a result of a point mutation within the RNAII regulatory molecule.

There are transcription and translation vectors. Transcription vectors are utilized when the DNA to be cloned has an ATG start codon and a prokaryotic ribosome-binding site. Translation vectors contain an efficient ribosome-binding site and, therefore, it is not necessary for the target DNA to contain one. This is particularly useful in cases where the initial portion of the gene may be cleaved in an effort to improve solubility. Another consideration when choosing a transcription or translation vector is the source of the DNA to be expressed. Prokaryotic genes usually have a ribosome-binding site that is compatible with the host *E. coli* translation machinery, whereas eukaryotic genes do not. Normal prokaryotic gene expression may be enhanced by use of an engineered promoter and ribosome-binding site.

### Promoters

A promoter is a region of DNA that initiates transcription of a particular gene. In bacteria, transcription is initiated by the promoter being recognized by RNA polymerase and an associated sigma factor, which are often brought to the promoter site by an activator protein's binding to its own DNA binding site located proximal or distal (*e.g*., near) to the promoter.

Promoter selection is an important factor when designing an expression plasmid system. A promoter is located upstream of the ribosome-binding site. Owing to the fact that many heterologous protein products are toxic to the cell, the promoter can be regulated so that the heterologous protein is expressed at the appropriate amount and time to reduce the burden on the cell host.

Historically, the most commonly used promoters have been the lactose (lac) and tryptophan (trp) promoters. These two promoters were combined to create the hybrid promoters tac and trc that are also commonly used. Other common promoters are the phage lambda promoters, the phage T7 promoter (T7), and the alkaline phosphatase promoter (phoA).

Promoters can be constitutive and inducible. Constitutive promoters are active in all circumstances in the cell, while regulated or inducible promoters become active in response to specific stimuli. In addition, the strength of the promoter can also differ. A strong promoter has a high frequency of transcription and can generate a heterologous protein that is about 10-30% of the total cellular protein production (for examples see **FIG. 8****)**. Chemically-inducible promoters that can be used in various aspects of the disclosureinclude, but are not limited to, promoters whose transcriptional activity is regulated by the presence or absence of alcohol, tetracycline, steroids, metal and other compounds. Physically-inducible promoters that can be used in various aspects of the disclosureinclude, but are not limited to, promoters whose transcriptional activity is regulated by the presence or absence of light and low or high temperatures.

In order to be an inducible promoter, the promoter should ideally initially be completely repressed to transcription. Although, most inducible promoters are known to have some leakage, these inducible promoters are also contemplated. Transcription is subsequently induced at any given time with the addition of an inducer into the desired host cell. Alternatively, an inducible promoter may be responsive to the lack of a substance, such as inorganic phosphate, such that the absence of inorganic phosphate will allow expression at a desired time in the host cell (for examples see **FIG. 8****)**.

### Ribosome Binding Sites

A Ribosome Binding Sites (RBS) is an RNA sequence upstream of the start codon that affects the rate at which a particular gene or open reading frame (ORF) is translated. A person of skill in the art can tailor an RBS site to a particular gene. Ribosome Binding Sites (RBSs) are typically short sequences, often less than 20 base pairs. Various aspects of RBS design are known to affect the rate at which the gene is translated in the cell. The RBS module can influence the translation rate of a gene largely by two known mechanisms. First, the rate at which ribosomes are recruited to the mRNA and initiate translation is dependent on the sequence of the RBS. Secondly, the sequence of the RBS can also affect the stability of the mRNA in the cell, which in turn affects the overall level of proteins. Through the use of genetic expression modules, the expression of desired genes, such as genes encoding enzymes in the biosynthetic pathway for 3-HP, can be tailored either at the transcriptional and translational level.

One can access the registry RBS collection as a starting point for designing an RBS <<http://partsregistry.org/Ribosome_Binding_Sites/Catalog>> ("Registry"). As of the date of application, the Registry has collections of RBSs that are recommended for general protein expression in *E. coli* and other prokaryotic hosts. In addition, each family of RBSs has multiple members covering a range of translation initiation rates. There are also several consensus RBS sequence for *E. coli* that have been described. However, it is important to keep in mind the known RBS functions and mechanisms in a larger context. For example, in certain contexts the RBS can interact with upstream sequences, such as sequence that comprise the promoter or an upstream ORF. In other contexts, the RBS may interact with downstream sequences, for example, the ribosome enzyme binds jointly to the RBS and start codon at about the same time. These potential interactions should be considered in the overall RBS sequence design. The degree of secondary structure near the RBS can affect the translation initiation rate. This fact can be used to produce regulated translation initiation rates.

The Shine-Dalgarno portion of the RBS is critical to the strength of the RBS. The Shine-Dalgarno is found at the end of the 16s rRNA and is the portion that binds with the mRNA and includes the sequence 5 '- ACCUCC - 3 '. RBSs will commonly include a portion of the Shine-Dalgarno sequence. One of the ribosomal proteins, S1, is known to bind to adenine bases upstream from the Shine-Dalgarno sequence. As a result, it is hypothesized that the RBS can be made stronger by adding more adenines in the sequence upstream of the RBS.

When considering the design of the spacing between the RBS and the start codon, it is important to think of the aligned spacing rather than just the absolute spacing. While the Shine-Dalgarno portion of the RBS is critical to the strength of the RBS, the sequence upstream of the Shine-Dalgarno sequence may also be important. Note that the promoter may add some bases onto the start of the mRNA that may affect the strength of the RBS by affecting S1 binding.

Computer programs that design RBS sequence to match protein coding sequences, desired upstream sequences including regulatory mRNA sequences, and account of secondary structure are known [*see, e.g.,* Salis, Mirsky, and Voight, Nature Biotechnology 27: 946-950, 2009] and were used to optimize RBSs for the ACCase subunit genes as described in (see **EXAMPLE 2).**

### X. EXPORT OF 3-HYDROXYPROPIONIC ACID FROM CELLS

3-HP is structurally and chemically similar to a number of amino acids (*e.g*., serine, theronine, and to a lesser degree cysteine). These amino acids can be removed from cells by amino acid-specific flux proteins that belong to the major facilitator family (MF). *See, e.g.,* Kramer, R. (1994) Secretion of amino acids by bacteria · physiology and mechanism FEMS Microbiol. Rev. 13, 75-93; *see also e.g.,* Kramer, R. (1994) Systems and mechanisms of amino acid uptake and secretion in prokaryotes Arch. Microbiol. 162, 1-1. This protein family is part of a larger family of previously described and inferred drug extrusion translocases that also include SMR (small multidrug resistance) family, RND (resistance nodulation cell division), ABC (ATP-b:inding cassette) family, and MATE (multidrug and toxic compound extrusion) family. *See, e.g.,* Yamada S, Awano N, Inubushi K, Maeda E, Nakamori S, Nishino K, Yamaguchi A, Takagi H. (2006) Effect of drug transporter genes on cysteine export and overproduction in Escherichia coli. Appl Environ Microbial. 2006 Jul;72(7):4735-42. Most of these proteins (all except the ABC family) can use the proton gradient or sodium gradient across the inner bacterial membrane to facilitate, *e.g*., power, translocation. Discussed herein, the proton gradient directed translocases are elucidated as increasing. Furthermore, modifying the effect of the proton gradient directed translocases is expected to preserve as much metabolic energy as possible. However, it can be appreciated that in some embodiments, ATP, sodium, or other metabolite gradient-directed translocases could also be used.

In some embodiments, the invention may be practiced in a range of microorganisms, such as those bacteria and yeast common use for industrial bioproduction of chemicals. Culture systems can comprise a population of a genetically modified microorganism according to an embodiment of the present invention placed in a media comprising suitable nutrients for growth.

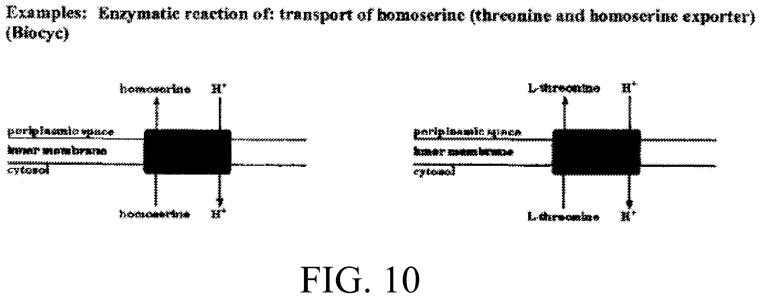

In some embodiments, the genetically modified microorganisms according to the present invention may be cultured under aerobic, anaerobic, or microaerobic conditions. *See e.g.,* FIG. 10. Although some of these proteins have been shown to be fairly specific, members of this protein family have been shown to transport several different amino acids. There are more than 30 identified and putative transporters in the *E. coli* genome. However, the specificities of most of these proteins are unknown. In the MF family, the following specificities have been reported.

| **Serine** | **Threonine** | **Cysteine** |
|---|---|---|
| rhtA | rhtA | eamA |
| rhtB | rhtC | eamB |

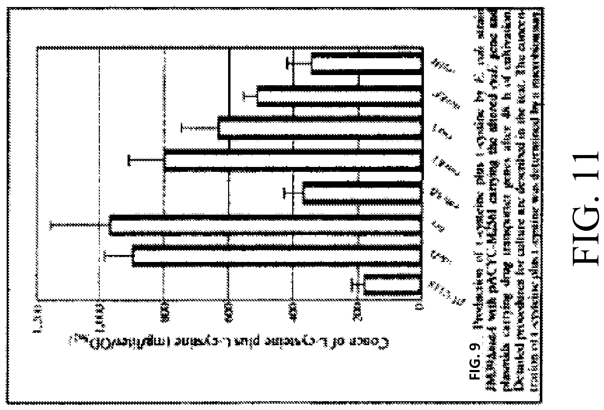

This list does not necessarily reflect the full set of proteins involved in the transportation of these amino acids. Overexpression of 33 transporters have identified seven transporters that resulted in a significant increase of cysteine efflux (*see e.g.,* FIG. 11, adapted from Yamada S, Awano N, Inubushi K, Maeda E, Nakamori S, Nishino K, Yamaguchi A, Takagi H. (2006) Effect of drug transporter genes on cysteine export and overproduction in Escherichia coli. Appl Environ Microbial. 2006 Jul;72(7):4735-42).

The table below (Table 1) lists paralogs for the identified transport proteins listed in Biocyc. The first column shows the serine and threonine transports, the second column shows the known cysteine transporters, and the third column shows other predicted transporters with operon information or expected functional information:

**Table 1**

| **rhtA, rhtB, rhtC, eamA, or eamB family** | **Bcrfamily** | **bcrfamily with unknown functions** |
|---|---|---|
| yedA | emrB | garP (in operon with garRLK) likely importer |
| yijE | emrD | gudP(in operon with gudPX) likely importer |
| ytfF | emrY | hsrA (in operon with yieP) likely exporter |
| leuE | ydeD (known cysteine) | mdtD (in operon with mdtABC) likely exporter |
| rhtC | yfiK (known cysteine) | mdtG likely exporter |
| yahN | | mdtl (next to tnaB - tryptophan transporter) likely exporter |
| | | mdtM |
| | | mhpT(3-(3-hydroxyphenyl)propionate transporter) likely exporter |
| | | yajR |
| | | ybjJ (next to riboflavin phosphates) |
| | | ydhC (next to fatty acid synthesis gene) likely exporter |
| | | ydjE |
| | | ydjK(in an operon with |
| | | putative threonine dehydrogenase ydjL) |
| | | yfd (next to fatty acid synthesis gene) likely exporter |
| | | yhjE |
| | | yidE |
| | | yjiJ |

In a first evaluation, a subset of these proteins can be selected and tested for 3-HP export. As noted, the focus can concentrate on the proton gradient driven translocases, but in general sodium driven, ATP-driven, or other metabolite gradient driven translocases can be evaluated.

### EXAMPLES

### EXAMPLE 1: SALT INHIBITION STUDIES IN E. COLI

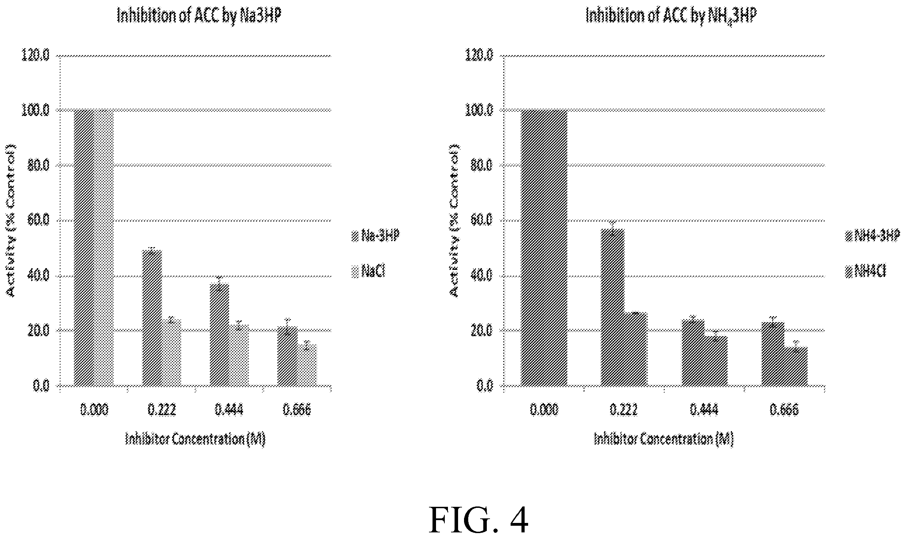

The activity of ACCase complex, a critical enzyme in the conversion of acetyl-CoA to malonyl-CoA, the immediate precursor for 3-HP, is severely inhibited by salt. Dose-dependent effects on ACCase activity was observed in the presence of NaCl, NH₄Cl, Na-3-HP , or NH₄-3-HP such that salt levels near 0.44M resulted in decreasing the activity of the ACCase enzyme by approximately 80%, while salts of 3-HP levels near 0.66M decreased the activity of the ACCase enzyme by approximately 80% relative to control (**FIG. 4**). Levels of greater than 0.66M (60 g/L) are expected to be present for commercially viable commercial production of 3-HP.

### EXAMPLE 2: OTHER SALT-TOLERANT ENZYMES

Microorganisms comprising a genetic modifications to enzymes from slight, moderate, and/or extreme halophiles organisms are made. Homology with genes are determined by analysis with BLASTN version 2.0 provided through the NCBI website. Homology with proteins are determined by analysis with BLASTP version 2.2.2 provided through the NCBI website.

The enzymes from the following halophilic organisms are used: *Chromohalobacter salexigens, Flexistipes sinusarabici strain (MAS10T), Halobacterium sp. NRC-1, Haloarcula marismortui, Natronomonas pharaonis, Haloquadratum walsbyi, Haloferax volcanii, Halorubrum lacusprofundi, Halobacterium sp. R-1, Halomicrobium mukohataei, Halorhabdus utahensis, Halogeometricum borinquense, Haloterrigena turkmenica, Natronobacterium gregoryi SP2, Halalkalicoccus jeotgali, Natrialba magadii, Haloarcula hispanica, Halopiger xanaduensis, Halophilic archaeon DL31, Haloferax mediterranei, Halovivax ruber, Natronococcus gregoryi,* and *Natronococcus occultus.*

Moderate halophilic organisms in which homologous enzymes are derived are: eukaryotes such as crustaceans (*e.g., Artemia salina),* insects (*e.g., Ephydra hians*), certain plants from the genera *Salicornia spp,* algae (*e.g., Dunaliella viridis*), fungi, and protozoa (*e.g., Fabrea salina*), phototrophic organisms, such as planktonic and microbial mat-formers cyanobacteria as well as other anaerobic red and green sulphur bacteria from the genera *Ectothiorhodospira spp.*) and non-sulphur bacteria from the genera *Chromatium spp.;* gram-negative anaerobic bacteria, for example from the genera *Haloanaerobacter spp.* some of which are methanogenic, for example from the genera *Methanohalophilus* spp. and either aerobic or facultative such as species from the genera *Halomonas, Chromohalobacter, Salinovibrio, Pseudomonas* (*e.g., Halomonase elongate*); gram-positive bacteria from genera such as *Halobacillus, Bacillus, Marinococcus,* etc. as well as some actinomycetes, *e.g., Actinopolyspora halophila.*

### Genomic and Proteomic Hallmarks of Halophilic Organisms

Proteins of microorganisms are genetically modified for salt tolerance such that they have low hydrophobicity, over-representation of acidic residues, especially Asp, under-representation of Cys, lower propensities for helix formation and higher propensities for coil structure.

Suitable salt-tolerant enzymes from salt-tolerant organisms are used. Salt-tolerant organisms (such as, *e.g.,* halophiles) include any living organism that are adapted to living in conditions of high salinity. Suitable salt-tolerant enzymes can include enzymes from salt-tolerant organism that are homologs of the following enzymes: Sucrose-6-phosphate hydrolase (*e.g.,* cscA from *E. coli*), glucose-6-phosphate isomerase (*e.g.,* pgi from *E. coli*), fructokinase (*e.g.,* cscK from *E. coli*), fructose-1,6-bisphosphatase (*e.g.,* yggF from *E. coli*), fructose 1,6-bisphosphatase (*e.g.,* ybhA from *E. coli*), fructose 1,6-bisphosphatase II (*e.g.,* glpX from *E. coli*), fructose-1,6-bisphosphatase monomer (*e.g.,* fbp from *E. coli*), 6-phosphofructokinase-1 monomer (*e.g.,* pfkA from *E. coli*), 6-phosphofructokinase-2 monomer (*e.g.,* pfkB from *E. coli*), fructose bisphosphate aldolase monomer (*e.g.,* fbaB from *E. coli*), fructose bisphosphate aldolase monomer (*e.g.,* fbaA from *E. coli*), triose phosphate isomerase monomer (*e.g.,* tpiA), glyceraldehyde 3-phosphate dehydrogenase-A monomer (*e.g.,* gapA from *E. coli*), phosphoglycerate kinase (*e.g.*, pgk), 2,3-bisphosphoglycerate-independent phosphoglycerate mutase (*e.g.,* gpmM from *E. coli*), 2,3-bisphosphoglycerate-dependent or tdcE (*e.g.,* from *E. coli*), phosphoglycerate mutase (*e.g.,* gpmA), enolase (*e.g.,* eno from *E. coli*), phosphoenolpyruvate carboxylase (*e.g.,* ppc from *E. coli*), malate dehydrogenase (*e.g.,* mdh), fumarase A (*e.g.,* fum from *E. coli*), fumarase B (*e.g.,* fumB), fumarase C (*e.g.,* fumC from *E*. *coli*), phosphoenolpyruvate synthetase (*e.g.,* ppsA from *E. coli),* pyruvate kinase I monomer (*e.g.,* pykF from *E. coli*), pyruvate kinase II monomer (*e.g.,* pykA from *E. coli*), fumarate reductase (*e.g.,* frdABCD from *E. coli*), lipoamide dehydrogenase (*e.g.,* lpd from *E. coli*), pyruvate dehydrogenase (*e.g.,* aceE from *E. coli*), pyruvate dehydrogenase (*e.g.,* aceF from *E. coli*), pyruvate formate-lyase (*e.g.,* pflb from *E. coli*), acetyl-CoA carboxylase (*e.g.,* accABCD from *E*. *coli*), malonyl CoA reductase (*e.g.,* mcr), 3HP dehydrogenase (*e.g.,* mmsB, NDSD, or ydfG), malonate semialdehyde reductase (*e.g.,* nemA, rutE from *E. coli*), or a combination thereof.

Suitable salt-tolerant enzyme homologs that can be used with the claimed invention can have at least and at least about 99%, 98%, 97%, 96%, 95%, 94%, 93%, 92%, 91%,90%, 89%, 88%, 87%, 86%, 85%, 84%, 83%, 82%, 81%, 80%, 75%, or 70% overall amino acid or nucleotide identity to the above enzymes. Suitable salt-tolerant enzyme homologs that can be used with the claimed invention can have at least about 99%, 98%, 97%, 96%, 95%, 94%, 93%, 92%, 91%,90% 89%, 88%, 87%, 86%, 85%, 84%, 83%, 82%, 81%, 80%, 75%, or 70% amino acid or nucleotide to the essential protein function domains of the enzymes above. Suitable salt-tolerant enzyme homologs that can be used with the claimed invention can have at least about 99%, 98%, 97%, 96%, 95%, 94%, 93%, 92%, 91%,90%, 89%, 88%, 87%, 86%, 85%, 84%, 83%, 82%, 81%, 80%, 75%, or 70% overall amino acid or nucleotide to the essential binding amino acids within an essential protein function domain of the enzymes above.

In accordance with a preferred embodiment of the invention, suitable salt-tolerant enzyme homologs are enzymes from one of the following organisms: *Halomonas elongata, Salinibacter rubur,* or *Halobacterium* species (Archaea).

In accordance with a preferred embodiment of the present invention, there is provided a non-salt-tolerant organism that is genetically modified to make 3-HP, wherein the genetic modification includes a polynucleotide encoding an acetyl-CoA carboxylase from a salt-tolerant organism. In accordance with a preferred embodiment, the acetyl-CoA carboxylase subunits accA, accB, accC and accD is from *Halomonas elongata.*

### EXAMPLE 3: ACCase FROM HALOPHILIC ORGANISM

Halophilic organisms, such as *Halomonas elongata,* are found in environments with high salt concentrations and, in general, have a salt internal concentration >2.5-3M. It is hypothesized that enzymes derived from any salt-tolerant species should be more resistant to enzyme inhibition by salts, such as 3-HP. Further, these enzymes that have greater salt tolerance should in turn have extended production under high salt conditions than enzymes with lower salt tolerance.

Accordingly, the genes encoding the accA, accB, accC, accD of *H. elongata* described in Table 1 were synthesized for expression in *E. coli* using codons optimized for this organism and supplied individually on pUC57 plasmids without promoters. Synthetic operons comprising the subunits were assembled using the Gibson assembly method.

**Table 1. Accession numbers for genes encoding ACCase subunits from Halomonas elongate.**

| **Gene** | **Accession number** | **SEQ ID NO.** |
|---|---|---|
| accA | YP_003898857.1 | SEQ ID NO. 1, 2 |
| accB | YP_ 003897250.1 | SEQ ID NO. 3, 4 |
| accC | YP_003897249.1 | SEQ ID NO. 5, 6 |
| accD | YP_003897309.1 | SEQ ID NO. 7, 8 |

Each gene was amplified by PCRs with *Pfu* Ultra II HS according to the manufacturer's instructions, and the PCR products were purified using the Zymo PCR Cleanup kit. Concentrations of products were measured using the Nanodrop spectophotometer. The Gibson Assembly kit (NEB) was used to construct plasmids expressing the ACCase subunit genes as directed by the manufacturer. The effect of NH₄-3-HP and NH₄Cl on *H. elongata* ACCase was tested and compared to the *E. coli* ACCase. As shown in **Fig. 4****,** whereas the *E. coli* ACCase is significantly inhibited by the salts, the ACCase from the halophile is less affected by either NH₄-3-HP or by NH₄Cl. This result indicated that use of the *H. elongata* ACCase in 3-HP production strains would in beneficial in relieving 3-HP inhibition of the conversion of acetyl-CoA to malonyl-CoA, a critical step in the pathway.

### EXAMPLE 4: RBS-OPTIMIZED GENES

Enzyme expression is regulated at transcriptional and translational levels in prokaryotes. Ribosome Binding Sites (RBS) are 15 nucleotide segments which are known to control the level of protein expression in microorganisms. To enhance *H. elongata* ACCase expression various customized RBS were constructed and optimized for *E. coli* translation expression. **Table 2** shows the RBS sequences used to increase expression of the individual subunits.

### Table 2:

**Table 2. RBS sequences used to enhance expression of H. elongate ACCase subunits.**

| *H. elongat a ACC* expression plasmid | Modified RBS sequences preceeding ATG (underlined) | | | |
|---|---|---|---|---|
| | *He_accD* | *He_accA* | *He_acc C* | *He_acc B* |
| P arent 2-4 | 5'- GCGTAGTAAAG GA GGTAAC ATATG | 5′- CAATTTATTTA AGGAGGACTCT TAAGATG | 5′- GAAATTTCAT ACCACAGGCG AAGGAGGAAA AACCATG | 5′- GGAAGAACAA GGGGTGTACA TG |
| B 2 | Same as 2-4 | Same as 2-4 | Same as 2-4 | 5′- ggaagaatta agggggacaa gggggaataA TG |
| 1 3A | 5'- gcgtagtagcc gggtgataagg agccgtaacAT G | Same as 2-4 | Same as 2-4 | |
| 1 4C | 5'- gcgtagtagct gatataaaagg aggtaacggAT G | Same as 2-4 | Same as 2-4 | Same as 2-4 |
| 1 5C | Same as 2-4 | 5'- caatttatttt tgttcacccaa ggagtattgct aATG | Same as 2-4 | Same as 2-4 |
| 1 7C | Same as 2-4 | 5′- caatttattta ccgaaataaaa ggagggatgcg aATG | Same as 2-4 | Same as 2-4 |
| 3 5C | 5′- gcgtagtagcc | 5'- caatttatttt | Same as 2-4 | Same as 2-4 |
| | gggtgataagg agccgtaacATG | tgttcaccca a ggagtattgc t aATG | | |
| 3 6C | 5'- gcgtagtagcc gggtgataagg agccgtaacAT G | 5'- caatttattta ccgaaataaaa ggagggatgcg aATG | Same as 2-4 | Same as 2-4 |
| 3 6C-8 | 5'- gcgtagtagcc gggtgataagg agccgtaacAT G | 5'- caatttattta ccgaaataaaa ggagggatgcg aATG | Same as 2-4 | 5'- ggaagaatta aggggacaa ggggaataA TG |
| 7 2B | 5'- gcgtagtagcc gggtgataagg agccgtaacAT G | 5'- caatttattta ccgaaataaaa ggagggatgcg aATG | 5′- TCTTCCCACA ACACTGGCGG ACTCCATCAT G | 5′- GAAATTTCAT ACCACAGGCG AAGGAGGAAA AACCATG |
| 1 05F | 5'- gcgtagtagcc gggtgataagg agccgtaacAT G | 5'- caatttatttt tgttcacccaa ggagtattgct aATG | 5′- TCTTCCCACA ACACTGGCGG ACTCCATCAT G | 5′- GAAATTTCAT ACCACAGGCG AAGGAGGAAA AACCATG |

The expression performance of the RBS-optimized *H. elongata* ACCases was evaluated by 3-HP production in a 96-well format, each in triplicate wells, and the averaged results shown in **Table 3.** Specific 3HP production is shown as g/L per OD₆₀₀. As may be seen in Table 3, enhancing the efficiency of the RBS in strains B2, 35C, and 72 B clearly resulted in increased malonyl-CoA production leading to increased 3-HP production. It is evident from these results that combinations of enhanced RBS's before each of the individual genes *accA, accB, accC,* and *accD* may result in strains with even higher ACCase expression and activity.

**Table 3. Improvement in 3-HP production by RBS-optimized expression of H. elongata ACCase subunits.**

| *H. elongata* ACCase expression plasmid | 3HP (g/l.OD) |
|---|---|
| Parent 2-4 | 0.06 |
| B2 | 0.81 |
| 13A | 0.01 |
| 14C | 0.54 |
| 15C | 0.14 |
| 17C | 0.08 |
| 35C | 0.68 |
| 36C | 0.31 |
| 36C-8 | 0.31 |
| 72B | 0.57 |
| 105F | 0.19 |

### EXAMPLE 5: COORDINATED EXPRESSION BY SUBUNIT FUSIONS

In nature ACCase subunit genes from prokaryotes such as *E. coli* and *H. elongata* have been shown to have a quaternary structure: *accA₂*:*accD₂*:*accB₄*:*accC₂*. However, the intrinsic levels of the ACCase subunit genes are too low for optimal production. Therefore, for optimal production it is ideal to have overexpression to be coordinated in a similar manner.

Expression of the genes encoding each ACCase subunit is regulated at transcriptional and translational levels. Coordinated overexpression of the ACCase subunit genes, *accA, accB, accC, accD* should give better ACCase activity. Examples of fusions of *accC-B* proteins exist in bacteria. It is hypothesized that coordinated overexpression may be achieved by fusion of subunit genes should ensures equimolar expression of the subunit genes at the optimal time.

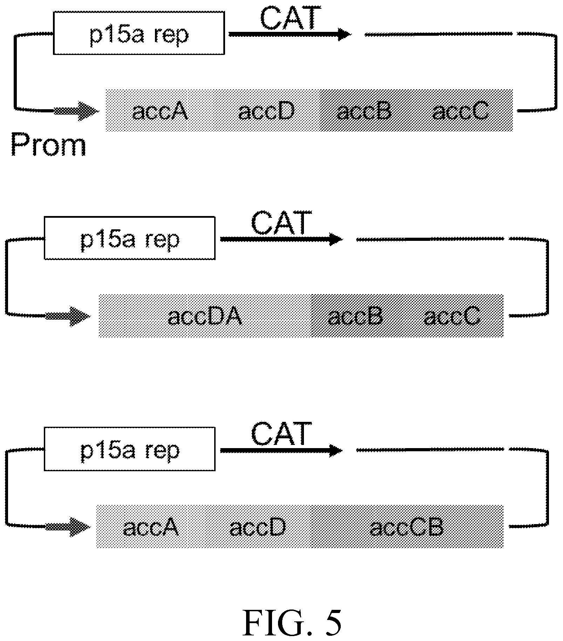

The following ACCase subunit gene fusion were constructed and the constructs overexpressed in *E. coli:* (A) Control ABCD, (B) fusion of *accC-B* (SEQ ID NOs. 9, 10) subunit genes as seen in bacteria, (C) fusion of *accD-A* subunit genes using a flexible 15-amino acid linker (Linker sequence LSGGGGSGGGGSGGGGSGGGGSAAA; SEQ ID NOs. 11, 12) as depicted in **FIG. 5****.**

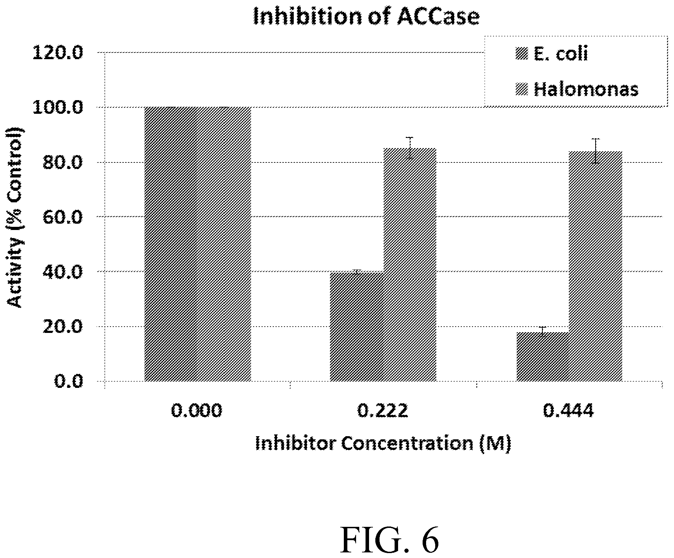

The performance of the ACC fusions were tested for their ACCase activity and for various 3-HP production metrics in **Table 4**. ACCase activity was determined in cell lysates using an assay for malonyl-CoA production as described in Kroeger, Zarzycki, and Fuchs, Analytical Biochem. 411:100-105, 2011. Production of 3-HP was determined in cells co-transformed with a plasmid bearing the genes encoding the malonyl-CoA reductase from *S. tokadaii* and *E. coli ydfG* providing a 3-HP dehydrogenase to complete the metabolic pathway from malonyl-CoA to 3HP. These results show that the strain with the fused *accDA* genes had higher average specific productivity of 3-HP compared to the parental strain in which the overexpressed ACCase is not fused. **Fig. 6** shows that the benefit of the *accDA* fusion were also manifested in 3-HP production in fermentors with environmental controls of nutrient feed, pH, aeration, and temperature.

**Table 4: ACC Fusions and ACCase activity**

| **Strain designation** | **Plasmid** | **Avg specific prodn rate (g/gDCW.h) at TS+6** | **Avg specific prodn rate (g/gDCW.hr) at TS+20** | **ACCase specific activity at TS+6 (U/mg)** |
|---|---|---|---|---|
| **BX3_783** | **Parent (unfused ACCase)** | **0.160** | **0.146** | **0.057** |
| **BX3_829** | **No ACC** | **0.069** | **0.062** | **0.000** |
| **BX3_837** | **EC ACC DA fusion** | **0.209** | **0.201** | **0.054** |

### EXAMPLE 6 3-HP EXPORTER

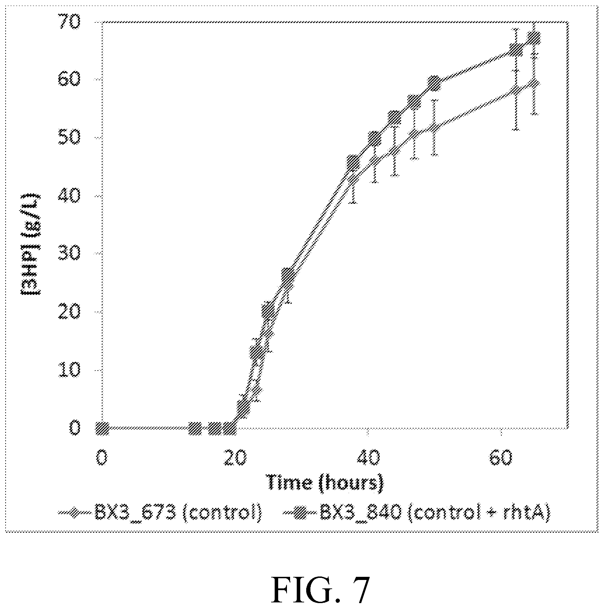

Growth inhibition has been demonstrated for E. coli strains grown in the presence of 3-HP at levels as low as 20 g/L. To produce high titers of 3-HP the production host is required to balance production with overcoming inhibition. A known chemical exporter from *E. coli* that has been previously characterized for homoserine transport, *rhtA,* was evaluated for increased production of 3-HP. A mutant version of the exporter, *rhtA*(P2S) (SEQ ID NOs. 30 and 31) was synthesized behind the PtpiA promoter and inserted into the pTRC-PyibD-MCR plasmid behind a terminator using the Gibson Assembly kit (NEB) according to manufacturer's instructions. The effects of overexpression of *rhtA* were evaluated in 1L fermentation compared to the control plasmid without *rhtA.* As shown in **FIG 7****,** overexpression of *rhtA* resulted in a significant improvement in 3HP titer compared to the control production strain. Construction of plasmids expressing another putative transporter, ydcO (SEQ ID NOs. 32 and 33) is carried out in the same manner.

### EXAMPLE 7 BICARBONATE IMPORTER

Increased import of bicarbonate to increase availability of bicarbonate for the ACCase reaction will increase production of malonyl-CoA and hence products derived metabolically from malonyl-CoA, such as 3-HP. The gene encoding the bicA bicarbonate transporter (SEQ ID NO. 13) of Synechococcus sp. was synthesized using codons optimized for expression in *E. coli* (SEQ ID NO. 14) and expressed using the *E. coli* tal promoter in a strain co-transformed with plasmids encoding ACCase and MCR functions. Production of 3-HP by this strain is compared to that achieved by a control strain without overexpressed bicA.

### EXAMPLE 8: MONOFUNCTIONAL MCR

One of the steps in the biosynthesis of 3-HP involves the conversion of malonyl-CoA (MCA) to malonate semialdehyde (MSA) and the conversion of malonate semialdehyde (MSA) to 3-HP. In order to enhance cellular bioproduction of 3-HP in a host cell, a genetically modified organism having novel enzymes and/or combinations of enzymes to catalyze the MCA to MSA reaction are made.

Organisms that comprise novel enzyme compositions or that co-express a combinations of enzymes to catalyze the conversion of malonyl-CoA to 3-HP are made. For example, malonyl-CoA is converted to malonate semialdehyde by a monofunctional malonyl-CoA reductase that catalyzes the malonyl-CoA conversion, but does not catalyze the malonate semialdehyde conversion.

The organism is genetically modified to comprise and/or express a monofunctional malonyl-CoA reductase from *Sulfolobus tokodaii* (stMCR) (SEQ ID NOs. 15 and 16) or a functional homolog of stMCR or a homolog with at least 70%, 75%, 80%, 85%, 90%, 91%, 92%, 93%, 94%, 95%, 96%, 97%, 98%, or 99%, sequence homology/identity. Other organisms are produced to comprise a genetic modification that include a bi-functional malonyl-CoA reductase comprising two protein fragments with at least one fragment that has malonyl-CoA reductase activity and the other fragment has malonate semialdehyde dehydrogenase activity that is derived from *Chloroflexus aurantiacus* (caMCR).

### EXAMPLE 9: MCR-DEHYDROGENASE ENZYMES FOR CONVERSION OF 3-HP IONS

Following the conversion of the malonyl-CoA to malonate semialdehyde, the malonate semialdehyde is converted to 3-HP through either or both of two alternative pathways. Malonate semialdehyde may exist in at least three states; the keto form, the enol form, and hydrate form.

A genetically modified organism comprising and/or expressing a 3-hydroxy acid dehydrogenase enzyme (*e.g., ydfG*; SEQ ID NOs. 21 and 22), a 3-hydroxyisobutyrate dehydrogenase enzyme (*e.g., Pseudomonas aeruginosa mmsB;* SEQ ID NOs. 23 and 24), and/or NAD+-dependent serine dehydrogenase (*e.g., Pseudomonas NDSD*; SEQ ID NOs. 25 and 26) are made.

Genetically modified organisms are made that also comprise and/or express an N-ethylmaleimide reductase (*e.g., nemA*; SEQ ID NOs. 17 and 18), and/or a malonic semialdehyde reductase (*e.g., rutE,* SEQ ID NOs. 19 and 20) from *E. coli,* with or without the previous aforementioned genes.

### EXAMPLE 10: GENETICALLY MODIFIED ORGANISMS AND/OR MODIFIED/FUSED ENZYMES

The genetically modified organisms are made to comprise and/or express a monofunctional malonyl-CoA reductase gene capable of catalyzing the conversion of malonyl-CoA to malonate semialdehyde; and one or more genes encoding one or more of the following enzymes: *ydfG*, *mmsB, NDSD, rutE,* and *nemA* or a functional homolog or a homolog with at least 70%, 75%, 80%, 85%, 90%, 91%, 92%, 93%, 94%, 95%, 96%, 97%, 98%, or 99%, sequence homology/identity.

A genetically modified organism capable of making 3-HP is also created. The genetically modified organism comprises and/or expresses a polynucleotide encoding a monofunctional malonyl-CoA reductase gene capable of catalyzing the conversion of malonyl-CoA to malonate semialdehyde; one or more genes encoding one or more enzymes capable of converting malonate semialdehyde keto form to 3-HP; and one or more genes encoding one or more enzymes capable of converting either the malonate semialdehyde enol form or the malonate semialdehyde hydrated form to 3-HP.

Also made are monofunctional malonyl-CoA reductase enzymes fused to a dehydrogenase enzyme that is either: (a) NADPH-dependent, (b) NADH-independent, (c) has the capability of using NADPH, (d) flavin-dependent, (e), flavin-independent, (f) has the capability of using flavin, (g) less susceptible to 3-HP inhibition at high concentration, and/or (h) catalyzes a reaction pathway to 3-HP that is substantially irreversible. Also made are monofunctional malonyl-CoA reductase enzymes fused to a dehydrogenase enzyme that is NADPH-dependent.

Also made are genetically modified organisms containing and/or expressing 3-HP dehydrogenase enzymes that are largely NADH-dependent, *e.g., mmsB* and/or *NDSD.* Also made are genetically modified organism containing and/or expressing malonate reductase enzymes that are flavin-dependent, *e.g., rutE* and/or *nemA.* Also made are genetically modified organism containing and/or expressing 3-HP dehydrogenase enzymes that are less susceptible 3-HP inhibition at high concentrations, including *e.g., ydfG* and/or *NDSD.* Also made are genetically modified organism containing and/or expressing 3-HP dehydrogenase or malonate semialdehyde dehydrogenase enzymes that catalyze a reaction pathway to 3-HP that is substantially irreversible, *e.g., rutE* and/or *nemA.*

Monofunctional malonyl-CoA reductase enzyme are also fused to one or more dehydrogenase enzymes. For example, malonyl-CoA reductase is fused with malonate semialdehyde dehydrogenase to create a multi-domain protein (*e.g.*, two domain protein) and having the MCR and dehydrogenase domains adjacent in the sequence.

A first monofunctional malonyl-CoA reductase enzyme is fused to a first dehydrogenase enzyme of one type and second monofunctional malonyl-CoA reductase enzyme is fused to a dehydrogenase enzyme of a different type than the first dehydrogenase enzyme.

In certain fusions, the malonyl-CoA reductase from *S. tokadaii* is fused to *ydfG*, *mmsB, NDSD, rutE,* and/or *nemA* (or some combination thereof). The fused enzyme includes one of the following the following configurations: *mcr* - *ydfG*, *mcr* - *mmsB, mcr* - *NDSD, mcr* - *rutE, mcr-nemA, mcr* - *ydfG* - *mmsB, mcr* - *ydfG* - *NDSD, mcr* - *ydfG* - *rutE, mcr* - *ydfG* - *nemA, mcr-mmsB* - *ydfG*, *mcr* - *mmsB* - *NDSD, mcr* - *mmsB* - *rutE, mcr* - *mmsB* - *nemA, mcr* - *NDSD* - *ydfG*, *mcr* - *NDSD* - *mmsB, mcr* - *NDSD* - *rutE, mcr* - *NDSD* - *nemA, mcr* - *rutE* - *ydfG*, *mcr* - *rutE-mmsB, mcr* - *rutE* - *NDSD, mcr* - *rutE* - *nemA, mcr* - *nemA* - *ydfG*, *mcr* - *nemA* - *mmsB, mcr-nemA* - *NDSD, mcr* - *nemA* - *rutE,* functional homologs, and/or homologs with 70%, 75%, 80%, 85%, 90%, 91%, 92%, 93%, 94%, 95%, 96%, 97%, 98%, or 99%, sequence homology/identity.

In other fusions, the malonyl-CoA reductase from *C. aggregans* is fused to *ydfG, mmsB, NDSD, rutE,* or *nemA* (or some combination thereof). The fused enzyme includes one of the following configurations: *mcr* - *ydfG*, *mcr* - *mmsB, mcr* - *NDSD, mcr* - *rutE, mcr* - nemA, mcr - ydfG - mmsB, mcr - *ydfG* - *NDSD, mcr* - *ydfG* - *rutE, mcr* - *ydfG* - *nemA, mcr* - *mmsB-ydfG*, *mcr* - *mmsB* - *NDSD, mcr* - *mmsB* - *rutE, mcr* - *mmsB* - *nemA, mcr* - *NDSD* - *ydfG*, *mcr-NDSD* - *mmsB, mcr* - *NDSD* - *rutE, mcr* - *NDSD* - *nemA, mcr* - *rutE* - *ydfG*, *mcr* - *rutE* - *mmsB, mcr* - *rutE* - *NDSD, mcr* - *rutE* - *nemA, mcr* - *nemA* - *ydfG*, *mcr* - *nemA* - *mmsB, mcr* - *nemA-NDSD, mcr* - *nemA* - *rutE,* functional homologs, and/or homologs with 70%, 75%, 80%, 85%, 90%, 91%, 92%, 93%, 94%, 95%, 96%, 97%, 98%, or 99%, sequence homology/identity.

In other fusions, the malonyl-CoA reductase from *O*. *trichoides* is fused to *ydfG*, *mmsB, NDSD, rutE,* or *nemA* (or some combination thereof). The fused enzyme may include one of the following configurations: *mcr* - *ydfG*, *mcr* - *mmsB, mcr* - *NDSD, mcr* - *rutE, mcr* - *nemA, mcr-ydfG* - *mmsB, mcr* - *ydfG* - *NDSD, mcr* - *ydfG* - *rutE, mcr* - *ydfG* - *nemA, mcr* - *mmsB* - *ydfG*, *mcr* - *mmsB* - *NDSD, mcr* - *mmsB* - *rutE, mcr* - *mmsB* - *nemA, mcr* - *NDSD* - *ydfG*, *mcr* - *NDSD - mmsB, mcr* - *NDSD* - *rutE, mcr* - *NDSD* - *nemA, mcr* - *rutE* - *ydfG*, *mcr* - *rutE* - *mmsB, mcr-rutE* - *NDSD, mcr* - *rutE* - *nemA, mcr* - *nemA* - *ydfG*, *mcr* - *nemA* - *mmsB, mcr* - *nemA* - *NDSD,* or *mcr* - *nemA* - *rutE,* functional homologs, or homologs with 70%, 75%, 80%, 85%, 90%, 91%, 92%, 93%, 94%, 95%, 96%, 97%, 98%, or 99%, sequence homology/identity.

### EXAMPLE 11: ENHANCED MUTATED MONOFUNCTIONAL MCR FOR BIOPRODUCTION

Microorganisms comprising a genetic modification having a mutated form of *stMCR* that have enhanced activity at about 0 °C to about 10°C, at about 8°C to about 15°C, at about 12°C to about 21°C, at 20°C to about 44°C, at about 30°C to about 37°C, at about 32°C to about 38°C, at about 35°C to about 42°C, at about 40°C to about 50°C, at about 50°C to about 60°C, and/or at about 59°C to less than equal to about 72°C, are made.

Other mutations are also created. The carboxylase domains of the malonyl-CoA reductase derived from *Chloroflexus aggregans* and/or *Oscillochloris trichoides* are enhanced by mutations in the carboxylase binding domain to provide increased 3-HP production over the natural occurring enzyme. Additional mutation are also made. For example, mutations where the carboxylase activity of the malonyl-CoA reductase derived from *Chloroflexus aurantiacus* is enhanced, are made.

Other fusions include microorganisms comprising a genetic modification that include carboxylase domains of the malonyl-CoA reductase derived from *C. aggregans* fused to *ydfG, mmsB, NDSD, rutE,* and/or *nemA* (or some combination thereof). Any of the enhanced mutated MCR are also fused one of the following configurations including *mcr* - *ydfG*, *mcr* - *mmsB, mcr-NDSD, mcr* - *rutE, mcr* - *nemA, mcr* - *ydfG* - *mmsB, mcr* - *ydfG* - *NDSD, mcr* - *ydfG* - *rutE, mcr-ydfG* - *nemA, mcr* - *mmsB* - *ydfG*, *mcr* - *mmsB* - *NDSD, mcr* - *mmsB* - *rutE, mcr* - *mmsB* - *nemA, mcr* - *NDSD* - *ydfG*, *mcr* - *NDSD* - *mmsB, mcr* - *NDSD* - *rutE, mcr* - *NDSD* - *nemA, mcr* - *rutE-ydfG*, *mcr* - *rutE* - *mmsB, mcr* - *rutE* - *NDSD, mcr* - *rutE* - *nemA, mcr* - *nemA* - *ydfG*, *mcr-nemA* - *mmsB,* mcr - *nemA* - *NDSD, mcr* - *nemA* - *rutE,* functional homologs, and/or homologs with 70%, 75%, 80%, 85%, 90%, 91%, 92%, 93%, 94%, 95%, 96%, 97%, 98%, or 99%, sequence homology/identity.

### EXAMPLE 12: TEMPERATURE SENSITIVE MALONYL-CoA REDUCTASE

In this example, a malonyl-CoA reductase (MCR) gene is mutated to enhance its activity at lower temperatures or enhance its activity within a specific temperature range. Any MCR can be used, including, an MCR gene derived from *Sulfolobus tokodaii.*

Mutation is achieved by site directed mutagenesis and/or random mutagenesis. Subsequent screen methods are used to isolate lower temperature enhanced MCRs and/or specific temperature enhanced MCRs. Screening methods can include any methods to measure any of the substrates and/or products of MCR, *e.g.*, malonate semialdehyde, malonyl-CoA, CoA, NADP+, NADPH, Mg²⁺, and/or H+. The substrates and/or products can be labeled, *e.g.,* fluorescence or radioactive.

By repeating this procedure, MCRs with further enhanced activity at lower temperatures and/or MCRs with enhanced activity within a specific temperature range, can be isolated. For example, a mutated MCR having enhanced activity at or about less than 0°C to at or about at 37°C; at or about at 20°C to at or about at 44°C; at or about at 30°C to at or about at 37°C; at or about at 32°C to at or about at 38°C, can be isolated.

### EXAMPLE 13: THE GROWTH PHASE

A genetically modified organism is used in a controlled multi-phase production process which includes an initiation and/or completion of one or more phases of the production process. For example, the bioproduction process includes one or more of: (1) a growth phase; (2) an induction phase; and (3) a production phase.

During the growth phase, the genetically modified organism is replicated while a biocatalyst is built up. During the induction phase, key enzymes are expressed, which are used in production. During the production phase, the genetically modified organism produces the desired chemical product.

During the entire process, the initiation and/or completion of the growth, induction and/or production phases are controlled. Because in some cases, the growth phase is dependent on the presence of a critical external reactant that will initiate growth, the initiation and completion of the growth phase is controlled by the addition and amount of the initiating reactant added to the reaction vessel. For example, the reactant may be phosphate, which is needed for replication of genetically modified organisms, such as, *E. coli* cells. In some cases, the growth phase is initiated by the addition of phosphate to a reaction vessel (together with a carbon source such as sugar and a genetically modified organism such as *E. coli* cell), and the duration of the growth phase is controlled by the amount of phosphate added to the system.

### EXAMPLE 14: THE INDUCTION PHASE

The induction phase can be controlled by genetic modifications additional genetic modifications. The key enzymes triggered during this phase are engineered using promoters that are sensitive to (*e.g.*, activated by or deactivated by) the depletion of the initiating reactant. As a result, once the initiating reactant is depleted, the growth phase ends, and the additional enzymes are activated and the induction phase begins.

The induction phase can be controlled by genes that encode for enzymes in the biosynthetic pathway for the product by using promoters that are activated by for example, phosphate depletion. When desiring to produce 3-HP, *E. coli* can be genetically modified by manipulating one or more of the following genes: mcr, mmsB, ydfG, rutE, nemA and NDSD; genes that encode individual or fused subunits of ACCase, such as accA, accB, accC, accD, accDA fusion, and accCB fusion, and the promoters may include one or more of the promoters that direct expression of the following *E. coli* genes: amn, tktB, xasA, yibD, ytfK, pstS, phoH, phnC, or other phosphate-regulated genes.

### EXAMPLE 15: THE PRODUCTION PHASE

The production phase may also be controlled by genetic modifications. For example, the organism is engineered to include mutated forms of enzymes for initiating production of the chemical product. These initiation enzymes facilitate initiation of production either by: (1) becoming active and serving a key function in the production pathway; and/or (2) becoming inactive and thereby turning off a branch pathway or other competitive pathway that prevents or limits the production pathway leading to the chemical product, for example, 3-HP.

For example, the initiation enzymes are mutated to form temperature sensitive variants of the enzymes that are either activated by or deactivated at certain temperatures. As a result, the production phase is initiated by changing the changing the temperature within the reaction vessel. For example, the production phase is controlled by genetically modifying the organism with a heterologous nucleotide sequence encoding for one or more of the following temperature sensitive enzymes: fabI^{ts} (SEQ ID NO. 27), fabB^{ts} (SEQ ID NO.28) and fabD^{ts} (SEQ ID NO. 29). These enzymes are deactivated or shut-off at the desired temperature for production of the chemical product. Once the reaction vessel temperature is changed, the temperature sensitive enzymes are deactivated and the production pathway of the chemical product is ramped up.

### EXAMPLE 16: CHEMICAL PRODUCTION

In this example, a genetically modified organism is produced, that is capable of converting a renewable carbon source to a chemical product. The genetically modified organism requires inorganic phosphate for growth and comprises at least one heterologous gene whose expression is regulated by a promoter sensitive to inorganic phosphate levels within a culture system. The at least one heterologous gene converts a carbon source to a chemical product and is not required for the genetically modified organism to replicate. The genetically modified organism also comprises a gene encoding a temperature-sensitive enzyme.

In this example, a culture system comprising a carbon source in an aqueous medium and the genetically modified organism is created. The culture system is maintained under conditions that allow the genetically modified microorganism to replicate. A sufficient level of inorganic phosphate within said culture system is also maintained. However, once the inorganic phosphate is allowed to be depleted, the expression of the gene regulated by a promoter sensitive to inorganic phosphate levels is triggered. The temperature of the culture system is also altered thereby activating or deactivating said temperature-sensitive enzyme and initiating the production of the desired chemical product.

### EXAMPLE 17: PRODUCING 3-HP

A genetically modified organism capable of converting a renewable carbon source to 3-HP is produced. The genetically modified organism comprises at least one heterologous gene whose expression is regulated by a promoter sensitive to inorganic phosphate levels within a culture system. The specific gene regulated by the phosphate sensitive promoter is selected from the group consisting of mcr, mmsB, ydfG, rutE, nemA, NDSD, accA, accB, accC, accD, accDA fusion, and accCB fusion. Also included in the genetically modified organism is a gene encoding a temperature-sensitive enzyme selected from the group consisting of fabI, fabB and fabD.

The genetically modified organism is combined with a carbon source in an aqueous medium comprising phosphate. This initiates the growth phase during which the genetically modified microorganism replicates. The level of inorganic phosphate is maintained until the desired level of cell growth is achieved. By allowing the inorganic phosphate to become depleted, initiates an induction phase which begins the expression of said gene regulated by a promoter sensitive to inorganic phosphate levels. Additionally, changing the temperature of the culture system, activates or deactivates said temperature-sensitive enzyme and initiating a growth phase during which said genetically modified microorganism produces 3-HP.

### EXAMPLE 18: FERMENTATION CONDITIONS

The growth phase can last between about 10 to about 40 hours, or about 15 to about 35 hours, or about 20 to about 30 hours. The induction phase can last between about 1 to about 6 hours, about 1 to about 5 hours, or about 2 to about 4 hours. The production phase can last from and from about 5, 10, 15, 20, 25, 30, 35, 40, 45, 50, 55, 60, 65, 70, 75, 80, 85, 90, 95, or 100 hours depending on the amount of chemical product that is desired.

The growth phase and induction phase are conducted at a temperature of about 25°C to about 35°C, about 28°C to about 32°C, or about 30 °C. The production phase is conducted at a temperature of about 35°C to about 45°C, about 35°C to about 40°C, or about 36°C to about 38 °C. The production phase temperature is typically higher than the induction phase temperature, and the increase in temperature that initiates the production phase occurs over a period of about 1 to about 5 hours, about 1 to about 3 hours, about 2 hours, or about 1 hour.

Depending on the host cell fermentation may be performed under aerobic, microaerobic, or anaerobic conditions, with or without agitation.

Suitable pH ranges for fermentation depend on the multiple factors such as the host cell. In some applications of the invention fermentation can occur between various pH ranges for example, pH 3.0 to pH 4.0, pH 4.0 to pH 5.0, pH 5.0 to pH 6.0, pH 6.0 to pH 7.0, pH 7.0 to pH 8.0, pH 8.0 to pH 9.0, or pH 9.0 to pH 10.0.

### EXAMPLE 19: pH VALUES ON 3-HP INHIBITION

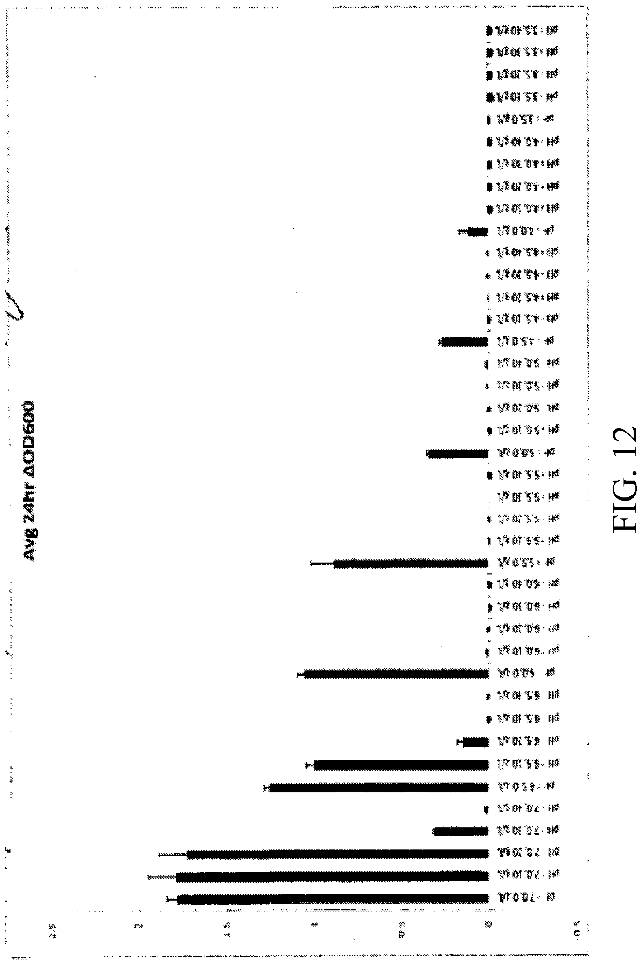

At pH 7.0, growth occurs up to about 30gr/L3-HP. However, at lower pHs growth in 3-HP becomes more inhibitory. As a result several pH and 3-HP values were screened in this example and compared to controls in order to possibly give greater sensitivity within these assays. In order to do this part of the evaluation, buffered media were evaluated for their overall effect, and then selected for use to evaluate the effect of different pH values on 3-HP inhibition on a microorganism (with or without the genetic modification introducing one or more nucleic acids encoding the proteins described herein). *See* FIG. 12.

### EXAMPLE 20: STRUCTURAL CONSIDERATIONS

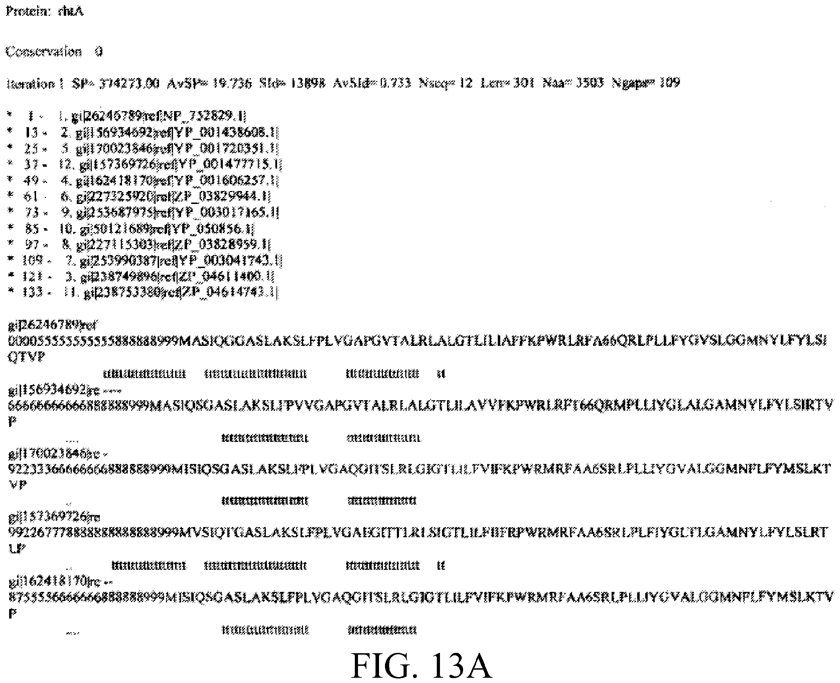

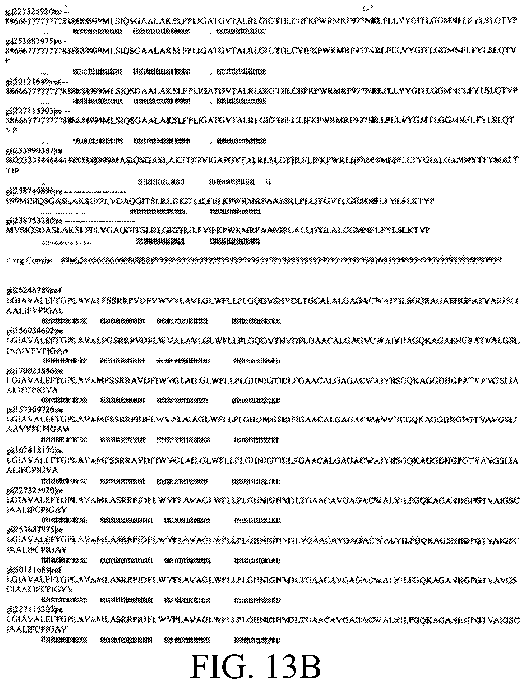

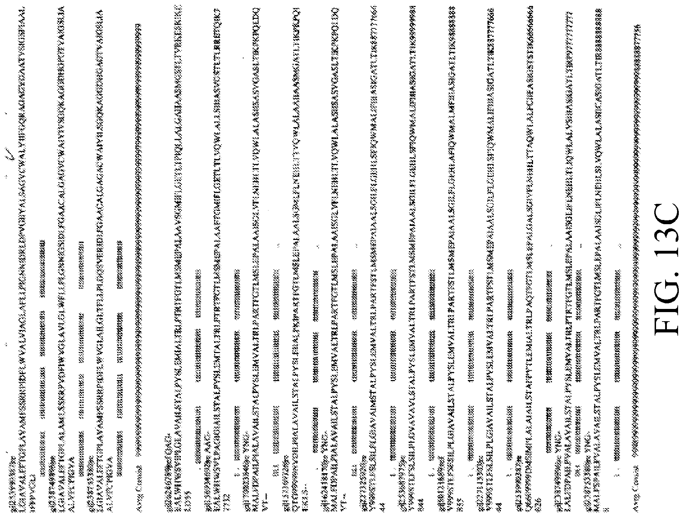

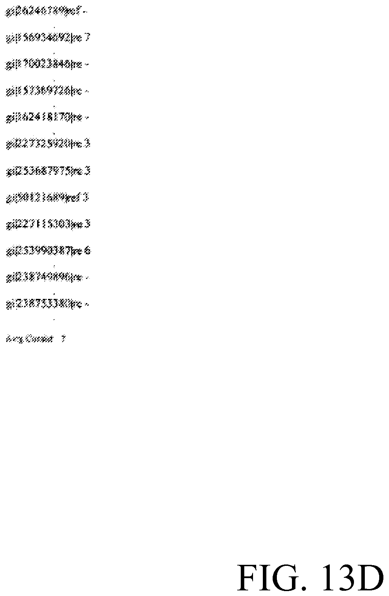

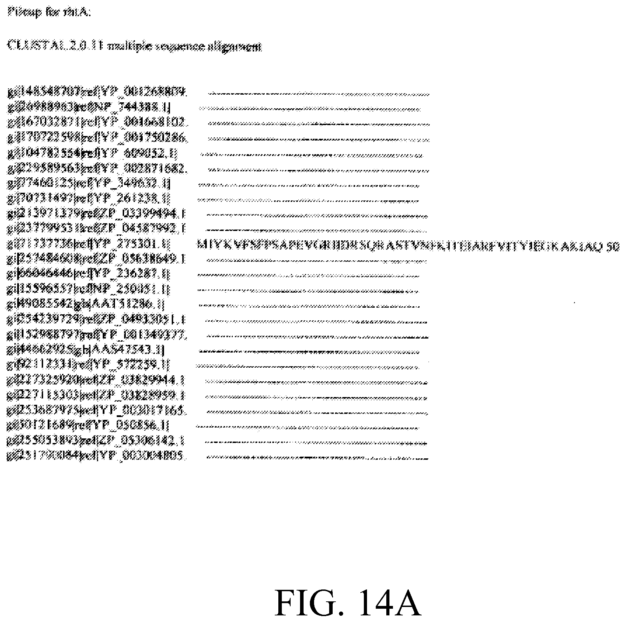

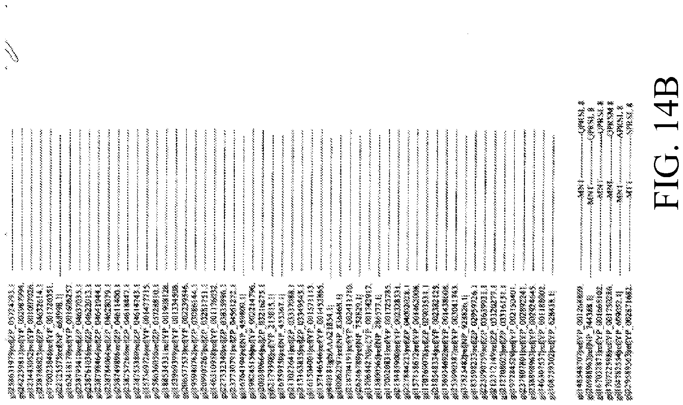

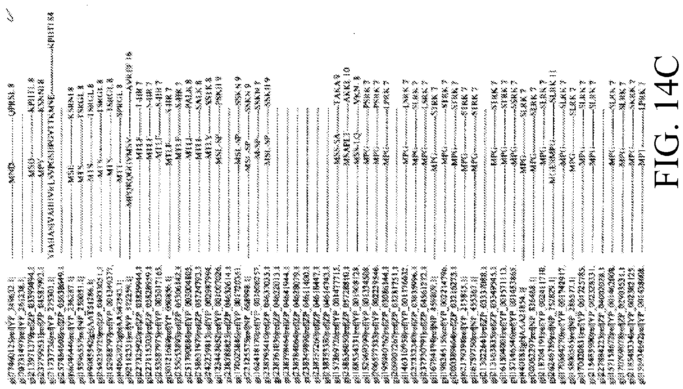

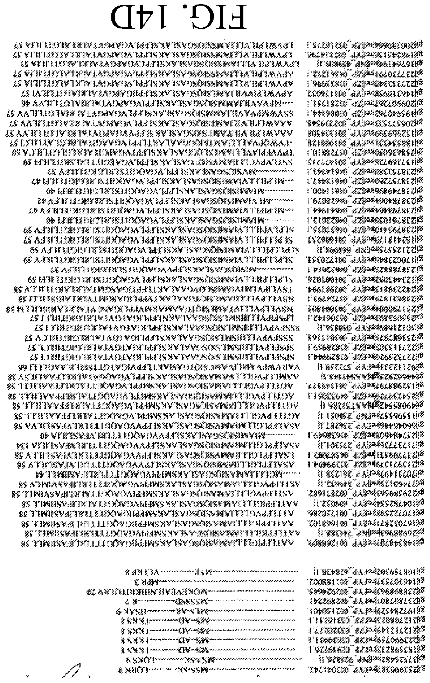

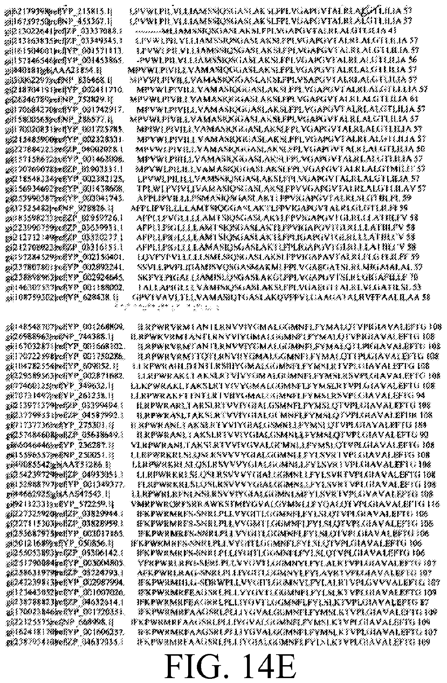

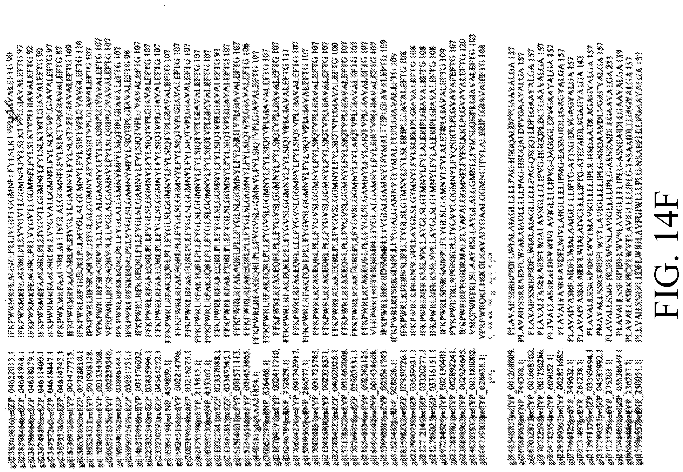

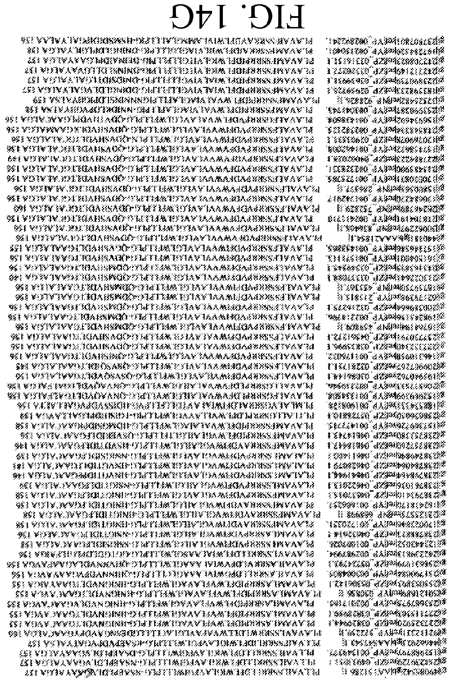

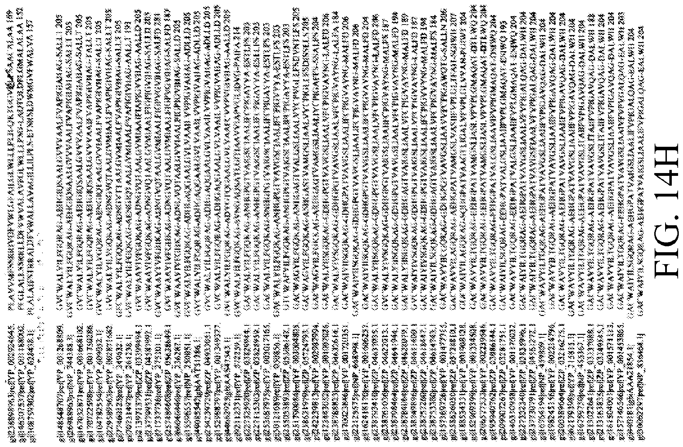

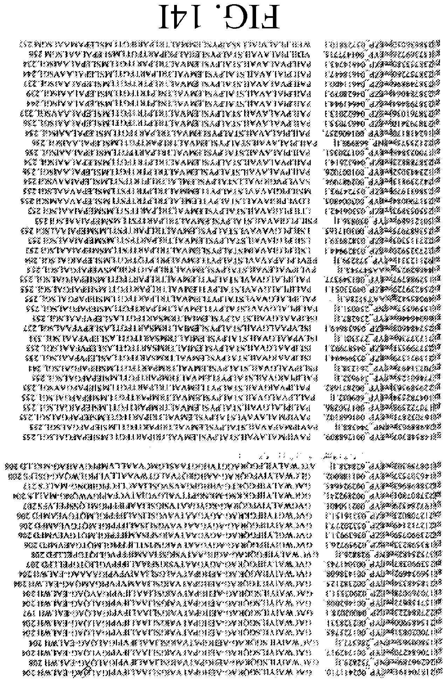

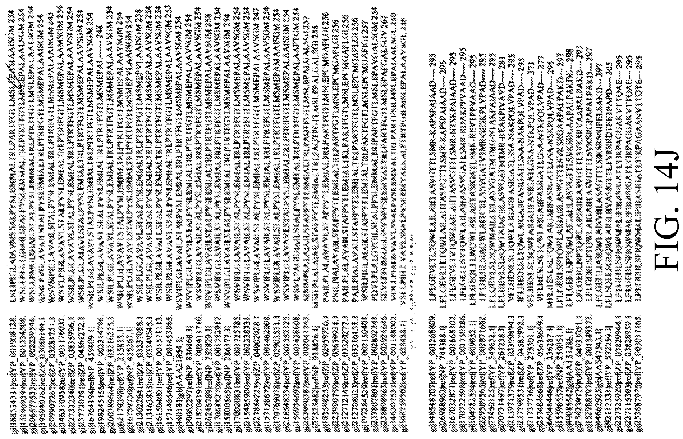

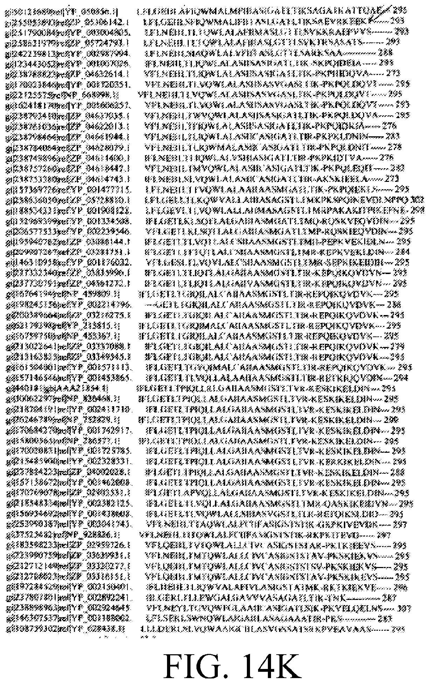

MF family proteins typically exhibit common transmembrane spanning structures, although there are varying reports of whether these protein work as monomers, dimer, or trimers. In the case of rhtA, multiple sequence alignments appear to show 10 transmembrane stretches (FIGs. 13A-D). Pileup sequence comparisons for directing protein evolution experiments were conducted (FIGs. 14A-K). Thus, site-saturated mutagenesis for perturbing functional aspects of the protein at specific spots without altering essential residues, are performed. Random mutagenesis are also performed to yield additional mutations for increasing function.

### EXAMPLE 21: EFFLUX PROTEINS

Accessory protein(s) known to work with these efflux proteins, are increased (*e.g.*, increased performance, expression and/or levels). For example, some accessory proteins include, but are not limited to, out membrane proteins belonging to the Tol family (such as talC) and Omp family (such as oprM).

### Cloning of potential efflux protein genes

The following genes were PCR amplified from genomic *E.coli* K12 DNA using a phosphorylated 5' containing forward primer and reverse primer containing both SnaBI and SphI sites:

| | | |
|---|---|---|
| rhtA | emaA | cusA |
| rhtB | emaB | Bcr |
| rhtC | emrKY | |

Two backbones were prepared by PCR amplification. The first contained a Ptac promoter with ribosome binding site using a pKK113 vector as template. The second backbone contains Psh and Ptrc promoters and a ribosome binding site at one end and a SphI site on the opposing end. The template for this PCR was a pJ284:29990 vector produced by the gene synthesis services of DNA2.0.

For the pKK223 backbone, inserts and backbone were gel purified and blunt end ligated together. Colonies were screened, and plasmids showing positive insertions were cultured and mini prepped. Finally the insets were check for proper orientation and will be sent for sequencing.

For pJ284 backbones, inserts and backbone pieces were digested with SphI, gel purified, and semi-directly ligated together. Colonies were screened, and plasmids showing positive insertions were cultured and miniprepped. Finally the insets were check for proper orientation and will be sent for sequencing.

Plasmids of each efflux protein are transformed into BW25113 strains, as well as their corresponding Kieo collection strain containing the Knockout of these genes. In other words, efflux proteins able to transport 3-HP are indentified.

Strains carrying the plasmids, and control strain (wild type and Keio deletion strains) are cultured in M9 media at several (2 to 3) of 3-HP and pH (2 to 3 levels). OD is checked at 600nm to evaluate growth (using IPTG were appropriate). Cultures showing growth are plated and isolated.

### EXAMPLE 22: MUTAGENESIS FOR INCREASED TOLERANCE TO 3-HP

Efflux proteins showing the most initial tolerance for 3-HP are subject to random mutagenesis and/or site-directed enzyme evolution to increase functionality for increased tolerance to 3-HP. Random mutagenesis are conducted by epPCR (error prone) and site specific mutagenesis is performed by site-saturation mutagenesis.

Efficient screening is accomplished by plating transformed cultures on minimal media plates prepared at increasing 3-HP levels and various pH values.

The best growing colonies are cultured and mutations are sequenced to determine the set of mutations conferring better fitness to 3-HP tolerance. The plasmids carrying gene conferring increased fitness are carried forward to repeated screen to develop greater tolerance in strains, and multiple combinations of the isolated mutations are combined.

Efflux proteins are also evaluated *in vitro.* In such a case, a simple membrane extraction protocol is used where by bacterial membranes are isolated and extruded into vesicles. These vesicles are used to collect 3-HP under the right conditions in an assay similar to that disclosed in Rayman MK, Lo TC, Sanwal BD. Transport of succinate in Escherichia coli. II. Characteristics of uptake and energy coupling with transport in membrane preparations. J. Biol. Chem. 1972 Oct 10;247(19):6332-9.

### EXAMPLE 23: DOWNSTREAM CHEMICAL PRODUCTS AND CONSUMER PRODUCTS

3-HP purified according to the methods provided in this disclosure are converted to various other products having industrial uses including, but not limited to, acrylic acid, acrylates (*e.g.,* acrylic acid salts and esters), 1,3-propanediol, malonic acid, ethyl-3-hydroxypropionate, ethyl ethoxy propionate, propiolactone, acrylamide, or acrylonitrile, and other chemicals, collectively referred to as "downstream chemical products" or "downstream products." For example, 3-HP is produced and converted to polymerized-3-HP (poly-3-HP) or acrylic acid. 3-HP or acrylic acid are also used to produce polyacrylic acid (polymerized acrylic acid, in various forms), methyl acrylate, acrylamide, acrylonitrile, propiolactone, ethyl 3-HP, malonic acid, 1,3-propanediol, ethyl acrylate, n-butyl acrylate, hydroxypropyl acrylate, hydroxyethyl acrylate, isobutyl acrylate, 2-ethylhexyl acrylate, and acrylic acid or an acrylic acid ester to which an alkyl or aryl addition are made, and/or to which halogens, aromatic amines or amides, and aromatic hydrocarbons are optionally added. In some instances the conversion is associated with the separation and/or purification steps.

The downstream chemical products are useful for producing a variety of consumer products. The methods of the present invention include steps to produce downstream products of 3-HP.

Numerous approaches are employed for such downstream conversions, generally falling into enzymatic, catalytic (chemical conversion process using a catalyst), thermal, and combinations thereof (including some wherein a desired pressure is applied to accelerate a reaction). For example, without being limiting, acrylic acid is made from 3-HP via a dehydration reaction, methyl acrylate is made from 3-HP via dehydration and esterification, the latter to add a methyl group (such as using methanol), acrylamide is made from 3-HP via dehydration and amidation reactions, acrylonitrile is made via a dehydration reaction and forming a nitrile moiety, propiolactone is made from 3-HP via a ring-forming internal esterification reaction, ethyl-3-HP is made from 3-HP via esterification with ethanol, malonic acid is made from 3-HP via an oxidation reaction, and 1,3-propanediol is made from 3-HP via a reduction reaction. Various derivatives of the derivatives of 3-HP and acrylic acid are also made, such as the various known polymers of acrylic acid and its derivatives. Additionally, copolymers containing acrylic acid and/or esters have been widely used in the pharmaceutical formulation to achieve extended or sustained release of active ingredients, for example as coating material. Downstream compounds may also be converted to consumer products such as diapers, carpet, paint, and adhesives.

3-HP is oligomerized or polymerized to form poly(3-hydroxypropionate) homopolymers, or co-polymerized with one or more other monomers to form various co-polymers. Because 3-HP has a single stereoisomer, polymerization of 3-HP is not complicated by the stereospecificity of monomers during chain growth.

3-HP is converted into derivatives starting (i) substantially as the protonated form of 3-hydroxypropionic acid; (ii) substantially as the deprotonated form, 3-hydroxypropionate; or (iii) as mixtures of the protonated and deprotonated forms. Generally, the fraction of 3-HP present as the acid versus the salt will depend on the pH, the presence of other ionic species in solution, temperature (which changes the equilibrium constant relating the acid and salt forms), and, to some extent, pressure. Chemical conversions are carried out from either of the 3-HP forms.

Acrylic acid obtained from 3-HP purified by the methods described in this disclosure can be further converted to various polymers. For example, the free-radical polymerization of acrylic acid takes place by polymerization methods known to the skilled worker and is carried out, for example, in an emulsion or suspension in aqueous solution or another solvent. Initiators, such as but not limited to organic peroxides, are optionally added to aid in the polymerization. Among the classes of organic peroxides that are used as initiators are diacyls, peroxydicarbonates, monoperoxycarbonates, peroxyketals, peroxyesters, dialkyls, and hydroperoxides. Another class of initiators is azo initiators, which are used for acrylate polymerization as well as co-polymerization with other monomers.

Co-monomers, such as crosslinkers, are also optionally present during the polymerization. The free-radical polymerization of the acrylic acid obtained from the dehydration of 3-HP, as produced herein, in at least partly neutralized form and in the presence of crosslinkers is also prepared. This polymerization may, in some case, result in hydrogels which are then comminuted, ground and, where appropriate, surface-modified.

Superabsorbent polymers are prepared from acrylic acid (such as acrylic acid derived from 3-HP provided herein) and a crosslinker, by solution or suspension polymerization. This superabsorbent polymer is used in the manufacture of products requiring absorbents for water and aqueous solutions, such as for diapers, adult incontinence products, feminine hygiene products, and similar consumer products. The superabsorbent polymer particles are also surface-modified to produce a shell structure with the shell being more highly cross-linked than the rest of the particle. This technique improves the balance of absorption, absorption under load, and resistance to gel-blocking. Superabsorbent polymers are also adapted for use in agriculture, horticulture, and medicine.

When making diapers and other personal hygiene products, the superabsorbent polymer first is molded into an absorbent pad that are vacuum formed, and in which other materials, such as a fibrous material (*e.g.*, wood pulp) are added. The absorbent pad then is assembled with sheet(s) of fabric, generally a nonwoven fabric (*e.g.*, made from one or more of nylon, polyester, polyethylene, and polypropylene plastics) to form diapers.

Multiple pressurized nozzles, located above a conveyer belt, spray superabsorbent polymer particles (*e.g.*, about 400 micron size or larger), fibrous material, and/or a combination of these onto the conveyer belt at designated spaces/intervals. The conveyor belt is perforated and under vacuum from below, so that the sprayed on materials are pulled toward the belt surface to form a flat pad. In various embodiments, fibrous material is applied first on the belt, followed by a mixture of fibrous material and the superabsorbent polymer particles, followed by fibrous material, so that the superabsorbent polymer is concentrated in the middle of the pad. A leveling roller is used toward the end of the belt path to yield pads of uniform thickness. Each pad thereafter are further processed, such as to cut it to a proper shape for the diaper, or the pad is in the form of a long roll sufficient for multiple diapers. Thereafter, the pad is sandwiched between a top sheet and a bottom sheet of fabric (one generally being liquid pervious, the other liquid impervious), for example, on a conveyor belt, and these are attached together, for example by gluing, heating or ultrasonic welding, and cut into diaper-sized units (if not previously so cut). Additional features are optionally provided, such as elastic components, strips of tape, etc., for fit and ease of wearing by a person.

Other disposable absorbent articles are constructed in a similar fashion, such as absorbent articles for adult incontinence, feminine hygiene (sanitary napkins), tampons, etc.

Low molecular weight polyacrylic acid made by the methods described herein, are used for water treatment, for example, as a flocculant and thickener for various applications including cosmetics and pharmaceutical preparations. For these applications, the polymer is uncrosslinked or lightly cross-linked. The molecular weights are typically from about 200 to about 1,000,000 g/mol.

Acrylic acid is also co-polymerized with one or more other monomers selected from the group consisting of acrylamide, 2-acrylamido-2-methylpropanesulfonic acid, N,N-dimethylacrylamide, N-isopropylacrylamide, methacrylic acid, and methacrylamide. Co-monomers are also derived from 3-HP, or otherwise provided, to produce co-polymers.

Acrylic acid is also copolymerized with any free-radically polymerizable monomers including, but not limited to, styrene, butadiene, acrylonitrile, acrylic esters, maleic acid, maleic anhydride, vinyl chloride, acrylamide, and itaconic acid. Acrylic acid (or one of its co-polymerization monomers) are also substituted by any substituent that does not interfere with the polymerization process, such as alkyl, alkoxy, aryl, heteroaryl, benzyl, vinyl, allyl, hydroxy, epoxy, amide, ethers, esters, ketones, maleimides, succinimides, sulfoxides, glycidyl and silyl.

Acrylic acid and its esters are also formed into paints. A desired mixture of homopolymers and/or copolymers, referred to in the paint industry as "vehicle" (or "binder") are added to an aqueous solution and agitated sufficiently to form an aqueous dispersion that includes sub-micrometer sized polymer particles. The paint cures by coalescence of these vehicle particles as the water and any other solvent evaporate. Other additives to the aqueous dispersion include, but are not limited to, pigment, filler (*e.g.*, calcium carbonate, aluminum silicate), solvent (*e.g.*, acetone, benzol, alcohols, etc., although these are not found in certain no VOC paints), thickener, and additional additives depending on the conditions, applications, intended surfaces, etc.

Acrylic-based polymers are used for many coatings in addition to paints. For example, for paper coating latexes, acrylic acid is used from 0.1-5.0%, along with styrene and butadiene, to enhance binding to the paper and modify rheology, freeze-thaw stability and shear stability. Acrylate-based polymers also are used in many inks, particularly UV curable printing inks. For water treatment, acrylamide and/or hydroxy ethyl acrylate are commonly co-polymerized with acrylic acid to produce low molecular-weight linear polymers. Co-polymers of acrylic acid with maleic acid or itaconic acid are also produced for water-treatment applications. Sodium acrylate (the sodium salt of glacial acrylic acid) are also co-polymerized with acrylamide (which may be derived from acrylic acid via amidation chemistry) to make an anionic co-polymer that is used as a flocculant in water treatment.

For thickening agents, a variety of co-monomers are used.

Conversion to downstream products are also made enzymatically. For example, 3-HP are converted to 3-HP-CoA, which then is converted into polymerized 3-HP with an enzyme having polyhydroxy acid synthase activity (EC 2.3.1.-). Also, 1,3-propanediol is made using polypeptides having oxidoreductase activity or reductase activity (*e.g.* , enzymes in the EC 1.1.1.- class of enzymes). Alternatively, when creating 1,3-propanediol from 3-HP, a combination of (1) a polypeptide having aldehyde dehydrogenase activity (*e.g.*, an enzyme from the 1.1.1.34 class) and (2) a polypeptide having alcohol dehydrogenase activity (*e.g.*, an enzyme from the 1.1.1.32 class) are used. Polypeptides having lipase activity are used to form esters. Enzymatic reactions such as these are conducted *in vitro,* such as using cell-free extracts, or *in vivo.*

Reactions that form downstream compounds such as acrylates or acrylamides are conducted in conjunction with use of suitable stabilizing agents or inhibiting agents reducing the likelihood of polymer formation. Stabilizing agents and/or inhibiting agents including, but not limited to, *e.g.,* phenolic compounds (*e.g.,* dimethoxyphenol (DMP) or alkylated phenolic compounds such as di-tert-butyl phenol), quinones (*e.g.*, t-butyl hydroquinone or the monomethyl ether of hydroquinone (MEHQ)), and/or metallic copper or copper salts (*e.g.,* copper sulfate, copper chloride, or copper acetate), are used. Inhibitors and/or stabilizers are also used individually or in combinations.

The one or more downstream compounds are also recovered at a molar yield of up to about 100 percent, or a molar yield in the range from about 70 percent to about 90 percent, or a molar yield in the range from about 80 percent to about 100 percent, or a molar yield in the range from about 90 percent to about 100 percent. Such yields are the result of single-pass (batch or continuous) or iterative separation and purification steps in a particular process.

In an embodiment of the invention, a microorganism comprises at least 10 contiguous polynucleotides or amino acids of any one of SEQ ID NOs: 1-100and/or comprises a polynucleotide or polypeptide having at least: 70%, homology to a polynucleotide or polypeptide of any one of SEQ ID NOS; 1-100. In any embodiment of the disclosure, a microorganism can comprise a polynucleotide coding for an MCR having at least about: 50%, 55%, 60%, 65%, 70%, 75%, 80%, 85%, 90%, 91%, 92%, 93%, 94%, 95%, 96%, 97%, 98%, or 99% homology to caMCR, stMCR, oaMCR and/or otMCR. In any embodiment herein, a microorganism can comprise a polynucleotide coding for ACCase or an ACCase and/or any subunit thereof having at least about: 50%, 55%, 60%, 65%, 70%, 75%, 80%, 85%, 90%, 91%, 92%, 93%, 94%, 95%, 96%, 97%, 98% or 99% homology to an ACCase or any subunit any subunit thereof. In an embodiment of the invention, the genetically modified microorganism has at least one disrupted gene selected from the group consisting of: araD, araB, lacZ, rhaD, rhaB, hsdR, ldhA, pflB, mgsA, poxB, pta-ack, fabI, fabB, fabF, fabD, aldA, aldB, puuC, and any combination thereof. In any embodiment of the disclosure, the genetically modified microorganism can comprise an exogenous polynucleotide encoding a malonyl-CoA reductase. In any embodiment of the disclosure, the malonyl-CoA reductase can be monofunctional or bifunctional (e.g., monofunctional reduces malonyl-CoA to malonate semialdehyde, and bifunctional reduces malonyl-CoA to 3-HP. In any embodiment of the disclosure, the genetically modified microorganism herein can comprise an acetyl-CoA carboxylase or one or more subunits thereof and/or a polynucleotide encoding these.

Acrylamide is also produced from 3-HP, for example, via an esterification-amidation-dehydration sequence. Refluxing an alcohol solution of 3-HP in the presence of an acid or Lewis acid catalyst described herein leads to an 3-HP ester. Treatment of the 3-HP ester with either an ammonia gas or an ammonium ion yields 3-HP amide. Finally, dehydration of the 3-HP amide with dehydration reagents described produces acrylamide. The steps mentioned herein are optionally rearranged to produce the same final product acrylamide. Polymerization of acrylamide is also achieved, for example, by radical polymerization. Polyacrylamide polymers are used as additives for treating municipal drinking water and waste water, in addition to applications in gel electrophoresis, oil-drilling, papermaking, ore processing, and the manufacture of permanent press fabrics.

### SEQUENCE LISTING

SEQ ID NO. 1: ACCA PROTEIN, HALOMONAS ELONGATA
MNPNYLDFEQPIAELQAKIEELRMVGNDSQVNLSDEIGRLEEKSRKLTES
IFKDLSAWQVSQLSRHPQRPYTLDYLEHVFTDFDELHGDRRFADDAAIVG
GVARLDDKPVMVIGHQKGRDVHEKVRRNFGMPRPEGYRKACRLMEMAERF
HMPVLTFIDTPGAYPGIDAEERGQSEAIAYNLGVMSRLKTPIISTVVGEG
GSGGALAIGVCDELAMLQYSTYSVISPEGCASILWKSADKASEAAQAMGI
TAERLKELGFVDTLIPEPLGGAHRQPSATAERIKTALLESLDRLETMETD
ALLERRYERLMSYGAPV
SEQ ID NO. 2: ACCA NUCLEIC ACID, HALOMONAS ELONGATA (SYNTHETIC, CODON
OPTIMIZE FOR E. COLI EXPRESSION)
ATGAATCCGAACTATCTGGACTTTGAACAACCGATCGCTGAACTGCAAGCCAAAATCGAA
GAACTGCGTATGGTGGGCAACGACTCACAGGTGAACCTGTCTGATGAAATTGGCCGTCTG
GAAGAAAAAAGTCGCAAACTGACCGAATCCATCTTTAAAGACCTGTCAGCGTGGCAAGTT
AGCCAACTGTCTCGTCATCCGCAACGCCCGTATACCCTGGATTACCTGGAACATGTCTTT
ACGGATTTCGACGAACTGCACGGTGACCGTCGCTTTGCAGATGACGCGGCCATTGTTGGC
GGTGTCGCTCGTCTGGATGACAAACCGGTCATGGTGATCGGCCATCAGAAAGGTCGTGAT
GTGCACGAAAAAGTTCGTCGCAACTTCGGCATGCCGCGCCCGGAAGGTTATCGTAAAGCG
TGCCGCCTGATGGAAATGGCCGAACGCTTTCACATGCCGGTGCTGACCTTCATTGATACG
CCGGGCGCATATCCGGGTATCGACGCTGAAGAACGTGGCCAAAGCGAAGCGATTGCCTAC
AATCTGGGTGTTATGTCGCGCCTGAAAACCCCGATTATCAGCACGGTGGTTGGCGAAGGC
GGTTCTGGCGGTGCACTGGCTATCGGTGTCTGCGATGAACTGGCGATGCTGCAATATAGT
ACCTACTCCGTGATTTCACCGGAAGGCTGTGCCTCGATCCTGTGGAAAGCGCAGATAAA
GCTTCTGAAGCAGCTCAAGCGATGGGCATTACCGCCGAACGTCTGAAAGAACTGGGTTTC
GTTGACACGCTGATCCCGGAACCGCTGGGCGGTGCACATCGTCAGCCGAGTGCGACCGCC
GAACGCATTAAAACGGCCCTGCTGGAAAGCCTGGATCGCCTGGAAACGATGGAAACGGAT
GCCCTGCTGGAACGCCGCTATGAACGCCTGATGTCTTACGGTGCCCCGGTCTGA
SEQ ID NO. 3: ACCB PROTEIN, HALOMONAS ELONGATA
MDIRKVKKLIELLEESNISEIEIQEGEESVRISRHPNGTEHPQPAAPAWP
ATAAAPAPQPAAAPVESPAEVDEGPAYQGQPIVSPMVGTFYRAPAPGAKA
FVELGQSVKKGETVCIVEAMKMMNQIEADRDGVVEAILVEDGEPVEFEQP

MVVIS
SEQ ID NO. 4: ACCB NUCLEIC ACID HALOMONAS ELONGATA (SYNTHETIC, CODON
OPTIMIZE FOR E. COLI EXPRESSION)
ATGGACATCCGCAAAGTGAAAAAACTGATCGAACTGCTGGAAGAAAGTAACATCTCTGAA
ATTGAAATCCAAGAAGGCGAAGAAAGCGTGCGTATTAGTCGCCATCCGAACGGCACCGAA
CACCCGCAGCCGGCGGCACCGGCATGGCCGGCCACGGCAGCTGCGCCGGCGCCGCAACCG
GCCGCAGCTCCGGTGGAAAGCCCGGCAGAAGTTGATGAAGGCCCGGCTTATCAGGGTCAA
CCGATCGTTTCTCCGATGGTCGGCACCTTTTACCGTGCGCCGGCACCGGGTGCAAAAGCT
TTCGTCGAACTGGGCCAGAGCGTTAAAAAAGGTGAAACGGTCTGCATTGTGGAAGCCATG
AAAATGATGAATCAAATCGAAGCCGATCGCGACGGTGTGGTTGAAGCAATCCTGGTGGAA
GATGGTGAACCGGTGGAATTTGAACAGCCGATGGTGGTGATTAGTTAA
SEQ ID NO. 5: ACCC PROTEIN, HALOMONAS ELONGATA
MLDKVLIANRGEIALRILRACKELGIRTVAVHSKADRELMHVRLADEAVC
IGPASSAQSYLNIPALISAAEVTDTSAIHPGYGFLSENADFAEQVERSGF
TFIGPSAETIRLMGDKVSAINAMKEAGVPTVPGSNGPLGDDEGEILATAR
RIGYPVIIKAAAGGGGRGMRVVHAEGHLLSAVNVTRTEAHSSFGDGTVYM
EKFLENPRHVEVQVLADGQGNAIHLYDRDCSLQRRHQKVLEEAPAPGLDQ
QAREQVFKACRDACVKIGYRGAGTFEFLYENGEFFFIEMNTRVQVEHPVT
EMVTGVDIVREQLRIASGLPLSIRQEDVELSGHAFECRINAEDSRTFMPS
PGRVTLYHPPGGLGVRMDSHVYTGYTVPPHYDSLIGKLITWGDDRETALI
RMRNALDELLVEGIKTNTDLHKDLVRDGYFQQGGVNIHYLEKKLGL
SEQ ID NO. 6: ACCC NUCLEIC ACID, HALOMONAS ELONGATA (SYNTHETIC, CODON
OPTIMIZE FOR E. COLI EXPRESSION)
ATGCTGGACAAAGTGCTGATTGCGAATCGTGGCGAAATTGCGCTGCGTATCCTGCGTGCG
TGTAAAGAACTGGGTATCCGTACCGTCGCTGTTCATTCAAAAGCGGACCGTGAACTGATG
CACGTCCGCCTGGCCGATGAAGCAGTGTGCATTGGTCCGGCTAGCTCTGCGCAGTCGTAT
CTGAACATTCCGGCACTGATCAGTGCGGCCGAAGTGACCGACACGTCCGCGATCCATCCG
GGCTACGGTTTCCTGAGCGAAAATGCCGATTTTGCAGAACAAGTCGAACGTTCAGGTTTC
ACCTTTATTGGCCCGTCGGCCGAAACGATCCGCCTGATGGGTGATAAAGTTAGTGCTATT
AACGCGATGAAAGAAGCAGGCGTGCCGACCGTTCCGGGTTCCAATGGTCCGCTGGGTGAT
GACGAAGGTGAAATTCTGGCCACCGCACGTCGCATCGGCTATCCGGTTATTATCAAAGCA
GCTGCGGGCGGTGGCGGTCGTGGTATGCGTGTGGTTCATGCTGAAGGCCACCTGCTGAGC

GCGGTCAACGTGACCCGTACGGAAGCGCATAGTTCCTTCGGCGATGGCACCGTTTATATG
GAAAAATTTCTGGAAAACCCGCGTCACGTTGAAGTCCAGGTGCTGGCCGATGGCCAGGGT
AATGCAATTCATCTGTACGATCGCGACTGCTCTCTGCAACGTCGCCACCAAAAAGTGCTG
GAAGAAGCTCCGGCACCGGGTCTGGACCAGCAAGCACGTGAACAGGTTTTTAAAGCCTGC
CGCGATGCATGTGTCAAAATTGGTTATCGTGGCGCGGGCACCTTCGAATTTCTGTACGAA
AACGGCGAATTTTTCTTTATCGAAATGAATACGCGCGTTCAGGTCGAACATCCGGTGACC
GAAATGGTCACGGGTGTGGATATTGTTCGTGAACAGCTGCGTATCGCATCAGGTCTGCCG
CTGTCGATTCGCCAAGAAGACGTTGAACTGAGCGGTCATGCCTTCGAATGTCGTATCAAT
GCAGAAGATAGCCGCACCTTTATGCCGTCTCCGGGTCGTGTCACGCTGTATCACCCGCCG
GGCGGTCTGGGTGTCCGTATGGACAGCCATGTGTATACCGGCTACACGGTTCCGCCGCAC
TACGATTCTCTGATTGGTAAACTGATCACCTGGGGCGATGACCGTGAAACGGCTCTGATT
CGTATGCGCAACGCCCTGGATGAACTGCTGGTTGAAGGCATCAAAACCAATACGGATCTG
CACAAAGACCTGGTTCGCGATGGCTACTTTCAGCAAGGCGGTGTCAACATTCACTACCTG
GAAAAAAAACTGGGTCTGTAA
SEQ ID NO. 7: ACCD PROTEIN, HALOMONAS ELONGATA
MSWLDKIVPSVGRIQRKERRTSVPDGLWRKCPKCESVLYLPELEKHHNVC
PKCDHHLRLTARKRLDWFLDKEGREEIAADLEPVDRLKFRDSKKYKDRLS
AAQKATGEKDGLVAMRGTLEGLPVVAVAFEFTFMGGSMGAVVGEKFVRAA
TQALDEGVPLVCFSASGGARMQEALFSLMQMAKTSAALEKLKQAGVPYIS
VLTDPVFGGVSASLAMLGDLNIAEPNALIGFAGPRVIEQTVREQLPEGFQ
RSEFLLEHGAVDMIVHRQQIRERLGGVLRKLTHQPASGPAVVENDEPDLV
DAAEQAEPQPEAPEAVETSESEAPTEKGVEADSEETDESPRSGDNR
SEQ ID NO. 8: ACCD NUCLEIC ACID, HALOMONAS ELONGATA (SYNTHETIC, CODON
OPTIMIZE FOR E. COLI EXPRESSION)
ATGTCTTGGTTAGATAAAATCGTCCCGTCAGTGGGTCGCATTCAACGCAAAGAACGTCGC
ACCTCGGTCCCGGATGGTCTGTGGCGTAAATGCCCGAAATGTGAATCAGTTCTGTATCTG
CCGGAACTGGAAAAACATCACAACGTCTGCCCGAAATGTGATCATCACCTGCGTCTGACC
GCGCGTAAACGCCTGGACTGGTTCCTGGATAAAGAAGGCCGCGAAGAAATTGCGGCCGAC
CTGGAACCGGTGGATCGTCTGAAATTTCGCGACAGCAAAAAATACAAAGATCGTCTGAGC
GCGGCGCAGAAAGCAACCGGTGAAAAAGACGGTCTGGTGGCCATGCGTGGCACGCTGGAA
GGTCTGCCGGTGGTTGCAGTTGCTTTTGAATTTACCTTTATGGGCGGTAGCATGGGCGCA
GTCGTGGGTGAAAAATTCGTTCGTGCGGCCACGCAGGCTCTGGATGAAGGTGTGCCGCTG

GTTTGCTTCAGCGCATCTGGCGGTGCCCGCATGCAGGAAGCACTGTTTAGTCTGATGCAA
ATGGCTAAAACCTCCGCAGCTCTGGAAAAACTGAAACAGGCGGGCGTGCCGTATATTTCT
GTTCTGACGGACCCGGTCTTCGGCGGTGTGAGTGCGTCCCTGGCCATGCTGGGTGATCTG
AACATTGCAGAACCGAATGCTCTGATCGGCTTTGCGGGTCCGCGTGTCATCGAACAGACC
GTGCGCGAACAACTGCCGGAAGGCTTCCAGCGTTCAGAATTTCTGCTGGAACATGGTGCC
GTTGATATGATTGTCCACCGTCAGCAAATCCGTGAACGCCTGGGCGGTGTGCTGCGCAAA
CTGACGCATCAACCGGCATCGGGTCCGGCCGTTGTCGAAAATGATGAACCGGACCTGGTC
GATGCGGCCGAACAGGCAGAACCGCAACCGGAAGCACCGGAAGCTGTTGAAACCTCAGAA
TCGGAAGCACCGACGGAAAAAGGCGTGGAAGCAGACTCGGAAGAAACGGATGAATCACCG
CGCTCAGGCGACAACCGCTAA
SEQ ID NO. 9: FUSION ACCCB PROTEIN, E. COLI
MLDKIVIANRGEIALRILRACKELGIKTVAVHSSADRDLKHVLLADETVC
IGPAPSVKSYLNIPAIISAAEITGAVAIHPGYGFLSENANFAEQVERSGF
IFIGPKAETIRLMGDKVSAIAAMKKAGVPCVPGSDGPLGDDMDKNRAIAK
RIGYPVIIKASGGGGGRGMRVVRGDAELAQSISMTRAEAKAAFSNDMVYM
EKYLENPRHVEIQVLADGQGNAIYLAERDCSMQRRHQKVVEEAPAPGITP
ELRRYIGERCAKACVDIGYRGAGTFEFLFENGEFYFIEMNTRIQVEHPVT
EMITGVDLIKEQLRIAAGQPLSIKQEEVHVRGHAVECRINAEDPNTFLPS
PGKITRFHAPGGFGVRWESHIYAGYTVPPYYDSMIGKLICYGENRDVAIA
RMKNALQELIIDGIKTNVDLQIRIMNDENFQHGGTNIHYLEKKLGLQEKD
IRKIKKLIELVEESGISELEISEGEESVRISRAAPAASFPVMQQAYAAPM
MQQPAQSNAAAPATVPSMEAPAAAEISGHIVRSPMVGTFYRTPSPDAKAF
IEVGQKVNVGDTLCIVEAMKMMNQIEADKSGTVKAILVESGQPVEFDEPL
VVIE
SEQ ID NO. 10: FUSION ACCCB NUCLEIC ACID, E. COLI
ATGCTGGACAAGATCGTCATCGCCAACCGCGGCGAAATCGCCCTGCGCATCTTGCGCGCG
TGTAAAGAGCTGGGCATTAAGACTGTTGCCGTGCATTCCAGCGCAGACCGCGACCTGAAG
CATGTTCTGCTGGCCGACGAAACGGTTTGCATCGGTCCGGCACCGAGCGTGAAAAGCTAT
CTGAACATCCCGGCCATCATCTCTGCGGCAGAGATCACCGGTGCAGTGGCGATTCATCCG
GGCTACGGTTTCCTGAGCGAGAACGCTAACTTTGCTGAACAAGTGGAGCGTAGCGGTTTC
ATCTTCATTGGCCCTAAGGCGGAGACGATTCGCCTGATGGGCGACAAAGTGAGCGCCATT
GCAGCGATGAAAAAGGCCGGTGTGCCGTGTGTTCCGGGCAGCGATGGTCCGCTGGGTGAC

GATATGGACAAGAACCGTGCCATCGCTAAACGTATTGGCTACCCGGTCATTATCAAAGCC
TCTGGTGGTGGCGGTGGCCGTGGTATGCGTGTCGTCCGTGGTGATGCGGAACTGGCGCAA
AGCATCAGCATGACCCGTGCGGAAGCCAAAGCGGCGTTCTCTAACGATATGGTGTATATG
GAGAAGTATCTGGAGAATCCGCGCCACGTTGAAATCCAAGTTCTGGCGGATGGTCAGGGC
AATGCGATCTACTTGGCAGAACGTGATTGCTCCATGCAACGCCGTCATCAGAAGGTGGTG
GAAGAGGCACCGGCTCCGGGTATTACGCCGGAACTGCGTCGCTACATCGGTGAGCGCTGT
GCGAAAGCGTGTGTGGACATTGGTTACCGTGGTGCGGGTACGTTTGAGTTCCTGTTCGAA
AATGGTGAGTTTTACTTCATTGAAATGAATACCCGCATCCAGGTTGAGCACCCGGTGACC
GAGATGATTACTGGCGTTGATCTGATCAAAGAGCAACTGCGCATTGCGGCTGGTCAGCCG
CTGTCGATCAAGCAAGAAGAGGTGCACGTTCGTGGTCACGCGGTCGAGTGCCGTATCAAT
GCGGAGGACCCGAATACCTTTCTGCCGAGCCCTGGTAAGATCACTCGTTTTCACGCGCCA
GGTGGTTTTGGCGTTCGTTGGGAGTCTCACATCTACGCCGGTTACACCGTGCCGCCGTAC
TATGACAGCATGATTGGTAAACTGATCTGCTATGGCGAAAATCGTGATGTCGCGATCGCC
CGCATGAAAAACGCGCTGCAAGAGCTGATCATTGATGGCATTAAGACCAATGTGGATTTG
CAGATCCGCATTATGAACGACGAGAATTTCCAGCACGGCGGTACGAACATTCACTACCTG
GAAAAGAAACTGGGCCTGCAAGAGAAAGACATCCGCAAGATCAAGAAGCTGATCGAACTG
GTGGAAGAGTCTGGCATCAGCGAGCTGGAGATCAGCGAAGGTGAAGAGAGCGTCCGTATT
TCCCGTGCGGCACCGGCAGCGAGCTTTCCGGTTATGCAGCAAGCATACGCCGCTCCGATG
ATGCAACAGCCGGCACAGAGCAACGCCGCTGCACCGGCGACCGTTCCAAGCATGGAGGCA
CCGGCAGCGGCCGAGATTTCGGGTCATATCGTGCGTAGCCCGATGGTGGGCACCTTCTAT
CGCACGCCGTCGCCGGACGCAAAAGCCTTCATCGAAGTCGGCCAGAAGGTCAATGTCGGC
GACACGCTGTGTATCGTTGAGGCAATGAAAATGATGAACCAGATTGAAGCGGATAAGAGC
GGTACTGTTAAAGCGATCCTGGTGGAATCCGGCCAGCCTGTTGAGTTCGATGAACCGCTG
GTTGTGATCGAGTAA
SEQ ID NO. 11: FUSION ACCDA PROTEIN, E. COLI
MSWIERIKSNITPTRKASIPEGVWTKCDSCGQVLYRAELERNLEVCPKCD
HHMRMTARNRLHSLLDEGSLVELGSELEPKDVLKFRDSKKYKDRLASAQK
ETGEKDALVVMKGTLYGMPVVAAAFEFAFMGGSMGSVVGARFVRAVEQAL
EDNCPLICFSASGGARMQEALMSLMQMAKTSAALAKMQERGLPYISVLTD
PTMGGVSASFAMLGDLNIAEPKALIGFAGPRVIEQTVREKLPPGFQRSEF
LIEKGAIDMIVRRPEMRLKLASILAKLMNLPAPNPEAPREGVVVPPVPDQ
EPEALSGGGGSGGGGSGGGGSGGGGSAAASLNFLDFEQPIAELEAKIDSL

TAVSRQDEKLDINIDEEVHRLREKSVELTRKIFADLGAWQIAQLARHPQR
PYTLDYVRLAFDEFDELAGDRAYADDKAIVGGIARLDGRPVMIIGHQKGR
ETKEKIRRNFGMPAPEGYRKALRLMQMAERFKMPIITFIDTPGAYPGVGA
EERGQSEAIARNLREMSRLGVPVVCTVIGEGGSGGALAIGVGDKVNMLQY
STYSVISPEGCASILWKSADKAPLAAEAMGIIAPRLKELKLIDSIIPEPL
GGAHRNPEAMAASLKAQLLADLADLDVLSTEDLKNRRYQRLMSYGYA
SEQ ID NO. 12: FUSION ACCDA NUCLEIC ACID; E. COLI
ATGAGCTGGATCGAGCGCATCAAGAGCAACATCACCCCGACCCGCAAGGCGAGCATCCCT
GAAGGCGTCTGGACCAAATGCGATAGCTGCGGTCAGGTTTTGTATCGTGCGGAGCTGGAG
CGTAACCTGGAAGTGTGCCCGAAATGCGACCATCACATGCGTATGACCGCTCGTAATCGT
CTGCATAGCCTGCTGGATGAGGGCAGCCTGGTCGAGCTGGGTAGCGAACTGGAACCGAAA
GATGTTCTGAAATTCCGTGATTCCAAGAAGTATAAGGATCGTTTGGCATCTGCACAAAAA
GAAACCGGTGAGAAGGACGCACTGGTTGTTATGAAAGGCACCCTGTATGGTATGCCGGTT
GTTGCTGCGGCGTTCGAGTTTGCGTTTATGGGTGGCAGCATGGGTTCCGTGGTGGGCGCA
CGCTTTGTGCGTGCCGTGGAGCAGGCGCTGGAGGATAACTGTCCTCTGATTTGTTTCAGC
GCGAGCGGTGGTGCGCGTATGCAAGAGGCCCTGATGAGCCTGATGCAGATGGCAAAAACC
TCGGCAGCCCTGGCGAAGATGCAAGAACGCGGCCTGCCGTACATTTCCGTCCTGACCGAC
CCTACGATGGGCGGTGTCAGCGCCAGCTTTGCGATGCTGGGTGATTTGAACATCGCAGAG
CCGAAGGCTCTGATTGGTTTTGCTGGTCCGCGTGTTATTGAACAGACGGTTCGCGAAAAG
TTGCCGCCTGGTTTCCAGCGCAGCGAGTTCCTGATTGAGAAAGGTGCCATCGACATGATC
GTTCGCCGTCCAGAAATGCGTCTGAAACTGGCGAGCATTCTGGCGAAATTGATGAATCTG
CCGGCTCCGAATCCTGAAGCACCGCGTGAGGGTGTCGTGGTTCCGCCGGTCCCGGACCAA
GAGCCGGAGGCTCTGAGCGGCGGAGGTGGCTCTGGTGGAGGCGGTTCAGGAGGCGGTGGC
AGTGGTGGCGGCGGATCTGCGGCAGCTTCTCTGAACTTCCTGGACTTCGAGCAGCCGATC
GCCGAACTGGAGGCGAAGATTGACAGCCTGACCGCGGTTAGCCGTCAAGATGAGAAACTG
GACATTAACATCGACGAAGAGGTCCACCGTTTGCGTGAGAAGTCTGTTGAACTGACTCGC
AAAATCTTTGCTGATTTGGGCGCATGGCAGATTGCCCAGTTGGCTCGCCACCCACAACGC
CCATATACCCTGGACTACGTGCGCCTGGCGTTTGACGAGTTCGACGAACTGGCAGGCGAC
CGCGCCTATGCGGACGATAAAGCAATTGTCGGCGGTATTGCTCGTTTGGATGGCCGTCCG
GTGATGATTATCGGCCATCAAAAAGGCCGCGAGACAAAGGAAAAGATTCGTCGTAATTTC
GGAATGCCGGCACCGGAGGGCTACCGCAAGGCCCTGCGTCTGATGCAAATGGCCGAACGC
TTTAAGATGCCGATTATCACGTTCATTGATACGCCTGGTGCGTACCCAGGCGTTGGTGCG

GAAGAGCGTGGTCAGAGCGAGGCCATCGCACGTAACCTGCGTGAGATGTCTCGTCTGGGT
GTGCCGGTCGTTTGCACCGTGATTGGCGAGGGCGGTAGCGGTGGTGCGTTGGCGATCGGT
GTCGGTGATAAGGTCAACATGCTGCAATACAGCACGTACAGCGTCATTAGCCCGGAAGGT
TGCGCTTCCATTCTGTGGAAGAGCGCGGATAAAGCACCATTGGCAGCGGAAGCGATGGGT
ATCATCGCACCGCGTCTGAAAGAACTGAAGTTGATTGATTCTATCATCCCGGAACCGCTG
GGCGGTGCTCACCGTAATCCGGAGGCGATGGCAGCCAGCCTGAAGGCCCAGCTGCTGGCG
GACCTGGCGGATCTGGACGTGCTGAGCACGGAGGATCTGAAAAACCGTCGCTATCAGCGC
TTGATGAGCTATGGCTACGCTTAA
SEQ ID NO. 13: BICA PROTEIN, SYNECHOCOCCUS SP.
MQITNKIHFRNIRGDIFGGLTAAVIALPMALAFGVASGAGAEAGLWGAVL
VGFFAALFGGTPTLISEPTGPMTVVMTAVIAHFTASAATPEEGLAIAFTV
VMMAGVFQIIFGSLKLGKYVTMMPYTVISGFMSGIGIILVILQLAPFLGQ
ASPGGGVIGTLQNLPTLLSNIQPGETALALGTVAIIWFMPEKFKKVIPPQ
LVALVLGTVIAFFVFPPEVSDLRRIGEIRAGFPELVRPSFSPVEFQRMIL
DAAVLGMLGCIDALLTSVVADSLTRTEHNSNKELIGQGLGNLFSGLFGGI
AGAGATMGTVVNIQSGGRTALSGLVRAFVLLVVILGAASLTATIPLAVLA
GIAFKVGVDIIDWSFLKRAHEISPKGALIMYGVILLTVLVDLIVAVGVGV
FVANVLTIERMSNLQSEKVQTVSDADDNIRLTTTEKRWLDEGQGRVLLFQ
LSGPMIFGVAKAIAREHNAMGDCDALVFDIGEVPHMGVTASLALENAIEE
ALDKERQVYIVGAAGQTRRRLEKLKLFKRVPPDKCLMSREEALKNAVLGI
YPHLADGVTAPSSEMG
SEQ ID NO. 14: BICA NUCLEIC ACID, SYNECHOCOCCUS SP.
ATGCAGATTACCAACAAGATCCATTTCCGTAACATTCGTGGCGACATTTTTGGTGGCCTG
ACCGCTGCTGTGATTGCGCTGCCGATGGCACTGGCTTTTGGTGTGGCAAGTGGTGCAGGT
GCAGAAGCAGGTCTGTGGGGTGCAGTTCTGGTGGGCTTTTTCGCAGCACTGTTCGGTGGT
ACGCCGACCCTGATTTCAGAACCGACGGGCCCGATGACCGTGGTTATGACGGCCGTGATC
GCACATTTTACCGCATCGGCAGCTACGCCGGAAGAAGGCCTGGCTATTGCGTTCACCGTC
GTGATGATGGCCGGTGTTTTTCAGATTATCTTCGGCAGCCTGAAACTGGGCAAGTATGTT
ACCATGATGCCGTACACGGTCATCAGTGGTTTTATGTCCGGTATTGGCATTATCCTGGTG
ATCCTGCAGCTGGCACCGTTCCTGGGTCAAGCCAGTCCGGGCGGTGGCGTTATTGGCACC
CTGCAGAACCTGCCGACGCTGCTGTCCAATATCCAACCGGGTGAAACCGCCCTGGCACTG
GGTACGGTCGCGATTATCTGGTTCATGCCGGAAAAGTTCAAGAAGGTTATCCCGCCGCAG

CTGGTTGCGCTGGTTCTGGGCACCGTCATCGCGTTTTTCGTGTTTCCGCCGGAAGTTAGC
GATCTGCGTCGCATTGGCGAAATCCGTGCAGGTTTCCCGGAACTGGTGCGTCCGAGCTTT
TCTCCGGTTGAATTTCAGCGCATGATTCTGGATGCGGCCGTGCTGGGCATGCTGGGTTGC
ATCGATGCGCTGCTGACCAGCGTTGTCGCCGACTCTCTGACGCGTACCGAACATAACAGC
AATAAAGAACTGATTGGTCAGGGCCTGGGTAACCTGTTTTCTGGCCTGTTCGGTGGTATT
GCTGGTGCAGGTGCAACGATGGGCACCGTGGTTAATATCCAAAGTGGTGGCCGTACCGCA
CTGTCCGGTCTGGTGCGTGCTTTTGTTCTGCTGGTCGTGATTCTGGGTGCAGCTTCTCTG
ACGGCAACCATTCCGCTGGCTGTGCTGGCAGGCATCGCCTTTAAAGTGGGTGTTGATATT
ATCGACTGGTCATTCCTGAAACGCGCCCACGAAATCTCGCCGAAGGGCGCACTGATTATG
TATGGTGTGATCCTGCTGACCGTCCTGGTGGATCTGATTGTTGCGGTCGGCGTGGGTGTT
TTTGTCGCCAACGTTCTGACCATCGAACGTATGTCAAATCTGCAGTCGGAAAAAGTCCAA
ACCGTGAGCGATGCGGATGACAACATTCGCCTGACCACGACCGAAAAGCGTTGGCTGGAC
GAGGGTCAGGGTCGTGTGCTGCTGTTTCAACTGTCTGGCCCGATGATTTTCGGTGTTGCA
AAAGCTATCGCGCGTGAACATAACGCAATGGGTGATTGCGACGCTCTGGTGTTTGATATT
GGCGAAGTCCCGCACATGGGTGTGACCGCAAGTCTGGCTCTGGAAAATGCGATTGAAGAA
GCCCTGGACAAAGAACGCCAGGTTTACATCGTCGGTGCAGCAGGTCAAACCCGTCGCCGT
CTGGAAAAACTGAAGCTGTTTAAACGCGTGCCGCCGGATAAGTGTCTGATGTCCCGTGAA
GAAGCACTGAAGAATGCTGTTCTGGGTATCTATCCGCATCTGGCTGACGGTGTTACGGCT
CCGAGTTCCGAAATGGGCTAA
SEQ ID NO. 15: MCR DNA ORGANISM NAME: SULFOLOBUS TOKODAII
ATGTCTCGTC GCACCCTGAA AGCGGCTATC CTGGGCGCCA CCGGCCTGGT TGGTATCGAA
60
TATGTCCGTA TGCTGTCAAA TCATCCGTAT ATCAAACCGG CGTATCTGGC CGGCAAAGGT
120
TCAGTTGGCA AACCGTACGG TGAAGTGGTT CGTTGGCAGA CCGTTGGCCA AGTCCCGAAA
180
GAAATCGCCG ATATGGAAAT TAAACCGACG GACCCGAAAC TGATGGATGA CGTGGATATT
240
ATCTTTTCGC CGCTGCCGCA GGGTGCGGCC GGTCCGGTTG AAGAACAATT TGCAAAAGAA
300
GGCTTCCCGG TCATCAGCAA CTCTCCGGAT CATCGTTTCG ATCCGGACGT CCCGCTGCTG
360

GTGCCGGAAC TGAATCCGCA CACCATTAGT CTGATCGATG AACAGCGCAA ACGTCGCGAA
420
TGGAAAGGTT TTATTGTTAC CACGCCGCTG TGCACGGCAC AAGGTGCAGC TATCCCGCTG
480
GGTGCTATCT TCAAAGATTA CAAAATGGAC GGCGCGTTCA TTACCACGAT CCAGAGTCTG
540
TCCGGTGCAG GTTACCCGGG TATCCCGTCT CTGGATGTCG TGGACAACAT TCTGCCGCTG
600
GGCGATGGTT ATGACGCGAA AACCATTAAA GAAATCTTCC GTATTCTGTC AGAAGTTAAA
660
CGCAATGTCG ATGAACCGAA ACTGGAAGAC GTTTCGCTGG CGGCCACCAC GCATCGTATC
720
GCCACCATTC ATGGCCACTA TGAAGTGCTG TACGTTAGTT TTAAAGAAGA AACCGCAGCT
780
GAAAAAGTGA AAGAAACGCT GGAAAACTTC CGCGGTGAAC CGCAGGATCT GAAACTGCCG
840
ACCGCACCGT CCAAACCGAT TATCGTCATG AATGAAGATA CGCGTCCGCA AGTGTACTTT
900
GATCGCTGGG CTGGCGACAT TCCGGGTATG AGCGTTGTCG TGGGCCGTCT GAAACAGGTG
960
AACAAACGTA TGATCCGCCT GGTGTCTCTG ATTCACAATA CCGTTCGCGG TGCGGCGGGC
1020
GGTGGCATCC TGGCTGCTGA ACTGCTGGTT GAAAAAGGTT ACATTGAAAA A 1071

<212> TYPE: DNA
<211> LENGTH : 1071
   SEQUENCENAME : SULFOLOBUS TOKODAII MCR DNA 
SEQ ID NO. 16: MCR PROTEIN ORGANISM NAME : SULFOLOBUS TOKODAII
MSRRTLKAAI LGATGLVGIE YVRMLSNHPY IKPAYLAGKG SVGKPYGEVV RWQTVGQVPK
60
EIADMEIKPT DPKLMDDVDI IFSPLPQGAA GPVEEQFAKE GFPVISNSPD HRFDPDVPLL 120
VPELNPHTIS LIDEQRKRRE WKGFIVTTPL CTAQGAAIPL GAIFKDYKMD GAFITTIQSL 180
SGAGYPGIPS LDVVDNILPL GDGYDAKTIK EIFRILSEVK RNVDEPKLED VSLAATTHRI 240
 
ATIHGHYEVL YVSFKEETAA EKVKETLENF RGEPQDLKLP TAPSKPIIVM NEDTRPQVYF 300
DRWAGDIPGM SVVVGRLKQV NKRMIRLVSL IHNTVRGAAG GGILAAELLV EKGYIEK 357
<212> TYPE: PRT
<211> LENGTH : 357
   SEQUENCENAME : SULFOLOBUS TOKODAII MCR PROTEIN 
SEQ ID NO. 17 : NEMA DNA ORGANISM NAME : ESCHERICHIA COLI
ATGTCATCTG AAAAACTGTA TTCCCCACTG AAAGTGGGCG CGATCACGGC GGCAAACCGT
60
ATTTTTATGG CACCGCTGAC GCGTCTGCGC AGTATTGAAC CGGGTGACAT TCCTACCCCG
120
TTGATGGCGG AATACTATCG CCAACGTGCC AGTGCCGGTT TGATTATTAG TGAAGCCACG
180
CAAATTTCTG CCCAGGCAAA AGGATATGCA GGTGCGCCTG GCATCCATAG TCCGGAGCAA
240
ATTGCCGCAT GGAAAAAAAT CACCGCTGGC GTTCATGCTG AAAATGGTCA TATGGCCGTG
300
CAGCTGTGGC ACACCGGACG CATTTCTCAC GCCAGCCTGC AACCTGGCGG TCAGGCACCG
360
GTAGCGCCTT CAGCACTTAG CGCGGGAACA CGTACTTCTC TGCGCGATGA AAATGGTCAG
420
GCGATCCGTG TTGAAACATC CATGCCGCGT GCGCTTGAAC TGGAAGAGAT TCCAGGTATC
480
GTCAATGATT TCCGTCAGGC CATTGCTAAC GCGCGTGAAG CCGGTTTTGA TCTGGTAGAG
540
CTCCACTCTG CTCACGGTTA TTTGCTGCAT CAGTTCCTTT CTCCTTCTTC AAACCATCGT
600
ACCGATCAGT ACGGCGGCAG CGTGGAAAAT CGCGCACGTT TGGTACTGGA AGTGGTCGAT
660
GCCGGGATTG AAGAATGGGG TGCCGATCGC ATTGGCATTC GCGTTTCACC AATCGGTACT
720
TTCCAGAACA CAGATAACGG CCCGAATGAA GAAGCCGATG CACTGTATCT GATTGAACAA
780
 
CTGGGTAAAC GCGGCATTGC TTATCTGCAT ATGTCAGAAC CAGATTGGGC GGGGGGTGAA
840
CCGTATACTG ATGCGTTCCG CGAAAAAGTA CGCGCCCGTT TCCACGGTCC GATTATCGGC
900
GCAGGTGCAT ACACAGTAGA AAAAGCTGAA ACGCTGATCG GCAAAGGGTT AATTGATGCG
960
GTGGCATTTG GTCGTGACTG GATTGCGAAC CCGGATCTGG TCGCCCGCTT GCAGCGCAAA
1020
GCTGAGCTTA ACCCACAGCG TGCCGAAAGT TTCTACGGTG GCGGCGCGGA AGGCTATACC
1080
GATTACCCGA CGTTGTAA 1098
<212> TYPE: DNA
<211> LENGTH : 1098
   SEQUENCENAME : ESCHERICHIA COLI NEMA DNA 
SEQ ID NO. 18: NEMA PROTEIN ORGANISM NAME: ESCHERICHIA COLI
MSSEKLYSPL KVGAITAANR IFMAPLTRLR SIEPGDIPTP LMAEYYRQRA SAGLIISEAT 60
QISAQAKGYA GAPGIHSPEQ IAAWKKITAG VHAENGHMAV QLWHTGRISH ASLQPGGQAP
120
VAPSALSAGT RTSLRDENGQ AIRVETSMPR ALELEEIPGI VNDFRQAIAN AREAGFDLVE 180
LHSAHGYLLH QFLSPSSNHR TDQYGGSVEN RARLVLEVVD AGIEEWGADR IGIRVSPIGT
240
FQNTDNGPNE EADALYLIEQ LGKRGIAYLH MSEPDWAGGE PYTDAFREKV RARFHGPIIG
300
AGAYTVEKAE TLIGKGLIDA VAFGRDWIAN PDLVARLQRK AELNPQRAES FYGGGAEGYT
360
DYPTL 365
<212> TYPE : PRT
<211> LENGTH : 365
SEQUENCENAME : ESCHERICHIA COLI NEMA PROTEIN
SEQ ID NO. 19: RUTE DNA ORGANISM NAME: ESCHERICHIA COLI
ATGAACGAAG CCGTTAGCCC AGGTGCGCTT AGCACCCTGT TCACCGATGC CCGCACTCAC
60
 
AACGGCTGGC GGGAGACACC CGTCAGCGAT GAGACGTTAC GGGAGATTTA TGCCCTGATG
120
AAATGGGGGC CGACATCAGC TAACTGTTCT CCGGCACGGA TCGTGTTTAC CCGCACGGCA
180
GAAGGAAAAG AACGTCTGCG CCCGGCACTT TCCAGCGGCA ATCTGCAAAA AACCCTGACC
240
GCGCCCGTCA CCGCTATCGT CGCCTGGGAC AGTGAATTTT ATGAACGGTT ACCACTACTG
300
TTTCCCCACG GTGATGCCCG CAGTTGGTTT ACCTCCAGCC CACAACTTGC CGAAGAAACA
360
GCGTTTCGCA ACAGTTCCAT GCAGGCGGCC TATCTGATCG TCGCCTGCCG GGCGCTGGGA
420
CTGGATACCG GCCCGATGTC GGGCTTTGAC CGTCAACACG TGGACGACGC CTTTTTTACG
480
GGCAGCACGC TGAAGAGCAA TCTGCTGATT AATATCGGCT ATGGCGATAG CAGCAAGCTT
540
TATGCGCGCC TGCCACGTCT GTCCTTTGAA GAAGCCTGCG GGCTGTTGTA A 591
<212> TYPE: DNA
<211> LENGTH : 591
   SEQUENCENAME : ESCHERICHIA COLI RUTE DNA 
SEQ ID NO. 20 RUTE PROTEIN ORGANISM NAME : ESCHERICHIA COLI
MNEAVSPGAL STLFTDARTH NGWRETPVSD ETLREIYALM KWGPTSANCS PARIVFTRTA
60
EGKERLRPAL SSGNLQKTLT APVTAIVAWD SEFYERLPLL FPHGDARSWF TSSPQLAEET 120
AFRNSSMQAA YLIVACRALG LDTGPMSGFD RQHVDDAFFT GSTLKSNLLI NIGYGDSSKL
180
YARLPRLSFE EACGLL 196
<212> TYPE: PRT
<211> LENGTH : 196
   SEQUENCENAME : ESCHERICHIA COLI RUTE PROTEIN SEQ ID NO. 21: YDFG DNA  ORGANISM NAME : ESCHERICHIA COLI 
ATGGTCGTTT TAGTAACTGG AGCAACGGCA GGTTTTGGTG AATGCATTAC TCGTCGTTTT
60
ATTCAACAAG GGCATAAAGT TATCGCCACT GGCCGTCGCC AGGAACGGTT GCAGGAGTTA
120
AAAGACGAAC TGGGAGATAA TCTGTATATC GCCCAACTGG ACGTTCGCAA CCGCGCCGCT
180
ATTGAAGAGA TGCTGGCATC GCTTCCTGCC GAGTGGTGCA ATATTGATAT CCTGGTAAAT
240
AATGCCGGCC TGGCGTTGGG CATGGAGCCT GCGCATAAAG CCAGCGTTGA AGACTGGGAA
300
ACGATGATTG ATACCAACAA CAAAGGCCTG GTATATATGA CGCGCGCCGT CTTACCGGGT
360
ATGGTTGAAC GTAATCATGG TCATATTATT AACATTGGCT CAACGGCAGG TAGCTGGCCG
420
TATGCCGGTG GTAACGTTTA CGGTGCGACG AAAGCGTTTG TTCGTCAGTT TAGCCTGAAT
480
CTGCGTACGG ATCTGCATGG TACGGCGGTG CGCGTCACCG ACATCGAACC GGGTCTGGTG
540
GGTGGTACCG AGTTTTCCAA TGTCCGCTTT AAAGGCGATG ACGGTAAAGC AGAAAAAACC
600
TATCAAAATA CCGTTGCATT GACGCCAGAA GATGTCAGCG AAGCCGTCTG GTGGGTGTCA
660
ACGCTGCCTG CTCACGTCAA TATCAATACC CTGGAAATGA TGCCGGTTAC CCAAAGCTAT
720
GCCGGACTGA ATGTCCACCG TCAGTAA 747
<212> TYPE: DNA
<211> LENGTH : 747
   SEQUENCENAME : ESCHERICHIA COLI YDFG DNA
   **SEQ ID NO. 22: YDFG PROTEIN ORGANISM NAME: ESCHERICHIA COLI** 
MVVLVTGATA GFGECITRRF IQQGHKVIAT GRRQERLQEL KDELGDNLYI AQLDVRNRAA

60
 
IEEMLASLPA EWCNIDILVN NAGLALGMEP AHKASVEDWE TMIDTNNKGL VYMTRAVLPG
120
MVERNHGHII NIGSTAGSWP YAGGNVYGAT KAFVRQFSLN LRTDLHGTAV RVTDIEPGLV
180
GGTEFSNVRF KGDDGKAEKT YQNTVALTPE DVSEAVWWVS TLPAHVNINT LEMMPVTQSY
240
AGLNVHRQ 248
<212> TYPE : PRT
<211> LENGTH : 248
   SEQUENCENAME : ESCHERICHIA COLI YDFG PROTEIN 
SEQ ID NO. 23: MMSB DNA ORGANISM NAME : P. AERUGINOSA
ATGGCCGACA TTGCGTTTCT GGGTCTGGGC AATATGGGCG GTCCGATGGC CGCGAACCTG
60
CTGAAAGCCG GCCACCGTGT GAATGTGTTC GACCTGCAAC CAAAAGCGGT CCTGGGCTTG
120
GTTGAGCAAG GCGCGCAGGG CGCAGACTCT GCTCTGCAAT GTTGTGAGGG TGCGGAGGTC
180
GTGATTTCTA TGCTGCCAGC AGGCCAGCAT GTGGAAAGCC TGTACCTGGG CGATGATGGT
240
CTGCTGGCAC GCGTGGCGGG CAAGCCTTTG CTGATTGACT GTAGCACCAT CGCACCGGAA
300
ACGGCGCGTA AGGTGGCGGA GGCAGCCGCA GCAAAGGGCC TGACGCTGCT GGATGCCCCG
360
GTTTCGGGCG GTGTCGGTGG TGCCCGTGCA GGTACGCTGT CGTTTATCGT GGGTGGTCCG
420
GCGGAGGGTT TTGCGCGTGC GCGTCCGGTT CTGGAGAATA TGGGTCGCAA CATTTTCCAC
480
GCGGGTGATC ACGGCGCTGG TCAGGTGGCG AAAATCTGTA ACAACATGCT GCTGGGTATC
540
TTGATGGCGG GCACCGCCGA AGCCTTGGCG CTGGGCGTCA AAAACGGTCT GGACCCGGCA
600
 
GTGCTGTCCG AAGTGATGAA ACAGAGCAGC GGTGGTAACT GGGCGCTGAA TCTGTACAAT
660
CCGTGGCCGG GTGTGATGCC GCAGGCCCCA GCCTCTAATG GCTACGCAGG CGGCTTCCAA
720
GTGCGCCTGA TGAACAAAGA CCTGGGCCTG GCGCTGGCGA ATGCGCAAGC GGTCCAAGCG
780
AGCACCCCGC TGGGCGCACT GGCCCGTAAC CTGTTTAGCC TGCACGCTCA AGCCGACGCC
840
GAGCACGAAG GTCTGGACTT CAGCTCTATT CAAAAACTGT ATCGCGGTAA GGATTAG
897
<212> TYPE: DNA
<211> LENGTH : 897
   SEQUENCE NAME: P. AERUGINOSA MMSB DNA 
SEQ ID NO. 24: MMSB PROTEIN ORGANISM NAME: P. AERUGINOSA
MADIAFLGLG NMGGPMAANL LKAGHRVNVF DLQPKAVLGL VEQGAQGADS ALQCCEGAEV
60
VISMLPAGQH VESLYLGDDG LLARVAGKPL LIDCSTIAPE TARKVAEAAA AKGLTLLDAP 120
VSGGVGGARA GTLSFIVGGP AEGFARARPV LENMGRNIFH AGDHGAGQVA KICNNMLLGI
180
LMAGTAEALA LGVKNGLDPA VLSEVMKQSS GGNWALNLYN PWPGVMPQAP ASNGYAGGFQ
240
VRLMNKDLGL ALANAQAVQA STPLGALARN LFSLHAQADA EHEGLDFSSI QKLYRGKD
298
<212> TYPE : PRT
<211> LENGTH : 298
SEQUENCENAME : P. AERUGINOSA MMSB PROTEIN
SEQ ID NO. 25: NDSD DNA ORGANISMNAME : PSEUDOMONAS AERUGINOSA
ATGGGCAAAC AGATCGCCTT CATCGGCCTG GGCCATATGG GCGCACCTAT GGCCACCAAC
60
CTGCTGAAGG CCGGCTACCT GCTGAATGTG TTCGACCTGG TGCAGAGCGC CGTGGATGGT
120
 
TTAGTGGCCG CAGGTGCAAG TGCAGCACGC AGTGCACGCG ATGCCGTTCA GGGTGCCGAC
180
GTGGTGATCA GCATGCTGCC TGCCAGCCAA CACGTGGAGG GTCTGTACCT GGACGACGAT
240
GGTCTGCTGG CCCACATTGC CCCTGGCACC TTAGTGCTGG AGTGCAGCAC AATCGCCCCG
300
ACCAGTGCAC GCAAGATTCA TGCAGCAGCC CGCGAGCGTG GTCTGGCAAT GCTGGACGCA
360
CCGGTTAGCG GTGGTACAGC AGGTGCCGCA GCAGGCACCC TGACCTTCAT GGTGGGCGGT
420
GACGCCGAAG CCCTGGAAAA AGCACGCCCG CTGTTTGAGG CAATGGGCCG TAACATCTTC
480
CATGCCGGCC CTGATGGCGC AGGTCAGGTG GCCAAAGTGT GCAATAACCA GCTGCTGGCA
540
GTGCTGATGA TCGGTACCGC CGAGGCAATG GCACTGGGCG TGGCAAACGG CTTAGAGGCC
600
AAGGTGCTGG CAGAAATCAT GCGCCGTAGT AGCGGCGGTA ACTGGGCCCT GGAGGTGTAC
660
AACCCGTGGC CTGGCGTGAT GGAGAATGCA CCGGCCAGTC GTGACTACAG CGGCGGTTTC
720
ATGGCACAGC TGATGGCCAA GGACCTGGGC TTAGCCCAAG AGGCAGCCCA AGCCAGCGCC
780
AGTAGTACCC CGATGGGCAG CTTAGCCCTG AGTCTGTACC GCTTACTGCT GAAGCAGGGC
840
TACGCCGAAC GCGACTTCAG CGTGGTGCAG AAGCTGTTCG ACCCGACCCA AGGCCAGTAA
900
<212> TYPE: DNA
<211> LENGTH : 900
   SEQUENCENAME : P. AERUGINOSA NDSD DNA 
SEQ ID NO. 26: NDSD PROTEIN PSEUDOMONAS AERUGINOSA
MGKQIAFIGL GHMGAPMATN LLKAGYLLNV FDLVQSAVDG LVAAGASAAR SARDAVQGAD
60
 
VVISMLPASQ HVEGLYLDDD GLLAHIAPGT LVLECSTIAP TSARKIHAAA RERGLAMLDA 120
PVSGGTAGAA AGTLTFMVGG DAEALEKARP LFEAMGRNIF HAGPDGAGQV AKVCNNQLLA
180
VLMIGTAEAM ALGVANGLEA KVLAEIMRRS SGGNWALEVY NPWPGVMENA PASRDYSGGF
240
MAQLMAKDLG LAQEAAQASA SSTPMGSLAL SLYRLLLKQG YAERDFSVVQ KLFDPTQGQ
299
<212> TYPE : PRT
<211> LENGTH : 299
   SEQUENCENAME : P. AERUGINOSA NDSD PROTEIN 
SEQ ID NO. 27: FABI(TS) PROTEIN ORGANISM NAME : ESCHERICHIA COLI
MGFLSGKRIL VTGVASKLSI AYGIAQAMHR EGAELAFTYQ NDKLKGRVEE FAAQLGSDIV 60
LQCDVAEDAS IDTMFAELGK VWPKFDGFVH SIGFAPGDQL DGDYVNAVTR EGFKIAHDIS
120
SYSFVAMAKA CRSMLNPGSA LLTLSYLGAE RAIPNYNVMG LAKASLEANV RYMANAMGPE
180
GVRVNAISAG PIRTLAASGI KDFRKMLAHC EAVTPIRRTV TIEDVGNSAA FLCSDLSAGI 240
FGEVVHVDGG FSIAAMNELE LK 262
<212> TYPE: PRT
<211> LENGTH : 262
   SEQUENCENAME: ESCHERICHIA COLI FABI(TS) PROTEIN 
SEQ ID NO. 28: FABB(TS) PROTEIN ORGANISM NAME : ESCHERICHIA COLI
MKRAVITGLG IVSSIGNNQQ EVLASLREGR SGITFSQELK DSGMRSHVWG NVKLDTTGLI
60
DRKVVRFMSD ASIYAFLSME QAIADAGLSP EAYQNNPRVG LIAGSGGGSP RFQVFGADAM
120
RGPRGLKAVG PYVVTKAMAS GVSACLATPF KIHGVNYSIS SACATSAHCI GNAVEQIQLG
180
KQDIVFAGGG EELCWEMACE FDAMGALSTK YNDTPEKASR TYDAHRDGFV IAGGGGMVVV
240
EELEHALARG AHIYAEIVGY GATSDGADMV APSGEGAVRC MKMAMHGVDT PIDYLNSHGT
300
 
STPVGDVKEL AAIREVFGDK SPAISATKVM TGHSLGAAGV QEAIYSLLML EHGFIAPSIN 360
IEELDEQAAG LNIVTETTDR ELTTVMSNSF GFGGTNATLV MRKLKD 406
<212> TYPE: PRT
<211> LENGTH : 406
   SEQUENCENAME : ESCHERICHIA COLI FABB(TS) PROTEIN 
SEQ ID NO. 29: FABD(TS) PROTEIN ORGANISM NAME : ESCHERICHIA COLI
MTQFAFVFPG QGSQTVGMLA DMAASYPIVE ETFAEASAAL GYDLWALTQQ GPAEELNKTW
60
QTQPALLTAS VALYRVWQQQ GGKAPAMMAG HSLGEYSALV CAGVIDFADA VRLVEMRGKF
120
MQEAVPEGTG AMAAIIGLDD ASIAKACEEA AEGQVVSPVN FNSPGQVVIA GHKEAVERAG
180
AACKAAGAKR ALPLPVSVPS HCALMKPAAD KLAVELAKIT FNAPTVPVVN NVDVKCETNG
240
DAIRDALVRQ LYNPVQQTKS VEYMAAQGVE HLYEVGPGKV LTGLTKRIVD TLTASALNEP
300
SAMAAALEL 309
<212> TYPE : PRT
<211> LENGTH : 309
   SEQUENCENAME : ESCHERICHIA COLI FABD(TS) PROTEIN 
SEQ ID NO. 30: RHTA DNA ORGANISM NAME : ESCHERICHIA COLI
ATGAGCGGTT CATTACGTAA AATGCCGGTC TGGTTACCAA TAGTCATATT GCTCGTTGCC
60
ATGGCGTCTA TTCAGGGTGG AGCCTCGTTA GCTAAGTCAC TTTTTCCTCT GGTGGGCGCA
120
CCGGGTGTCA CTGCGCTGCG TCTGGCATTA GGAACGCTGA TCCTCATCGC GTTCTTTAAG
180
CCATGGCGAC TGCGCTTTGC CAAAGAGCAA CGGTTACCGC TGTTGTTTTA CGGCGTTTCG
240
CTGGGTGGGA TGAATTATCT TTTTTATCTT TCTATTCAGA CAGTACCGCT GGGTATTGCG
300
 
GTGGCGCTGG AGTTCACCGG ACCACTGGCG GTGGCGCTGT TCTCTTCTCG TCGCCCGGTA
360
GATTTCGTCT GGGTTGTGCT GGCGGTTCTT GGTCTGTGGT TCCTGCTACC GCTGGGGCAA
420
GACGTTTCCC ATGTCGATTT AACCGGCTGT GCGCTGGCAC TGGGGGCCGG GGCTTGTTGG
480
GCTATTTACA TTTTAAGTGG GCAACGCGCA GGAGCGGAAC ATGGCCCTGC GACGGTGGCA
540
ATTGGTTCGT TGATTGCAGC GTTAATTTTC GTGCCAATTG GAGCGCTTCA GGCTGGTGAA
600
GCACTCTGGC ACTGGTCGGT TATTCCATTG GGTCTGGCTG TCGCTATTCT CTCGACCGCT
660
CTGCCTTATT CGCTGGAAAT GATTGCCCTC ACCCGTTTGC CAACACGGAC ATTTGGTACG
720
CTGATGAGCA TGGAACCGGC GCTGGCTGCC GTTTCCGGGA TGATTTTCCT CGGAGAAACA
780
CTGACACCCA TACAGCTACT GGCGCTCGGC GCTATCATCG CCGCTTCAAT GGGGTCTACG
840
CTGACAGTAC GCAAAGAGAG CAAAATAAAA GAATTAGACA TTAATTAA 888
<212> TYPE: DNA
<211> LENGTH : 888
   SEQUENCENAME : ESCHERICHIA COLI RHTA DNA 
SEQ ID NO. 31: RHTA PROTEIN ORGANISM NAME : ESCHERICHIA COLI
MSGSLRKMPV WLPIVILLVA MASIQGGASL AKSLFPLVGA PGVTALRLAL GTLILIAFFK 60
PWRLRFAKEQ RLPLLFYGVS LGGMNYLFYL SIQTVPLGIA VALEFTGPLA VALFSSRRPV 120
DFVWVVLAVL GLWFLLPLGQ DVSHVDLTGC ALALGAGACW AIYILSGQRA GAEHGPATVA
180
IGSLIAALIF VPIGALQAGE ALWHWSVIPL GLAVAILSTA LPYSLEMIAL TRLPTRTFGT 240
LMSMEPALAA VSGMIFLGET LTPIQLLALG AIIAASMGST LTVRKESKIK ELDIN 295
<212> TYPE : PRT
<211> LENGTH : 295
   SEQUENCENAME : ESCHERICHIA COLI RHTA PROTEIN 
SEQ ID NO. 32: YDCO DNA ORGANISM NAME : ESCHERICHIA COLI
ATGCGACTCC TCCGGCAAAA CGGAAGTTTA TCACTTGTGC GTTATAACGG ACAAATGCTA
60
CGGTGCCTGT ACGCTATAAC GCACGAGGTG ACTATGCGTC TGTTTTCTAT TCCTCCACCC
120
ACGCTACTGG CGGGGTTTCT GGCGGTATTA ATTGGCTACG CCAGTTCAGC GGCAATAATC
180
TGGCAAGCAG CGATTGTCGC CGGAGCCACC ACTGCACAAA TCTCTGGCTG GATGACGGCG
240
CTGGGGCTGG CAATGGGCGT CAGTACGCTG ACTCTGACAT TATGGTATCG CGTACCTGTT
300
CTCACCGCAT GGTCAACGCC TGGCGCGGCT TTGTTGGTCA CCGGATTGCA GGGACTAACA
360
CTTAACGAAG CCATCGGCGT TTTTATTGTC ACCAACGCGC TAATAGTCCT CTGCGGCATA
420
ACGGGACTCT TTGCTCGTCT GATGCGCATT ATTCCGCACT CGCTTGCGGC GGCAATGCTT
480
GCCGGGATTT TATTACGCTT TGGTTTACAG GCGTTTGCCA GTCTGGACGG TCAATTTACG
540
TTGTGTGGAA GTATGTTGCT GGTATGGCTG GCAACCAAGG CCGTTGCGCC GCGCTATGCG
600
GTAATTGCCG CGATGATTAT TGGGATCGTG ATCGTCATCG CGCAAGGTGA CGTTGTCACA
660
ACTGATGTTG TCTTTAAACC CGTTCTCCCC ACTTATATTA CCCCTGATTT TTCGTTTGCT
720
CACAGCCTGA GCGTTGCACT CCCCCTTTTT CTGGTGACGA TGGCATCGCA AAACGCACCG
780
GGTATCGCAG CAATGAAAGC AGCTGGATAT TCGGCTCCTG TTTCGCCATT AATTGTATTT
840
ACTGGATTGC TGGCACTGGT TTTTTCCCCT TTCGGCGTTT ATTCCGTCGG TATTGCGGCA
900
 
ATCACCGCGG CTATTTGCCA AAGCCCGGAA GCGCATCCGG ATAAAGATCA ACGTTGGCTG
960
GCCGCTGCCG TTGCAGGCAT TTTCTATTTG CTCGCAGGTC TGTTTGGTAG TGCCATTACC
1020
GGGATGATGG CTGCCCTGCC CGTAAGTTGG ATCCAGATGC TGGCAGGTCT GGCGCTGTTA
1080
AGTACCATCG GCGGCAGTTT GTATCAGGCG CTGCATAATG AGCGTGAGCG AGACGCGGCG
1140
GTGGTGGCAT TTCTGGTAAC GGCAAGTGGA TTGACGCTGG TCGGGATTGG TTCTGCGTTT
1200
TGGGGATTAA TTGCCGGAGG CGTTTGTTAC GTGGTGTTGA ATTTAATCGC TGACAGAAAC
1260
CGATATTGA 1269
<212> TYPE: DNA
<211> LENGTH : 1269
   SEQUENCENAME : ESCHERICHIA COLI YDCO DNA 
SEQ ID NO. 33: YDCO PROTEIN ORGANISM NAME : ESCHERICHIA COLI
MRLLRQNGSL SLVRYNGQML RCLYAITHEV TMRLFSIPPP TLLAGFLAVL IGYASSAAII 60
WQAAIVAGAT TAQISGWMTA LGLAMGVSTL TLTLWYRVPV LTAWSTPGAA LLVTGLQGLT
120
LNEAIGVFIV TNALIVLCGI TGLFARLMRI IPHSLAAAML AGILLRFGLQ AFASLDGQFT 180
LCGSMLLVWL ATKAVAPRYA VIAAMIIGIV IVIAQGDVVT TDVVFKPVLP TYITPDFSFA 240
HSLSVALPLF LVTMASQNAP GIAAMKAAGY SAPVSPLIVF TGLLALVFSP FGVYSVGIAA 300
ITAAICQSPE AHPDKDQRWL AAAVAGIFYL LAGLFGSAIT GMMAALPVSW IQMLAGLALL
360
STIGGSLYQA LHNERERDAA VVAFLVTASG LTLVGIGSAF WGLIAGGVCY VVLNLIADRN 420
RY 422
<212> TYPE: PRT
<211> LENGTH : 422
   SEQUENCENAME : ESCHERICHIA COLI YDCO PROTEIN SEQ ID NO. 34: ACRD ORGANISM NAME : ESCHERICHIA COLI 
MANFFIDRPI FAWVLAILLC LTGTLAIFSL PVEQYPDLAP PNVRVTANYP GASAQTLENT
VTQVIEQNMT GLDNLMYMSS QSSGTGQASV TLSFKAGTDP DEAVQQVQNQ LQSAMRKLPQ
AVQNQGVTVR KTGDTNILTI AFVSTDGSMD KQDIADYVAS NIQDPLSRVN GVGDIDAYGS
QYSMRIWLDP AKLNSFQMTA KDVTDAIESQ NAQIAVGQLG GTPSVDKQAL NATINAQSLL
QTPEQFRDIT LRVNQDGSEV RLGDVATVEM GAEKYDYLSR FNGKPASGLG VKLASGANEM
ATAELVLNRL DELAQYFPHG LEYKVAYETT SFVKASIEDV VKTLLEAIAL VFLVMYLFLQ
NFRATLIPTI AVPVVLMGTF SVLYAFGYSV NTLTMFAMVL AIGLLVDDAI VVVENVERIM
SEEGLTPREA TRKSMGQIQG ALVGIAMVLS AVFVPMAFFG GTTGAIYRQF SITIVAAMVL
SVLVAMILTP ALCATLLKPL KKGEHHGQKG FFAWFNQMFN RNAERYEKGV AKILHRSLRW
IVIYVLLLGG MVFLFLRLPT SFLPLEDRGM FTTSVQLPSG STQQQTLKVV EQIEKYYFTH
EKDNIMSVFA TVGSGPGGNG QNVARMFIRL KDWSERDSKT GTSFAIIERA TKAFNQIKEA
RVIASSPPAI SGLGSSAGFD MELQDHAGAG HDALMAARNQ LLALAAENPE LTRVRHNGLD
DSPQLQIDID QRKAQALGVA IDDINDTLQT AWGSSYVNDF MDRGRVKKVY VQAAAPYRML
PDDINLWYVR NKDGGMVPFS AFATSRWETG SPRLERYNGY SAVEIVGEAA PGVSTGTAMD
IMESLVKQLP NGFGLEWTAM SYQERLSGAQ APALYAISLL VVFLCLAALY ESWSVPFSVM
LVVPLGVIGA LLATWMRGLE NDVYFQVGLL TVIGLSAKNA ILIVEFANEM NQKGHDLFEA
TLHACRQRLR PILMTSLAFI FGVLPMATST GAGSGGQHAV GTGVMGGMIS ATILAIYFVP
LFFVLVRRRF PLKPRPE
SEQ ID NO. 35: ATTA ORGANISM NAME : ESCHERICHIA COLI
MAHPPLLHLQ DITLSLGGNP LLDGAGFAVG RGERLCLVGR NGSGKSTLLK IAAGVIQPDS
GSVFVQPGAS LRYLPQEPDL SAYATTADYV VGQIGDPDMA WRATPLLDAL GLTGRESTQN
LSGGEGRRCA IAGVLAAAPD VLLLDEPTNH LDMPTIEWLE RELLSLGAMV IISHDRRLLS
TLSRSVVWLD RGVTRRLDEG FGRFEAWREE VLEQEERDAH KLDRKIAREE DWMRYGVTAR
RKRNVRRVRE LADLRTARKE AIRAPGTLTL NTQLRPHRKL VAVAEDISKA WGEKQVVRHL
DLRILRGDRL GIVGANGAGK TTLLRMLTGL DQPDSGTISL GPSLNMVTLD QQRRTLNPER
TLADTLTEGG GDMVQVGTEK RHVVGYMKDF LFRPEQARTP VSALSGGERG RLMLACALAK
PSNLLVLDEP TNDLDLETLD ILQDMLASCE GTVLLVSHDR DFLDRVATSV LATEGDGNWI
EYAGGYSDML AQRHQKPLTT ASVVENEPTK PKETTAARGP TKKLSYKDQF ALDNLPKEME
KLEAQAANCV KNWQIQIYME KTPRSLRNFR LIYRSSKQSW QNLKNAGWNW K
SEQ ID NO. 36: BCR ORGANISM NAME : ESCHERICHIA COLI
MTTRQHSSFA IVFILGLLAM LMPLSIDMYL PALPVISAQF GVPAGSTQMT LSTYILGFAL
GQLIYGPMAD SFGRKPVVLG GTLVFAAAAV ACALANTIDQ LIVMRFFHGL AAAAASVVIN
 
ALMRDIYPKE EFSRMMSFVM LVTTIAPLMA PIVGGWVLVW LSWHYIFWIL ALAAILASAM
IFFLIKETLP PERRQPFHIR TTIGNFAALF RHKRVLSYML ASGFSFAGMF SFLSAGPFVY
IEINHVAPEN FGYYFALNIV FLFVMTIFNS RFVRRIGALN MFRSGLWIQF IMAAWMVISA
LLGLGFWSLV VGVAAFVGCV SMVSSNAMAV ILDEFPHMAG TASSLAGTFR FGIGAIVGAL
LSLATFNSAW PMIWSIAFCA TSSILFCLYA SRPKKR
SEQ ID NO. 37: CUSA ORGANISM NAME : ESCHERICHIA COLI
MIEWIIRRSV ANRFLVLMGA LFLSIWGTWT IINTPVDALP DLSDVQVIIK TSYPGQAPQI
VENQVTYPLT TTMLSVPGAK TVRGFSQFGD SYVYVIFEDG TDPYWARSRV LEYLNQVQGK
LPAGVSAELG PDATGVGWIY EYALVDRSGK HDLADLRSLQ DWFLKYELKT IPDVAEVASV
GGVVKEYQVV IDPQRLAQYG ISLAEVKSAL DASNQEAGGS SIELAEAEYM VRASGYLQTL
DDFNHIVLKA SENGVPVYLR DVAKVQIGPE MRRGIAELNG EGEVAGGVVI LRSGKNAREV
IAAVKDKLET LKSSLPEGVE IVTTYDRSQL IDRAIDNLSG KLLEEFIVVA VVCALFLWHV
RSALVAIISL PLGLCIAFIV MHFQGLNANI MSLGGIAIAV GAMVDAAIVM IENAHKRLEE
WQHQHPDATL DNKTRWQVIT DASVEVGPAL FISLLIITLS FIPIFTLEGQ EGRLFGPLAF
TKTYAMAGAA LLAIVVIPIL MGYWIRGKIP PESSNPLNRF LIRVYHPLLL KVLHWPKTTL
LVAALSVLTV LWPLNKVGGE FLPQINEGDL LYMPSTLPGI SAAEAASMLQ KTDKLIMSVP
EVARVFGKTG KAETATDSAP LEMVETTIQL KPQEQWRPGM TMDKIIEELD NTVRLPGLAN
LWVPPIRNRI DMLSTGIKSP IGIKVSGTVL ADIDAMAEQI EEVARTVPGV ASALAERLEG
GRYINVEINR EKAARYGMTV ADVQLFVTSA VGGAMVGETV EGIARYPINL RYPQSWRDSP
QALRQLPILT PMKQQITLAD VADIKVSTGP SMLKTENARP TSWIYIDARD RDMVSVVHDL
QKAIAEKVQL KPGTSVAFSG QFELLERANH KLKLMVPMTL MIIFVLLYLA FRRVGEALLI
ISSVPFALVG GIWLLWWMGF HLSVATGTGF IALAGVAAEF GVVMLMYLRH AIEAVPSLNN
PQTFSEQKLD EALYHGAVLR VRPKAMTVAV IIAGLLPILW GTGAGSEVMS RIAAPMIGGM
ITAPLLSLFI IPAAYKLMWL HRHRVRK
SEQ ID NO. 38: CYNX ORGANISM NAME: ESCHERICHIA COLI
MLLVLVLIGL NMRPLLTSVG PLLPQLRQAS GMSFSVAALL TALPVVTMGG LALAGSWLHQ
HVSERRSVAI SLLLIAVGAL MRELYPQSAL LLSSALLGGV GIGIIQAVMP SVIKRRFQQR
TPLVMGLWSA ALMGGGGLGA AITPWLVQHS ETWYQTLAWW ALPAVVALFA WWWQSAREVA
SSHKTTTTPV RVVFTPRAWT LGVYFGLING GYASLIAWLP AFYIEIGASA QYSGSLLALM
TLGQAAGALL MPAMARHQDR RKLLMLALVL QLVGFCGFIW LPMQLPVLWA MVCGLGLGGA
FPLCLLLALD HSVQPAIAGK LVAFMQGIGF IIAGLAPWFS GVLRSISGNY LMDWAFHALC
VVGLMIITLR FAPVRFPQLW VKEA
 
SEQ ID NO. 39: DEDA ORGANISM NAME : ESCHERICHIA COLI
MDLIYFLIDF ILHIDVHLAE LVAEYGVWVY AILFLILFCE TGLVVTPFLP GDSLLFVAGA
LASLETNDLN VHMMVVLMLI AAIVGDAVNY TIGRLFGEKL FSNPNSKIFR RSYLDKTHQF
YEKHGGKTII LARFVPIVRT FAPFVAGMGH MSYRHFAAYN VIGALLWVLL FTYAGYFFGT
IPMVQDNLKL LIVGIIVVSI LPGVIEIIRH KRAAARAAK
SEQ ID NO. 40: EAMA ORGANISM NAME : ESCHERICHIA COLI
MSRKDGVLAL LVVVVWGLNF VVIKVGLHNM PPLMLAGLRF MLVAFPAIFF VARPKVPLNL
LLGYGLTISF AQFAFLFCAI NFGMPAGLAS LVLQAQAFFT IMLGAFTFGE RLHGKQLAGI
ALAIFGVLVL IEDSLNGQHV AMLGFMLTLA AAFSWACGNI FNKKIMSHST RPAVMSLVIW
SALIPIIPFF VASLILDGSA TMIHSLVTID MTTILSLMYL AFVATIVGYG IWGTLLGRYE
TWRVAPLSLL VPVVGLASAA LLLDERLTGL QFLGAVLIMT GLYINVFGLR WRKAVKVGS
SEQ ID NO. 41: EAMB ORGANISM NAME : ESCHERICHIA COLI
MTPTLLSAFW TYTLITAMTP GPNNILALSS ATSHGFRQST RVLAGMSLGF LIVMLLCAGI
SFSLAVIDPA AVHLLSWAGA AYIVWLAWKI ATSPTKEDGL QAKPISFWAS FALQFVNVKI
ILYGVTALST FVLPQTQALS WVVGVSVLLA MIGTFGNVCW ALAGHLFQRL FRQYGRQLNI
VLALLLVYCA VRIFY
SEQ ID NO. 42: EMRB ORGANISM NAME : ESCHERICHIA COLI
MQQQKPLEGA QLVIMTIALS LATFMQVLDS TIANVAIPTI AGNLGSSLSQ GTWVITSFGV
ANAISIPLTG WLAKRVGEVK LFLWSTIAFA IASWACGVSS SLNMLIFFRV IQGIVAGPLI
PLSQSLLLNN YPPAKRSIAL ALWSMTVIVA PICGPILGGY ISDNYHWGWI FFINVPIGVA
VVLMTLQTLR GRETRTERRR IDAVGLALLV IGIGSLQIML DRGKELDWFS SQEIIILTVV
AVVAICFLIV WELTDDNPIV DLSLFKSRNF TIGCLCISLA YMLYFGAIVL LPQLLQEVYG
YTATWAGLAS APVGIIPVIL SPIIGRFAHK LDMRRLVTFS FIMYAVCFYW RAYTFEPGMD
FGASAWPQFI QGFAVACFFM PLTTITLSGL PPERLAAASS LSNFTRTLAG SIGTSITTTM
WTNRESMHHA QLTESVNPFN PNAQAMYSQL EGLGMTQQQA SGWIAQQITN QGLIISANEI
FWMSAGIFLV LLGLVWFAKP PFGAGGGGGG AH
SEQ ID NO. 43: EMRD ORGANISM NAME : ESCHERICHIA COLI
MKRQRNVNLL LMLVLLVAVG QMAQTIYIPA IADMARDLNV REGAVQSVMG AYLLTYGVSQ
LFYGPISDRV GRRPVILVGM SIFMLATLVA VTTSSLTVLI AASAMQGMGT GVGGVMARTL
PRDLYERTQL RHANSLLNMG ILVSPLLAPL IGGLLDTMWN WRACYLFLLV LCAGVTFSMA
RWMPETRPVD APRTRLLTSY KTLFGNSGFN CYLLMLIGGL AGIAAFEACS GVLMGAVLGL
SSMTVSILFI LPIPAAFFGA WFAGRPNKRF STLMWQSVIC CLLAGLLMWI PDWFGVMNVW
 
TLLVPAALFF FGAGMLFPLA TSGAMEPFPF LAGTAGALVG GLQNIGSGVL ASLSAMLPQT
GQGSLGLLMT LMGLLIVLCW LPLATRMSHQ GQPV
SEQ ID NO. 44: EMRKY ORGANISM NAME : ESCHERICHIA COLI
MAITKSTPAP LTGGTLWCVT IALSLATFMQ MLDSTISNVA IPTISGFLGA STDEGTWVIT
SFGVANAIAI PVTGRLAQRI GELRLFLLSV TFFSLSSLMC SLSTNLDVLI FFRVVQGLMA
GPLIPLSQSL LLRNYPPEKR TFALALWSMT VIIAPICGPI LGGYICDNFS WGWIFLINVP
MGIIVLTLCL TLLKGRETET SPVKMNLPGL TLLVLGVGGL QIMLDKGRDL DWFNSSTIII
LTVVSVISLI SLVIWESTSE NPILDLSLFK SRNFTIGIVS ITCAYLFYSG AIVLMPQLLQ
ETMGYNAIWA GLAYAPIGIM PLLISPLIGR YGNKIDMRLL VTFSFLMYAV CYYWRSVTFM
PTIDFTGIIL PQFFQGFAVA CFFLPLTTIS FSGLPDNKFA NASSMSNFFR TLSGSVGTSL
TMTLWGRRES LHHSQLTATI DQFNPVFNSS SQIMDKYYGS LSGVLNEINN EITQQSLSIS
ANEIFRMAAI AFILLTVLVW FAKPPFTAKG VG
SEQ ID NO. 45: EMRY ORGANISM NAME : ESCHERICHIA COLI
MAITKSTPAP LTGGTLWCVT IALSLATFMQ MLDSTISNVA IPTISGFLGA STDEGTWVIT
SFGVANAIAI PVTGRLAQRI GELRLFLLSV TFFSLSSLMC SLSTNLDVLI FFRVVQGLMA
GPLIPLSQSL LLRNYPPEKR TFALALWSMT VIIAPICGPI LGGYICDNFS WGWIFLINVP
MGIIVLTLCL TLLKGRETET SPVKMNLPGL TLLVLGVGGL QIMLDKGRDL DWFNSSTIII
LTVVSVISLI SLVIWESTSE NPILDLSLFK SRNFTIGIVS ITCAYLFYSG AIVLMPQLLQ
ETMGYNAIWA GLAYAPIGIM PLLISPLIGR YGNKIDMRLL VTFSFLMYAV CYYWRSVTFM
PTIDFTGIIL PQFFQGFAVA CFFLPLTTIS FSGLPDNKFA NASSMSNFFR TLSGSVGTSL
TMTLWGRRES LHHSQLTATI DQFNPVFNSS SQIMDKYYGS LSGVLNEINN EITQQSLSIS
ANEIFRMAAI AFILLTVLVW FAKPPFTAKG VG
SEQ ID NO. 46: GARP ORGANISM NAME : ESCHERICHIA COLI
MILDTVDEKK KGVHTRYLIL LIIFIVTAVN YADRATLSIA GTEVAKELQL SAVSMGYIFS
AFGWAYLLMQ IPGGWLLDKF GSKKVYTYSL FFWSLFTFLQ GFVDMFPLAW AGISMFFMRF
MLGFSEAPSF PANARIVAAW FPTKERGTAS AIFNSAQYFS LALFSPLLGW LTFAWGWEHV
FTVMGVIGFV LTALWIKLIH NPTDHPRMSA EELKFISENG AVVDMDHKKP GSAAASGPKL
HYIKQLLSNR MMLGVFFGQY FINTITWFFL TWFPIYLVQE KGMSILKVGL VASIPALCGF
AGGVLGGVFS DYLIKRGLSL TLARKLPIVL GMLLASTIIL CNYTNNTTLV VMLMALAFFG
KGFGALGWPV ISDTAPKEIV GLCGGVFNVF GNVASIVTPL VIGYLVSELH SFNAALVFVG
CSALMAMVCY LFVVGDIKRM ELQK
SEQ ID NO. 47: GUDP ORGANISM NAME: ESCHERICHIA COLI
 
MSSLSQAASS VEKRTNARYW IVVMLFIVTS FNYGDRATLS IAGSEMAKDI GLDPVGMGYV
FSAFSWAYVI GQIPGGWLLD RFGSKRVYFW SIFIWSMFTL LQGFVDIFSG FGIIVALFTL
RFLVGLAEAP SFPGNSRIVA AWFPAQERGT AVSIFNSAQY FATVIFAPIM GWLTHEVGWS
HVFFFMGGLG IVISFIWLKV IHEPNQHPGV NKKELEYIAA GGALINMDQQ NTKVKVPFSV
KWGQIKQLLG SRMMIGVYIG QYCINALTYF FITWFPVYLV QARGMSILKA GFVASVPAVC
GFIGGVLGGI ISDWLMRRTG SLNIARKTPI VMGMLLSMVM VFCNYVNVEW MIIGFMALAF
FGKGIGALGW AVMADTAPKE ISGLSGGLFN MFGNISGIVT PIAIGYIVGT TGSFNGALIY
VGVHALIAVL SYLVLVGDIK RIELKPVAGQ
SEQ ID NO. 48: HSRA ORGANISM NAME: ESCHERICHIA COLI
MSDKKKRSMA GLPWIAAMAF FMQALDATIL NTALPAIAHS LNRSPLAMQS AIISYTLTVA
MLIPVSGWLA DRFGTRRIFT LAVSLFTLGS LACALSNSLP QLVVFRVIQG IGGAMMMPVA
RLALLRAYPR NELLPVLNFV AMPGLVGPIL GPVLGGVLVT WATWHWIFLI NIPIGIAGLL
YARKHMPNFT TARRRFDITG FLLFGLSLVL FSSGIELFGE KIVASWIALT VIVTSIGLLL
LYILHARRTP NPLISLDLFK TRTFSIGIVG NIATRLGTGC VPFLMPLMLQ VGFGYQAFIA
GCMMAPTALG SIIAKSMVTQ VLRRLGYRHT LVGITVIIGL MIAQFSLQSP AMAIWMLILP
LFILGMAMST QFTAMNTITL ADLTDDNASS GNSVLAVTQQ LSISLGVAVS AAVLRVYEGM
EGTTTVEQFH YTFITMGIIT VASAAMFMLL KTTDGNNLIK RQRKSKPNRV PSESE
SEQ ID NO. 49: LEUE ORGANISM NAME : ESCHERICHIA COLI
MFAEYGVLNY WTYLVGAIFI VLVPGPNTLF VLKNSVSSGM KGGYLAACGV FIGDAVLMFL
AWAGVATLIK TTPILFNIVR YLGAFYLLYL GSKILYATLK GKNSEAKSDE PQYGAIFKRA
LILSLTNPKA ILFYVSFFVQ FIDVNAPHTG ISFFILAATL ELVSFCYLSF LIISGAFVTQ
YIRTKKKLAK VGNSLIGLMF VGFAARLATL QS
SEQ ID NO. 50: MDLB ORGANISM NAME : ESCHERICHIA COLI
MRSFSQLWPT LKRLLAYGSP WRKPLGIAVL MMWVAAAAEV SGPLLISYFI DNMVAKNNLP
LKVVAGLAAA YVGLQLFAAG LHYAQSLLFN RAAVGVVQQL RTDVMDAALR QPLSEFDTQP
VGQVISRVTN DTEVIRDLYV TVVATVLRSA ALVGAMLVAM FSLDWRMALV AIMIFPVVLV
VMVIYQRYST PIVRRVRAYL ADINDGFNEI INGMSVIQQF RQQARFGERM GEASRSHYMA
RMQTLRLDGF LLRPLLSLFS SLILCGLLML FGFSASGTIE VGVLYAFISY LGRLNEPLIE
LTTQQAMLQQ AVVAGERVFE LMDGPRQQYG NDDRPLQSGT IEVDNVSFAY RDDNLVLKNI
NLSVPSRNFV ALVGHTGSGK STLASLLMGY YPLTEGEIRL DGRPLSSLSH SALRQGVAMV
QQDPVVLADT FLANVTLGRD ISEERVWQAL ETVQLAELAR SMSDGIYTPL GEQGNNLSVG
 
QKQLLALARV LVETPQILIL DEATASIDSG TEQAIQHALA AVREHTTLVV IAHRLSTIVD
ADTILVLHRG QAVEQGTHQQ LLAAQGRYWQ MYQLQLAGEE LAASVREEES LSA
SEQ ID NO. 51: MDTD ORGANISM NAME : ESCHERICHIA COLI
MTDLPDSTRW QLWIVAFGFF MQSLDTTIVN TALPSMAQSL GESPLHMHMV IVSYVLTVAV
MLPASGWLAD KVGVRNIFFT AIVLFTLGSL FCALSGTLNE LLLARALQGV GGAMMVPVGR
LTVMKIVPRE QYMAAMTFVT LPGQVGPLLG PALGGLLVEY ASWHWIFLIN IPVGIIGAIA
TLLLMPNYTM QTRRFDLSGF LLLAVGMAVL TLALDGSKGT GLSPLTIAGL VAVGVVALVL
YLLHARNNNR ALFSLKLFRT RTFSLGLAGS FAGRIGSGML PFMTPVFLQI GLGFSPFHAG
LMMIPMVLGS MGMKRIVVQV VNRFGYRRVL VATTLGLSLV TLLFMTTALL GWYYVLPFVL
FLQGMVNSTR FSSMNTLTLK DLPDNLASSG NSLLSMIMQL SMSIGVTIAG LLLGLFGSQH
VSVDSGTTQT VFMYTWLSMA LIIALPAFIF ARVPNDTHQN VAISRRKRSA Q
SEQ ID NO. 52: MDTG ORGANISM NAME : ESCHERICHIA COLI
MSPCENDTPI NWKRNLIVAW LGCFLTGAAF SLVMPFLPLY VEQLGVTGHS ALNMWSGIVF
SITFLFSAIA SPFWGGLADR KGRKLMLLRS ALGMGIVMVL MGLAQNIWQF LILRALLGLL
GGFVPNANAL IATQVPRNKS GWALGTLSTG GVSGALLGPM AGGLLADSYG LRPVFFITAS
VLILCFFVTL FCIREKFQPV SKKEMLHMRE VVTSLKNPKL VLSLFVTTLI IQVATGSIAP
ILTLYVRELA GNVSNVAFIS GMIASVPGVA ALLSAPRLGK LGDRIGPEKI LITALIFSVL
LLIPMSYVQT PLQLGILRFL LGAADGALLP AVQTLLVYNS SNQIAGRIFS YNQSFRDIGN
VTGPLMGAAI SANYGFRAVF LVTAGVVLFN AVYSWNSLRR RRIPQVSN
SEQ ID NO. 53: MDTL ORGANISM NAME : ESCHERICHIA COLI
MSRFLICSFA LVLLYPAGID MYLVGLPRIA ADLNASEAQL HIAFSVYLAG MAAAMLFAGK
VADRSGRKPV AIPGAALFII ASVFCSLAET STLFLAGRFL QGLGAGCCYV VAFAILRDTL
DDRRRAKVLS LLNGITCIIP VLAPVLGHLI MLKFPWQSLF WAMAMMGIAV LMLSLFILKE
TRPAAPAASD KPRENSESLL NRFFLSRVVI TTLSVSVILT FVNTSPVLLM EIMGFERGEY
ATIMALTAGV SMTVSFSTPF ALGIFKPRTL MITSQVLFLA AGITLAVSPS HAVSLFGITL
ICAGFSVGFG VAMSQALGPF SLRAGVASST LGIAQVCGSS LWIWLAAVVG IGAWNMLIGI
LIACSIVSLL LIMFVAPGRP VAAHEEIHHH A
SEQ ID NO. 54: MDTM ORGANISM NAME : ESCHERICHIA COLI
MPRFFTRHAA TLFFPMALIL YDFAAYLSTD LIQPGIINVV RDFNADVSLA PAAVSLYLAG
GMALQWLLGP LSDRIGRRPV LITGALIFTL ACAATMFTTS MTQFLIARAI QGTSICFIAT
VGYVTVQEAF GQTKGIKLMA IITSIVLIAP IIGPLSGAAL MHFMHWKVLF AIIAVMGFIS
FVGLLLAMPE TVKRGAVPFS AKSVLRDFRN VFCNRLFLFG AATISLSYIP MMSWVAVSPV
 
ILIDAGSLTT SQFAWTQVPV FGAVIVANAI VARFVKDPTE PRFIWRAVPI QLVGLSLLIV
GNLLSPHVWL WSVLGTSLYA FGIGLIFPTL FRFTLFSNKL PKGTVSASLN MVILMVMSVS
VEIGRWLWFN GGRLPFHLLA VVAGVIVVFT LAGLLNRVRQ HQAAELVEEQ
SEQ ID NO. 55: MHPT ORGANISM NAME : ESCHERICHIA COLI
MSTRTPSSSS SRLMLTIGLC FLVALMEGLD LQAAGIAAGG IAQAFALDKM QMGWIFSAGI
LGLLPGALVG GMLADRYGRK RILIGSVALF GLFSLATAIA WDFPSLVFAR LMTGVGLGAA
LPNLIALTSE AAGPRFRGTA VSLMYCGVPI GAALAATLGF AGANLAWQTV FWVGGVVPLI
LVPLLMRWLP ESAVFAGEKQ SAPPLRALFA PETATATLLL WLCYFFTLLV VYMLINWLPL
LLVEQGFQPS QAAGVMFALQ MGAASGTLML GALMDKLRPV TMSLLIYSGM LASLLALGTV
SSFNGMLLAG FVAGLFATGG QSVLYALAPL FYSSQIRATG VGTAVAVGRL GAMSGPLLAG
KMLALGTGTV GVMAASAPGI LVAGLAVFIL MSRRSRIQPC ADA
SEQ ID NO. 56: RHTA ORGANISM NAME : ESCHERICHIA COLI
MPGSLRKMPV WLPIVILLVA MASIQGGASL AKSLFPLVGA PGVTALRLAL GTLILIAFFK
PWRLRFAKEQ RLPLLFYGVS LGGMNYLFYL SIQTVPLGIA VALEFTGPLA VALFSSRRPV
DFVWVVLAVL GLWFLLPLGQ DVSHVDLTGC ALALGAGACW AIYILSGQRA GAEHGPATVA
IGSLIAALIF VPIGALQAGE ALWHWSVIPL GLAVAILSTA LPYSLEMIAL TRLPTRTFGT
LMSMEPALAA VSGMIFLGET LTPIQLLALG AIIAASMGST LTVRKESKIK ELDIN
SEQ ID NO. 57: RHTB ORGANISM NAME : ESCHERICHIA COLI
MTLEWWFAYL LTSIILSLSP GSGAINTMTT SLNHGYRGAV ASIAGLQTGL AIHIVLVGVG
LGTLFSRSVI AFEVLKWAGA AYLIWLGIQQ WRAAGAIDLK SLASTQSRRH LFQRAVFVNL
TNPKSIVFLA ALFPQFIMPQ QPQLMQYIVL GVTTIVVDII VMIGYATLAQ RIALWIKGPK
QMKALNKIFG SLFMLVGALL ASARHA
SEQ ID NO. 58: RHTC ORGANISM NAME : ESCHERICHIA COLI
MLMLFLTVAM VHIVALMSPG PDFFFVSQTA VSRSRKEAMM GVLGITCGVM VWAGIALLGL
HLIIEKMAWL HTLIMVGGGL YLCWMGYQML RGALKKEAVS APAPQVELAK SGRSFLKGLL
TNLANPKAII YFGSVFSLFV GDNVGTTARW GIFALIIVET LAWFTVVASL FALPQMRRGY
QRLAKWIDGF AGALFAGFGI HLIISR
SEQ ID NO. 59: YAHN ORGANISM NAME : ESCHERICHIA COLI
MMQLVHLFMD EITMDPLHAV YLTVGLFVIT FFNPGANLFV VVQTSLASGR RAGVLTGLGV
ALGDAFYSGL GLFGLATLIT QCEEIFSLIR IVGGAYLLWF AWCSMRRQST PQMSTLQQPI
SAPWYVFFRR GLITDLSNPQ TVLFFISIFS VTLNAETPTW ARLMAWAGIV LASIIWRVFL
SQAFSLPAVR RAYGRMQRVA SRVIGAIIGV FALRLIYEGV TQR
 
SEQ ID NO. 60: YAJR ORGANISM NAME : ESCHERICHIA COLI
MNDYKMTPGE RRATWGLGTV FSLRMLGMFM VLPVLTTYGM ALQGASEALI GIAIGIYGLT
QAVFQIPFGL LSDRIGRKPL IVGGLAVFAA GSVIAALSDS IWGIILGRAL QGSGAIAAAV
MALLSDLTRE QNRTKAMAFI GVSFGITFAI AMVLGPIITH KLGLHALFWM IAILATTGIA
LTIWVVPNSS THVLNRESGM VKGSFSKVLA EPRLLKLNFG IMCLHILLMS TFVALPGQLA
DAGFPAAEHW KVYLATMLIA FGSVVPFIIY AEVKRKMKQV FVFCVGLIVV AEIVLWNAQT
QFWQLVVGVQ LFFVAFNLME ALLPSLISKE SPAGYKGTAM GVYSTSQFLG VAIGGSLGGW
INGMFDGQGV FLAGAMLAAV WLTVASTMKE PPYVSSLRIE IPANIAANEA LKVRLLETEG
IKEVLIAEEE HSAYVKIDSK VTNRFEIEQA IRQA
SEQ ID NO. 61: YBBP ORGANISM NAME: ESCHERICHIA COLI
MIARWFWREW RSPSLLIVWL ALSLAVACVL ALGNISDRME KGLSQQSREF MAGDRALRSS
REVPQAWLEE AQKRGLKVGK QLTFATMTFA GDTPQLANVK AVDDIYPMYG DLQTNPPGLK
PQAGSVLLAP RLMALLNLKT GDTIDVGDAT LRIAGEVIQE PDSGFNPFQM APRLMMNLAD
VDKTGAVQPG SRVTWRYKFG GNENQLDGYE KWLLPQLKPE QRWYGLEQDE GALGRSMERS
QQFLLLSALL TLLLAVAAVA VAMNHYCRSR YDLVAILKTL GAGRAQLRKL IVGQWLMVLT
LSAVTGGAIG LLFENVLMVL LKPVLPAALP PASLWPWLWA LGTMTVISLL VGLRPYRLLL
ATQPLRVLRN DVVANVWPLK FYLPIVSVVV VLLLAGLMGG SMLLWAVLAG AVVLALLCGV
LGWMLLNVLR RMTLKSLPLR LAVSRLLRQP WSTLSQLSAF SLSFMLLALL LVLRGDLLDR
WQQQLPPESP NYFLINIATE QVAPLKAFLA EHQIVPESFY PVVRARLTAI NDKPTEGNED
EALNRELNLT WQNTRPDHNP IVAGNWPPKA DEVSMEEGLA KRLNVALGDT VTFMGDTQEF
RAKVTSLRKV DWESLRPNFY FIFPEGALDG QPQSWLTSFR WENGNGMLTQ LNRQFPTISL
LDIGAILKQV GQVLEQVSRA LEVMVVLVTA CGMLLLLAQV QVGMRQRHQE LVVWRTLGAG
KKLLRTTLWC EFAMLGFVSG LVAAIGAETA LAVLQAKVFD FPWEPDWRLW IVLPCSGALL
LSLFGGWLGA RLVKGKALFR QFAG
SEQ ID NO.62: YBIF (RHTA) ORGANISM NAME : ESCHERICHIA COLI
MPGSLRKMPV WLPIVILLVA MASIQGGASL AKSLFPLVGA PGVTALRLAL GTLILIAFFK
PWRLRFAKEQ RLPLLFYGVS LGGMNYLFYL SIQTVPLGIA VALEFTGPLA VALFSSRRPV
DFVWVVLAVL GLWFLLPLGQ DVSHVDLTGC ALALGAGACW AIYILSGQRA GAEHGPATVA
IGSLIAALIF VPIGALQAGE ALWHWSVIPL GLAVAILSTA LPYSLEMIAL TRLPTRTFGT
LMSMEPALAA VSGMIFLGET LTPIQLLALG AIIAASMGST LTVRKESKIK ELDIN
SEQ ID NO.63: YBJJ ORGANISM NAME : ESCHERICHIA COLI
 
MTVNSSRNAL KRRTWALFMF FFLPGLLMAS WATRTPAIRD ILSVSIAEMG GVLFGLSIGS
MSGILCSAWL VKRFGTRNVI LVTMSCALIG MMILSLALWL TSPLLFAVGL GVFGASFGSA
EVAINVEGAA VEREMNKTVL PMMHGFYSLG TLAGAGVGMA LTAFGVPATV HILLAALVGI
APIYIAIQAI PDGTGKNAAD GTQHGEKGVP FYRDIQLLLI GVVVLAMAFA EGSANDWLPL
LMVDGHGFSP TSGSLIYAGF TLGMTVGRFT GGWFIDRYSR VAVVRASALM GALGIGLIIF
VDSAWVAGVS VVLWGLGASL GFPLTISAAS DTGPDAPTRV SVVATTGYLA FLVGPPLLGY
LGEHYGLRSA MLVVLALVIL AAIVAKAVAK PDTKTQTAME NS
SEQ ID NO. 64: YCAP ORGANISM NAME: ESCHERICHIA COLI
MKAFDLHRMA FDKVPFDFLG EVALRSLYTF VLVFLFLKMT GRRGVRQMSL FEVLIILTLG
SAAGDVAFYD DVPMVPVLIV FITLALLYRL VMWLMAHSEK LEDLLEGKPV VIIEDGELAW
SKLNNSNMTE FEFFMELRLR GVEQLGQVRL AILETNGQIS VYFFEDDKVK PGLLILPSDC
TQRYKVVPES ADYACIRCSE IIHMKAGEKQ LCPRCANPEW TKASRAKRVT
SEQ ID NO. 65: YDCO ORGANISM NAME : ESCHERICHIA COLI
MRLFSIPPPT LLAGFLAVLI GYASSAAIIW QAAIVAGATT AQISGWMTAL GLAMGVSTLT
LTLWYRVPVL TAWSTPGAAL LVTGLQGLTL NEAIGVFIVT NALIVLCGIT GLFARLMRII
PHSLAAAMLA GILLRFGLQA FASLDGQFTL CGSMLLVWLA TKAVAPRYAV IAAMIIGIVI
VIAQGDVVTT DVVFKPVLPT YITPDFSFAH SLSVALPLFL VTMASQNAPG IAAMKAAGYS
APVSPLIVFT GLLALVFSPF GVYSVGIAAI TAAICQSPEA HPDKDQRWLA AAVAGIFYLL
AGLFGSAITG MMAALPVSWI QMLAGLALLS TIGGSLYQAL HNERERDAAV VAFLVTASGL
TLVGIGSAFW GLIAGGVCYV VLNLIADRNRY
SEQ ID NO. 66: YDDG ORGANISM NAME : ESCHERICHIA COLI
MTRQKATLIG LIAIVLWSTM VGLIRGVSEG LGPVGGAAAI YSLSGLLLIF TVGFPRIRQI
PKGYLLAGSL LFVSYEICLA LSLGYAATHH QAIEVGMVNY LWPSLTILFA ILFNGQKTNW
LIVPGLLLAL VGVCWVLGGD NGLHYDEIIN NITTSPLSYF LAFIGAFIWA AYCTVTNKYA
RGFNGITVFV LLTGASLWVY YFLTPQPEMI FSTPVMIKLI SAAFTLGFAY AAWNVGILHG
NVTIMAVGSY FTPVLSSALA AVLLSAPLSF SFWQGALMVC GGSLLCWLAT RRG
SEQ ID NO. 67: YDED (EAMA) ORGANISM NAME : ESCHERICHIA COLI
MSRKDGVLAL LVVVVWGLNF VVIKVGLHNM PPLMLAGLRF MLVAFPAIFF VARPKVPLNL
LLGYGLTISF AQFAFLFCAI NFGMPAGLAS LVLQAQAFFT IMLGAFTFGE RLHGKQLAGI
ALAIFGVLVL IEDSLNGQHV AMLGFMLTLA AAFSWACGNI FNKKIMSHST RPAVMSLVIW
SALIPIIPFF VASLILDGSA TMIHSLVTID MTTILSLMYL AFVATIVGYG IWGTLLGRYE
TWRVAPLSLL VPVVGLASAA LLLDERLTGL QFLGAVLIMT GLYINVFGLR WRKAVKVGS
 
SEQ ID NO. 68: YDGE (MDTI) ORGANISM NAME : ESCHERICHIA COLI
MAQFEWVHAA WLALAIVLEI VANVFLKFSD GFRRKIFGLL SLAAVLAAFS ALSQAVKGID
LSVAYALWGG FGIAATLAAG WILFGQRLNR KGWIGLVLLL AGMIMVKLA
SEQ ID NO. 69: YDHC ORGANISM NAME : ESCHERICHIA COLI
MQPGKRFLVW LAGLSVLGFL ATDMYLPAFA AIQADLQTPA SAVSASLSLF LAGFAAAQLL
WGPLSDRYGR KPVLLIGLTI FALGSLGMLW VENAATLLVL RFVQAVGVCA AAVIWQALVT
DYYPSQKVNR IFAAIMPLVG LSPALAPLLG SWLLVHFSWQ AIFATLFAIT VVLILPIFWL
KPTTKARNNS QDGLTFTDLL RSKTYRGNVL IYAACSASFF AWLTGSPFIL SEMGYSPAVI
GLSYVPQTIA FLIGGYGCRA ALQKWQGKQL LPWLLVLFAV SVIATWAAGF ISHVSLVEIL
IPFCVMAIAN GAIYPIVVAQ ALRPFPHATG RAAALQNTLQ LGLCFLASLV VSWLISISTP
LLTTTSVMLS TVVLVALGYM MQRCEEVGCQ NHGNAEVAHS ESH
SEQ ID NO. 70: YDHP ORGANISM NAME : ESCHERICHIA COLI
MKINYPLLAL AIGAFGIGTT EFSPMGLLPV IARGVDVSIP AAGMLISAYA VGVMVGAPLM
TLLLSHRARR SALIFLMAIF TLGNVLSAIA PDYMTLMLSR ILTSLNHGAF FGLGSVVAAS
VVPKHKQASA VATMFMGLTL ANIGGVPAAT WLGETIGWRM SFLATAGLGV ISMVSLFFSL
PKGGAGARPE VKKELAVLMR PQVLSALLTT VLGAGAMFTL YTYISPVLQS ITHATPVFVT
AMLVLIGVGF SIGNYLGGKL ADRSVNGTLK GFLLLLMVIM LAIPFLARNE FGAAISMVVW
GAATFAVVPP LQMRVMRVAS EAPGLSSSVN IGAFNLGNAL GAAAGGAVIS AGLGYSFVPV
MGAIVAGLAL LLVFMSARKQ PETVCVANS
SEQ ID NO. 71: YDIN ORGANISM NAME: ESCHERICHIA COLI
MSQNKAFSTP FILAVLCIYF SYFLHGISVI TLAQNMSSLA EKFSTDNAGI AYLISGIGLG
RLISILFFGV ISDKFGRRAV ILMAVIMYLL FFFGIPACPN LTLAYGLAVC VGIANSALDT
GGYPALMECF PKASGSAVIL VKAMVSFGQM FYPMLVSYML LNNIWYGYGL IIPGILFVLI
TLMLLKSKFP SQLVDASVTN ELPQMNSKPL VWLEGVSSVL FGVAAFSTFY VIVVWMPKYA
MAFAGMSEAE ALKTISYYSM GSLVCVFIFA ALLKKMVRPI WANVFNSALA TITAAIIYLY
PSPLVCNAGA FVIGFSAAGG ILQLGVSVMS EFFPKSKAKV TSIYMMMGGL ANFVIPLITG
YLSNIGLQYI IVLDFTFALL ALITAIIVFI RYYRVFIIPE NDVRFGERKF CTRLNTIKHR
G
SEQ ID NO. 72: YDIM ORGANISM NAME: ESCHERICHIA COLI
MKNPYFPTAL GLYFNYLVHG MGVLLMSLNM ASLETLWQTN AAGVSIVISS LGIGRLSVLL
FAGLLSDRFG RRPFIMLGMC CYMAFFFGIL QTNNIIIAYV FGFLAGMANS FLDAGTYPSL
MEAFPRSPGT ANILIKAFVS SGQFLLPLII SLLVWAELWF GWSFMIAAGI MFINALFLYR
 
CTFPPHPGRR LPVIKKTTSS TEHRCSIIDL ASYTLYGYIS MATFYLVSQW LAQYGQFVAG
MSYTMSIKLL SIYTVGSLLC VFITAPLIRN TVRPTTLLML YTFISFIALF TVCLHPTFYV
VIIFAFVIGF TSAGGVVQIG LTLMAERFPY AKGKATGIYY SAGSIATFTI PLITAHLSQR
SIADIMWFDT AIAAIGFLLA LFIGLRSRKK TRHHSLKENV APGG
SEQ ID NO. 73: YDJE ORGANISM NAME : ESCHERICHIA COLI
MEQYDQIGAR LDRLPLARFH YRIFGIISFS LLLTGFLSYS GNVVLAKLVS NGWSNNFLNA
AFTSALMFGY FIGSLTGGFI GDYFGRRRAF RINLLIVGIA ATGAAFVPDM YWLIFFRFLM
GTGMGALIMV GYASFTEFIP ATVRGKWSAR LSFVGNWSPM LSAAIGVVVI AFFSWRIMFL
LGGIGILLAW FLSGKYFIES PRWLAGKGQI AGAECQLREV EQQIEREKSI RLPPLTSYQS
NSKVKVIKGT FWLLFKGEML RRTLVAITVL IAMNISLYTI TVWIPTIFVN SGIDVDKSIL
MTAVIMIGAP VGIFIAALII DHFPRRLFGS TLLIIIAVLG YIYSIQTTEW AILIYGLVMI
FFLYMYVCFA SAVYIPELWP THLRLRGSGF VNAVGRIVAV FTPYGVAALL THYGSITVFM
VLGVMLLLCA LVLSIFGIET RKVSLEEISE VN
SEQ ID NO. 74: YDJF ORGANISM NAME: ESCHERICHIA COLI





SEQ ID NO. 75: YDJI ORGANISM NAME : ESCHERICHIA COLI
MLADIRYWEN DATNKHYAIA HFNVWNAEML MGVIDAAEEA KSPVIISFGT GFVGNTSFED
FSHMMVSMAQ KATVPVITHW DHGRSMEIIH NAWTHGMNSL MRDASAFDFE ENIRLTKEAV
DFFHPLGIPV EAELGHVGNE TVYEEALAGY HYTDPDQAAE FVERTGCDSL AVAIGNQHGV
YTSEPQLNFE VVKRVRDAVS VPLVLHGASG ISDADIKTAI SLGIAKINIH TELCQAAMVA
VKENQDQPFL HLEREVRKAV KERALEKIKL FGSDGKAE
SEQ ID NO. 76: YDJK ORGANISM NAME : ESCHERICHIA COLI
MEQITKPHCG ARLDRLPDCR WHSSMFAIVA FGLLVCWSNA VGGLILAQLK ALGWTDNSTT
ATFSAITTAG MFLGALVGGI IGDKTGRRNA FILYEAIHIA SMVVGAFSPN MDFLIACRFV
MGVGLGALLV TLFAGFTEYM PGRNRGTWSS RVSFIGNWSY PLCSLIAMGL TPLISAEWNW
RVQLLIPAIL SLIATALAWR YFPESPRWLE SRGRYQEAEK VMRSIEEGVI RQTGKPLPPV
VIADDGKAPQ AVPYSALLTG VLLKRVILGS CVLIAMNVVQ YTLINWLPTI FMTQGINLKD
SIVLNTMSMF GAPFGIFIAM LVMDKIPRKT MGVGLLILIA VLGYIYSLQT SMLLITLIGF
 
FLITFVYMYV CYASAVYVPE IWPTEAKLRG SGLANAVGRI SGIAAPYAVA VLLSSYGVTG
VFILLGAVSI IVAIAIATIG IETKGVSVES LSIDAVANK
SEQ ID NO. 77: YEAN ORGANISM NAME : ESCHERICHIA COLI
MTCSTSLSGK NRIVLIAGIL MIATTLRVTF TGAAPLLDTI RSAYSLTTAQ TGLLTTLPLL
AFALISPLAA PVARRFGMER SLFAALLLIC AGIAIRSLPS PYLLFGGTAV IGGGIALGNV
LLPGLIKRDF PHSVARLTGA YSLTMGAAAA LGSAMVVPLA LNGFGWQGAL LMLMCFPLLA
LFLWLPQWRS QQHANLSTSR ALHTRGIWRS PLAWQVTLFL GINSLVYYVI IGWLPAILIS
HGYSEAQAGS LHGLLQLATA APGLLIPLFL HHVKDQRGIA AFVALMCAVG AVGLCFMPAH
AITWTLLFGF GSGATMILGL TFIGLRASSA HQAAALSGMA QSVGYLLAAC GPPLMGKIHD
ANGNWSVPLM GVAILSLLMA IFGLCAGRDK EIR
SEQ ID NO. 78: YEAS (LEUE) ORGANISM NAME : ESCHERICHIA COLI
MFAEYGVLNY WTYLVGAIFI VLVPGPNTLF VLKNSVSSGM KGGYLAACGV FIGDAVLMFL
AWAGVATLIK TTPILFNIVR YLGAFYLLYL GSKILYATLK GKNSEAKSDE PQYGAIFKRA
LILSLTNPKA ILFYVSFFVQ FIDVNAPHTG ISFFILAATL ELVSFCYLSF LIISGAFVTQ
YIRTKKKLAK VGNSLIGLMF VGFAARLATL QS
SEQ ID NO. 79: YEBS ORGANISM NAME : ESCHERICHIA COLI
MALNTPQITP TKKITVRAIG EELPRGDYQR CPQCDMLFSL PEINSHQSAY CPRCQAKIRD
GRDWSLTRLA AMAFTMLLLM PFAWGEPLLH IWLLGIRIDA NVMQGIWQMT KQGDAITGSM
VFFCVIGAPL ILVTSIAYLW FGNRLGMNLR PVLLMLERLK EWVMLDIYLV GIGVASIKVQ
DYAHIQAGVG LFSFVALVIL TTVTLSHLNV EELWERFYPQ RPATRRDEKL RVCLGCHFTG
YPDQRGRCPR CHIPLRLRRR HSLQKCWAAL LASIVLLLPA NLLPISIIYL NGGRQEDTIL
SGIMSLASSN IAVAGIVFIA SILVPFTKVI VMFTLLLSIH FKCQQGLRTR ILLLRMVTWI
GRWSMLDLFV ISLTMSLINR DQILAFTMGP AAFYFGAAVI LTILAVEWLD SRLLWDAHES
GNARFDD
SEQ ID NO. 80: YEDA ORGANISM NAME : ESCHERICHIA COLI
MRFRQLLPLF GALFALYIIW GSTYFVIRIG VESWPPLMMA GVRFLAAGIL LLAFLLLRGH
KLPPLRPLLN AALIGLLLLA VGNGMVTVAE HQNVPSGIAA VVVATVPLFT LCFSRLFGIK
TRKLEWVGIA IGLAGIIMLN SGGNLSGNPW GAILILIGSI SWAFGSVYGS RITLPVGMMA
GAIEMLAAGV VLMIASMIAG EKLTALPSLS GFLAVGYLAL FGSIIAINAY MYLIRNVSPA
LATSYAYVNP VVAVLLGTGL GGETLSKIEW LALGVIVFAV VLVTLGKYLF PAKPVVAPVI
QDASSE
SEQ ID NO. 81: YEEO ORGANISM NAME : ESCHERICHIA COLI
 
MLRHILTAKN LLSNPIFKFP NCLPFLSTVC CICRQFVGEN LCSFADSPSL FEMWFHFLQL
RSALNISSAL RQVVHGTRWH AKRKSYKVLF WREITPLAVP IFMENACVLL MGVLSTFLVS
WLGKDAMAGV GLADSFNMVI MAFFAAIDLG TTVVVAFSLG KRDRRRARVA TRQSLVIMTL
FAVLLATLIH HFGEQIIDFV AGDATTEVKA LALTYLELTV LSYPAAAITL IGSGALRGAG
NTKIPLLING SLNILNIIIS GILIYGLFSW PGLGFVGAGL GLTISRYIGA VAILWVLAIG
FNPALRISLK SYFKPLNFSI IWEVMGIGIP ASVESVLFTS GRLLTQMFVA GMGTSVIAGN
FIAFSIAALI NLPGSALGSA STIITGRRLG VGQIAQAEIQ LRHVFWLSTL GLTAIAWLTA
PFAGVMASFY TQDPQVKHVV VILIWLNALF MPIWSASWVL PAGFKGARDA RYAMWVSMLS
MWGCRVVVGY VLGIMLGWGV VGVWMGMFAD WAVRAVLFYW RMVTGRWLWK YPRPEPQKCE
KKPVVSE
SEQ ID NO. 82: YEGH ORGANISM NAME: ESCHERICHIA COLI

MEWIADPSIW AGLITLIVIE LVLGIDNLVF IAILAEKLPP KQRDRARVTG LLLAMLMRLL
LLASISWLVT LTQPLFSFRS FTFSARDLIM LFGGFFLLFK ATMELNERLE GKDSNNPTQR
KGAKFWGVVT QIVVLDAIFS LDSVITAVGM VDHLLVMMAA VVIAISLMLM ASKPLTQFVN
SHPTIVILCL SFLLMIGFSL VAEGFGFVIP KGYLYAAIGF SVMIEALNQL AIFNRRRFLS
ANQTLRQRTT EAVMRLLSGQ KEDAELDAET ASMLVDHGNQ QIFNPQERRM IERVLNLNQR
TVSSIMTSRH DIEHIDLNAP EEEIRQLLER NQHTRLVVTD GDDAEDLLGV VHVIDLLQQS
LRGEPLNLRV LIRQPLVFPE TLPLLPALEQ FRNARTHFAF VVDEFGSVEG IVTLSDVTET
IAGNLPNEVE EIDARHDIQK NADGSWTANG HMPLEDLVQY VPLPLDEKRE YHTIAGLLME
YLQRIPKPGE EVQVGDYLLK TLQVESHRVQ KVQIIPLRKD GEMEYEV
SEQ ID NO. 83: YGGA (ARGO) ORGANISM NAME: ESCHERICHIA COLI
MFSYYFQGLA LGAAMILPLG PQNAFVMNQG IRRQYHIMIA LLCAISDLVL ICAGIFGGSA
LLMQSPWLLA LVTWGGVAFL LWYGFGAFKT AMSSNIELAS AEVMKQGRWK IIATMLAVTW
LNPHVYLDTF VVLGSLGGQL DVEPKRWFAL GTISASFLWF FGLALLAAWL APRLRTAKAQ
RIINLVVGCV MWFIALQLAR DGIAHAQALF S
SEQ ID NO. 84: YFCJ ORGANISM NAME : ESCHERICHIA COLI





 


SEQ ID NO. 85: YFIK (EAMB) ORGANISM NAME: ESCHERICHIA COLI
MTPTLLSAFW TYTLITAMTP GPNNILALSS ATSHGFRQST RVLAGMSLGF LIVMLLCAGI
SFSLAVIDPA AVHLLSWAGA AYIVWLAWKI ATSPTKEDGL QAKPISFWAS FALQFVNVKI
ILYGVTALST FVLPQTQALS WVVGVSVLLA MIGTFGNVCW ALAGHLFQRL FRQYGRQLNI
VLALLLVYCA VRIFY
SEQ ID NO. 86: YHJE ORGANISM NAME: ESCHERICHIA COLI
MQATATTLDH EQEYTPINSR NKVLVASLIG TAIEFFDFYI YATAAVIVFP HIFFPQGDPT
AATLQSLATF AIAFVARPIG SAVFGHFGDR VGRKATLVAS LLTMGISTVV IGLLPGYATI
GIFAPLLLAL ARFGQGLGLG GEWGGAALLA TENAPPRKRA LYGSFPQLGA PIGFFFANGT
FLLLSWLLTD EQFMSWGWRV PFIFSAVLVI IGLYVRVSLH ESPVFEKVAK AKKQVKIPLG
TLLTKHVRVT VLGTFIMLAT YTLFYIMTVY SMTFSTAAAP VGLGLPRNEV LWMLMMAVIG
FGVMVPVAGL LADAFGRRKS MVIITTLIIL FALFAFNPLL GSGNPILVFA FLLLGLSLMG
LTFGPMGALL PELFPTEVRY TGASFSYNVA SILGASVAPY IAAWLQTNYG LGAVGLYLAA
MAGLTLIALL LTHETRHQSL
SEQ ID NO. 87:YIDE ORGANISM NAME: ESCHERICHIA COLI
MSDIALTVSI LALVAVVGLF IGNVKFRGIG LGIGGVLFGG IIVGHFVSQA GMTLSSDMLH
VIQEFGLILF VYTIGIQVGP GFFASLRVSG LRLNLFAVLI VIIGGLVTAI LHKLFDIPLP
VVLGIFSGAV TNTPALGAGQ QILRDLGTPM EMVDQMGMSY AMAYPFGICG ILFTMWMLRV
IFRVNVETEA QQHESSRTNG GALIKTINIR VENPNLHDLA IKDVPILNGD KIICSRLKRE
ETLKVPSPDT IIQLGDLLHL VGQPADLHNA QLVIGQEVDT SLSTKGTDLR VERVVVTNEN
VLGKRIRDLH FKERYDVVIS RLNRAGVELV ASGDISLQFG DILNLVGRPS AIDAVANVLG
NAQQKLQQVQ MLPVFIGIGL GVLLGSIPVF VPGFPAALKL GLAGGPLIMA LILGRIGSIG
KLYWFMPPSA NLALRELGIV LFLSVVGLKS GGDFVNTLVN GEGLSWIGYG ALITAVPLIT
VGILARMLAK MNYLTMCGML AGSMTDPPAL AFANNLHPTS GAAALSYATV YPLVMFLRII
TPQLLAVLFW SIG
SEQ ID NO. 88: YIGK (RHTB) ORGANISM NAME: ESCHERICHIA COLI
MTLEWWFAYL LTSIILSLSP GSGAINTMTT SLNHGYRGAV ASIAGLQTGL AIHIVLVGVG
LGTLFSRSVI AFEVLKWAGA AYLIWLGIQQ WRAAGAIDLK SLASTQSRRH LFQRAVFVNL
TNPKSIVFLA ALFPQFIMPQ QPQLMQYIVL GVTTIVVDII VMIGYATLAQ RIALWIKGPK
QMKALNKIFG SLFMLVGALL ASARHA
 
SEQ ID NO. 89:YIGJ (RHTC) ORGANISM NAME : ESCHERICHIA COLI
MLMLFLTVAM VHIVALMSPG PDFFFVSQTA VSRSRKEAMM GVLGITCGVM VWAGIALLGL
HLIIEKMAWL HTLIMVGGGL YLCWMGYQML RGALKKEAVS APAPQVELAK SGRSFLKGLL
TNLANPKAII YFGSVFSLFV GDNVGTTARW GIFALIIVET LAWFTVVASL FALPQMRRGY
QRLAKWIDGF AGALFAGFGI HLIISR
SEQ ID NO. 90: YIJE ORGANISM NAME : ESCHERICHIA COLI
MSAAGKSNPL AISGLVVLTL IWSYSWIFMK QVTSYIGAFD FTALRCIFGA LVLFIVLLLR
GRGMRPTPFK YTLAIALLQT CGMVGLAQWA LVSGGAGKVA ILSYTMPFWV VIFAALFLGE
RLRRGQYFAI LIAAFGLFLV LQPWQLDFSS MKSAMLAILS GVSWGASAIV AKRLYARHPR
VDLLSLTSWQ MLYAALVMSV VALLVPQREI DWQPTVFWAL AYSAILATAL AWSLWLFVLK
NLPASIASLS TLAVPVCGVL FSWWLLGENP GAVEGSGIVL IVLALALVSR KKKEAVSVKRI

SEQ ID NO. 91: YJII (KPTA) ORGANISM NAME : ESCHERICHIA COLI

MAKYNEKELA DTSKFLSFVL RHKPEAIGIV LDREGWADID KLILCAQKAG KRLTRALLDT
VVATSDKKRF SYSSDGRCIR AVQGHSTSQV AISFAEKTPP QFLYHGTASR FLDEIKKQGL
IAGERHYVHL SADEATARKV GARHGSPVIL TVKAQEMAKR GLPFWQAENG VWLTSTVAVE
FLEW
SEQ ID NO. 92: YJIJ ORGANISM NAME : ESCHERICHIA COLI
MPSSTHPVER FSFSTALFGM LVLTLGMGLG RFLYTPMLPV MMAEGSFSFS QLSWIASGNY
AGYLAGSLLF SFGAFHQPSR LRPFLLASAL ASGLLILAMA WLPPFILVLL IRVLAGVASA
GMLIFGSTLI MQHTRHPFVL AALFSGVGIG IALGNEYVLA GLHFDLSSQT LWQGAGALSG
MMLIALTLLM PSKKHAITPM PLAKTEQQIM SWWLLAILYG LAGFGYIIVA TYLPLMAKDA
GSPLLTAHLW TLVGLSIVPG CFGWLWAAKR WGALPCLTAN LLVQAICVLL TLASDSPLLL
IISSLGFGGT FMGTTSLVMT IARQLSVPGN LNLLGFVTLI YGIGQILGPA LTSMLSNGTS
ALASATLCGA AALFIAALIS TVQLFKLQVV TS
SEQ ID NO. 93: YJIO (MDTM) ORGANISM NAME : ESCHERICHIA COLI
MPRFFTRHAA TLFFPMALIL YDFAAYLSTD LIQPGIINVV RDFNADVSLA PAAVSLYLAG
GMALQWLLGP LSDRIGRRPV LITGALIFTL ACAATMFTTS MTQFLIARAI QGTSICFIAT
VGYVTVQEAF GQTKGIKLMA IITSIVLIAP IIGPLSGAAL MHFMHWKVLF AIIAVMGFIS
FVGLLLAMPE TVKRGAVPFS AKSVLRDFRN VFCNRLFLFG AATISLSYIP MMSWVAVSPV
ILIDAGSLTT SQFAWTQVPV FGAVIVANAI VARFVKDPTE PRFIWRAVPI QLVGLSLLIV
 
GNLLSPHVWL WSVLGTSLYA FGIGLIFPTL FRFTLFSNKL PKGTVSASLN MVILMVMSVS
VEIGRWLWFN GGRLPFHLLA VVAGVIVVFT LAGLLNRVRQ HQAAELVEEQ
SEQ ID NO. 94: YKGH ORGANISM NAME : ESCHERICHIA COLI
MREQIKQDID LIEILFYLKK KIRVILFIMA ICMAMVLLFL YINKDNIKVI YSLKINQTTP
GILVSCDSNN NFACQTTMTE DVIQRITTFF HTSPDVKNRE IRLEWSGDKR ALPTAEEEIS
RVQASIIKWY ASEYHNGRQV LDEIQTPSAI NSELYTKMIY LTRNWSLYPN GDGCVTISSP
EIKNKYPAAI CLALGFFLSI VISVMFCLVK KMVDEYQQNS GQ
SEQ ID NO. 95: YPJD ORGANISM NAME : ESCHERICHIA COLI
MQRLEQRSPD AILLLFLIAQ TVDITMPVFA LLALVAYSVS LALIVPGLLQ KNGGWRRMAI
ISAVIALVCH AIALEARILP DGDSGQNLSL LNVGSLVSLM ICTVMTIVAS RNRGWLLLPI
VYAFALINLA LATFMPNEYI THLEATPGML VHIGLSLFSY ATLIIAALYA LQLAWIDYQL
KNKKLAFNQE MPPLMSIERK MFHITQIGVV LLTLTLCTGL FYMHNLFSME NIDKAVLSIV
AWFVYIVLLW GHYHEGWRGR RVVWFNVAGA VILTLAYFGS RIVQQLIS
SEQ ID NO. 96: YTFF ORGANISM NAME : ESCHERICHIA COLI
MPVMISGVLY ALLAGLMWGL IFVGPLIVPE YPAMLQSMGR YLALGLIALP IAWLGRVRLR
QLARRDWLTA LMLTMMGNLI YYFCLASAIQ RTGAPVSTMI IGTLPVVIPV FANLLYSQRD
GKLAWGKLAP ALICIGIGLA CVNIAELNHG LPDFDWARYT SGIVLALVSV VCWAWYALRN
ARWLRENPDK HPMMWATAQA LVTLPVSLIG YLVACYWLNT QTPDFSLPFG PRPLVFISLM
VAIAVLCSWV GALCWNVASQ LLPTVILGPL IVFETLAGLL YTFLLRQQMP PLMTLSGIAL
LVIGVVIAVR AKPEKPLTES VSES
SEQ ID NO. 97: YTFL ORGANISM NAME: ESCHERICHIA COLI
MLNSILVILC LIAVSAFFSM SEISLAASRK IKLKLLADEG NINAQRVLNM QENPGMFFTV
VQIGLNAVAI LGGIVGDAAF SPAFHSLFSR YMSAELSEQL SFILSFSLVT GMFILFADLT
PKRIGMIAPE AVALRIINPM RFCLYVCTPL VWFFNGLANI IFRIFKLPMV RKDDITSDDI
YAVVEAGALA GVLRKQEHEL IENVFELESR TVPSSMTPRE NVIWFDLHED EQSLKNKVAE
HPHSKFLVCN EDIDHIIGYV DSKDLLNRVL ANQSLALNSG VQIRNTLIVP DTLTLSEALE
SFKTAGEDFA VIMNEYALVV GIITLNDVMT TLMGDLVGQG LEEQIVARDE NSWLIDGGTP
IDDVMRVLDI DEFPQSGNYE TIGGFMMFML RKIPKRTDSV KFAGYKFEVV DIDNYRIDQL
LVTRIDSKAT ALSPKLPDAK DKEESVA
SEQ ID NO. 98: CHLOROFLEXUS AGGREGANS (CGMCR)
<400> 785

MET SER VAL THR GLY ARG LEU ALA GLY LYS ILE ALA LEU ILE THR GLY

1               5                   10                  15
GLY ALA GLY ASN ILE GLY SER GLU MET THR ARG ARG PHE LEU ALA GLU
            20                  25                  30
GLY ALA THR VAL ILE ILE SER GLY ARG ASN SER ALA LYS LEU ALA ALA
        35                  40                  45
LEU ALA GLU ARG LEU ARG SER GLU ALA GLY VAL PRO ALA LYS ARG ILE
    50                  55                  60
ASP LEU GLU VAL MET ASP GLY SER ASP PRO ALA ALA VAL ARG ALA GLY
65                  70                  75                  80
VAL ALA ALA ILE ILE GLY ARG HIS GLY HIS ILE ASP ILE LEU VAL ASN
                85                  90                  95
ASN ALA GLY SER THR GLY ALA GLN ARG ARG LEU ALA GLU ILE PRO LEU
            100                 105                 110
ASN GLU THR ASP ARG ASP LEU ASP ASP GLU GLU ALA LEU SER THR SER
        115                 120                 125
VAL ALA ASN LEU LEU GLY MET ALA TRP HIS LEU MET ARG ILE LEU SER
    130                 135                 140
PRO HIS MET PRO PRO GLY SER ALA ILE ILE ASN ILE SER THR ILE PHE
145                 150                 155                 160
SER ARG ALA GLU TYR TYR GLY ARG ILE PRO TYR VAL VAL PRO LYS ALA
                165                 170                 175
ALA LEU ASN THR LEU THR GLN ILE ALA ALA ARG GLU LEU GLY ILE ARG
            180                 185                 190
GLY ILE ARG VAL ASN THR ILE PHE PRO GLY PRO ILE GLU SER GLU ARG
        195                 200                 205
ILE GLN THR VAL PHE GLN ARG MET ASP GLN LEU LYS GLY ARG PRO GLU
    210                 215                 220
GLY ASP THR ALA SER GLN PHE LEU ALA THR MET ARG LEU TYR ARG ALA
225                 230                 235                 240
ASN ASP GLN GLY GLN LEU GLU ARG ARG PHE PRO THR ILE CYS ASP VAL
                245                 250                 255
ALA ASP ALA ALA VAL PHE LEU ALA SER ASP GLU ALA ALA ALA LEU THR

            260                 265                 270
GLY GLU THR ILE GLU VAL THR HIS GLY MET GLU LEU PRO THR SER SER
        275                 280                 285
GLU THR SER LEU LEU ALA ARG THR ASP LEU ARG THR ILE ASP ALA ASN
    290                 295                 300
GLY ARG THR THR LEU ILE CYS ALA GLY ASP GLN ILE GLU GLU VAL MET
305                 310                 315                 320
ALA LEU THR GLY MET LEU ARG THR CYS GLY SER GLU VAL ILE ILE GLY
                325                 330                 335
PHE ARG SER GLU ALA ALA LEU ALA GLN PHE GLU GLN ALA ILE GLY GLU
            340                 345                 350
SER ARG ARG LEU ALA GLY GLU SER PHE ILE PRO PRO ILE ALA LEU PRO
        355                 360                 365
ILE ASP LEU ARG ASN PRO SER THR ILE ASP ALA LEU PHE ASP TRP ALA
    370                 375                 380
GLY GLU ASN THR GLY GLY ILE HIS ALA ALA VAL ILE LEU PRO ALA SER
385                 390                 395                 400
GLY ARG GLU PRO ALA THR GLN VAL ILE ASP ILE ASP ASP ALA HIS VAL
                405                 410                 415
GLN ALA PHE LEU ASN ASP GLU ILE VAL GLY SER ILE ILE ILE ALA SER
            420                 425                 430
ARG LEU ALA ARG TYR TRP GLN ALA GLN ARG ILE ALA PRO GLY ALA ARG
        435                 440                 445
ALA ARG GLU PRO ARG VAL ILE PHE LEU SER ASN GLY ALA SER THR ALA
    450                 455                 460
GLY ASN PRO TYR GLY ARG ILE GLN SER ALA ALA ILE GLU GLN LEU ILE
465                 470                 475                 480
ARG VAL TRP ARG HIS GLU ALA ALA LEU ASP TYR GLU ARG ALA THR ALA
                485                 490                 495
ALA GLY GLU ARG VAL LEU PRO ALA VAL TRP ALA SER GLN ILE VAL ARG
            500                 505                 510
PHE ALA ASN ARG SER LEU GLU GLY LEU GLU PHE ALA CYS ALA TRP THR

        515                 520                 525
ALA GLN LEU LEU HIS SER GLN ARG ARG ILE ASN GLU ILE THR LEU THR
    530                 535                 540
ILE PRO ALA ASP ILE SER ALA THR THR GLY ALA ARG SER ALA SER VAL
545                 550                 555                 560
GLY TRP ALA GLU SER LEU ILE GLY LEU HIS LEU GLY LYS VAL ALA LEU
                565                 570                 575
ILE THR GLY GLY SER ALA GLY ILE GLY GLY GLN ILE GLY ARG LEU LEU
            580                 585                 590
ALA LEU SER GLY ALA ARG VAL MET LEU ALA ALA ARG ASP PRO HIS LYS
        595                 600                 605
LEU GLU GLN ILE GLN ALA THR ILE ARG ALA GLU LEU ALA GLU VAL GLY
    610                 615                 620
TYR THR ASP VAL GLU GLU ARG VAL GLN ILE ALA PRO GLY CYS ASP VAL
625                 630                 635                 640
SER SER GLU GLU GLN LEU VAL ASP LEU VAL GLU ARG THR LEU ALA ALA
                645                 650                 655
PHE GLY THR VAL ASP TYR LEU ILE ASN ASN ALA GLY ILE ALA GLY VAL
            660                 665                 670
GLU GLU MET VAL ILE ASP MET PRO VAL GLU GLY TRP ARG ASN THR LEU
        675                 680                 685
TYR ALA ASN LEU ILE SER ASN TYR SER LEU MET ARG LYS LEU ALA PRO
    690                 695                 700
LEU MET LYS LYS GLN GLY SER GLY TYR VAL LEU ASN VAL SER SER TYR
705                 710                 715                 720
PHE GLY GLY GLU LYS ASP ALA ALA ILE PRO TYR PRO ASN ARG ALA ASP
                725                 730                 735
TYR ALA VAL SER LYS ALA GLY GLN ARG ALA MET ALA GLU VAL PHE ALA
            740                 745                 750
ARG PHE LEU GLY PRO GLU ILE GLN ILE ASN ALA ILE ALA PRO GLY PRO
        755                 760                 765
VAL GLU GLY ASP ARG LEU ARG GLY THR GLY GLU ARG PRO GLY LEU PHE

    770                 775                 780
ALA ARG ARG ALA ARG LEU ILE LEU GLU ASN LYS ARG LEU ASN GLU LEU
785                 790                 795                 800
HIS ALA ALA LEU ILE THR ALA ALA ARG THR ASP ASN ARG PRO MET ARG
                805                 810                 815
GLU LEU VAL GLU LEU LEU LEU PRO ASN ASP VAL ALA ALA LEU ALA GLN
            820                 825                 830
HIS PRO ALA ALA PRO ASP VAL LEU ARG THR LEU ALA LYS ARG PHE GLN
        835                 840                 845
SER GLU GLY ASP PRO ALA ALA SER SER SER SER PHE LEU LEU ASN ARG
    850                 855                 860
SER ILE ALA ALA LYS LEU LEU ALA ARG LEU ILE ASN GLY GLY TYR ASP
865                 870                 875                 880
LEU PRO ALA ASP ILE PHE ALA ASN LEU ALA VAL PRO PRO ASP PRO PHE
                885                 890                 895
PHE THR ARG ALA GLN ILE ASP ARG GLU ALA ARG LYS VAL ARG ASP GLY
            900                 905                 910
ILE MET GLY MET LEU TYR LEU GLN ARG MET PRO THR GLU PHE ASP VAL
        915                 920                 925
ALA MET ALA THR VAL TYR TYR LEU ALA ASP ARG ASN VAL SER GLY GLU
    930                 935                 940
THR PHE HIS PRO SER GLY GLY LEU ARG TYR GLU ARG THR PRO THR GLY
945                 950                 955                 960
GLY GLU LEU PHE GLY LEU PRO ALA PRO GLU ARG LEU ALA GLU LEU VAL
                965                 970                 975
GLY SER THR VAL TYR LEU ILE GLY GLU HIS LEU THR GLU HIS LEU ASN
            980                 985                 990
LEU LEU ALA ARG ALA TYR LEU GLU ARG TYR GLY ALA ARG GLN VAL VAL
        995                 1000                1005
MET ILE VAL GLU THR GLU ALA GLY ALA GLU LYS MET ARG HIS LEU
    1010                1015                1020
LEU HIS ASP HIS VAL GLU ALA GLY ARG LEU PRO ILE ILE VAL ALA

    1025                1030                1035

GLY ASP GLN ILE GLU ALA ALA ILE ASP GLN ALA ILE ALA ASN TYR

    1040                1045                1050

GLY ARG PRO GLY PRO VAL VAL CYS THR PRO PHE ARG PRO LEU PRO

    1055                1060                1065

SER ALA PRO LEU VAL GLY ARG LYS ASP SER ASP TRP SER THR VAL

    1070                1075                1080

LEU SER GLU ALA GLU PHE ALA GLU LEU CYS GLU HIS GLN LEU THR

    1085                1090                1095

HIS HIS PHE ARG VAL ALA ARG LYS ILE ALA LEU SER ASP GLY ALA

    1100                1105                1110

SER LEU ALA LEU VAL THR PRO GLU THR THR ALA THR SER SER THR

    1115                1120                1125

GLU GLN PHE ALA LEU ALA ASN PHE VAL LYS THR THR LEU HIS ALA

    1130                1135                1140

PHE THR ALA THR ILE GLY VAL GLU SER GLU ARG THR ALA GLN ARG

    1145                1150                1155

ILE LEU ILE ASN GLN VAL ASP LEU THR ARG ARG ALA ARG ALA GLU

    1160                1165                1170

GLU PRO ARG ASP PRO ARG GLU ARG GLN GLN GLU LEU GLU ARG PHE

    1175                1180                1185

ILE GLU ALA VAL LEU LEU VAL THR ALA PRO LEU PRO PRO GLU ALA

    1190                1195                1200

ASP THR ARG TYR ALA GLY ARG ILE HIS ARG GLY ARG ALA ILE THR VAL

    1205                1210                1215

SEQ ID NO. 99: MCR OSCILLOCHLORIS TRICHOIDES (OTMCR)

MFMTRLNDKIALITGGAGTIGEVITRRYLEEGATVVMAGRNRDKLDRYRERLITEFHALP

ERVMVVRMDGSSNAEVRMGIAAVVAHFGRIDILVNNAGSAGARQRLPAIPLLRSELQADE

TETLADSIGNLIGITWNLIRAAAPFMPAGSSVINISTIFARTDYYGRIPYVVPKAALHAL

TLAAATELGERGIRVNQINPGPIDSDRIRTVFRRMDELKGVPEQSTADGFFQMMRLRRPN

AEGDLVKGFPKTLDVANVAVFLGSAESAALSGETLDVTHGMAVPTESRTTLTSRPGLRAV

DGSGHTTLICVGDQIEEAAALTGVLRACGAEVVIGFRSRAAIARFDHLIERGRHLPSQEH

VAPVLLYLNPTEPESIDQALRWMATNLDLPTSVIILPAQRQPLPPSVVRASDEEVAYFLR
DELSGMIVLASRLARFWQQATLAPGNAPIQPRVLFMTNPDDGQGNLYAEILRAGVEQLCR
VWRHESQLDYTRLAQMDAHPPHIRPVWANQLVRFANNEQENLEYCCAWVAKILLSERTIE
ELNLYLPRQIGSTTGSRQPSFGWAENLIGLHLGKTALITGGSAGIGSQIARLLALSGARV
MLCARDERKLIQMRDMIIAELTEVGYNQVESRVQICAGCDVGEEEQLEIAVQRTLDLFGH
VDYLINNAGIAGAEEMVLDLPLEAWQRTLRTNLISNYSLIRKLAPQMKSRGSGYILNVSS
YFGGEKYAAIPYPNRADYAVSKAGQRALGEALARLLGPEVQINAMAPGPVEGERLRGSGD
RPGLFLRRGRLILENKRLNDLHATLIAAERETQVGMRDLLARLLHNDVCALIDDPAAPTH
LRALAERIWEQSDPNSYARAFFMNANIATKLLARLFNADQIDAQTFHTSQPNLPPEPFFA
RTQIDREARRVRDGVMSMLYLQRMPTEFDVALATVYYLNDRSVSGETFHPSGGLRHERTP
TGAELYGSPAPQRLASLAGSTVYLIGESMAAHLEALARAYIERYAATRVVLICATPAGVE
RFSHHLADHLASGALAILSAEEGIEAALSEALRRFGPPGPVVSTPFQPLPSQPLIGRNDS
DWSTVLDVAGFSAMCEQQLTHHFRVTRKLSLVAGVSLVLVTPETDSHSSTEQFALANFVK
TTLHAFTATVGVECERTAHRILVNQVDLGRQARAEEPRSPAEQAQEMERFIDAIMLTTAP
IPAEEDNRYTGRIYRGRAITV
SEQ ID NO. 100: CHLOROFLEXUS AURANTIACUS (CAMCR)
MSGTGRLAGKIALITGGAGNIGSELTRRFLAEGATVIISGRNRAKLTALAERMQAEAGVP
AKRIDLEVMDGSDPVAVRAGIEAIVARHGQIDILVNNAGSAGAQRRLAEIPLTEAELGPG
AEETLHASIANLLGMGWHLMRIAAPHMPVGSAVINVSTIFSRAEYYGRIPYVTPKAALNA
LSQLAARELGARGIRVNTIFPGPIESDRIRTVFQRMDQLKGRPEGDTAHHFLNTMRLCRA
NDQGALERRFPSVGDVADAAVFLASAESAALSGETIEVTHGMELPACSETSLLARTDLRT
IDASGRTTLICAGDQIEEVMALTGMLRTCGSEVIIGFRSAAALAQFEQAVNESRRLAGAD
FTPPIALPLDPRDPATIDAVFDWGAGENTGGIHAAVILPATSHEPAPCVIEVDDERVLNF
LADEITGTIVIASRLARYWQSQRLTPGARARGPRVIFLSNGADQNGNVYGRIQSAAIGQL
IRVWRHEAELDYQRASAAGDHVLPPVWANQIVRFANRSLEGLEFACAWTAQLLHSQRHIN
EITLNIPANISATTGARSASVGWAESLIGLHLGKVALITGGSAGIGGQIGRLLALSGARV
MLAARDRHKLEQMQAMIQSELAEVGYTDVEDRVHIAPGCDVSSEAQLADLVERTLSAFGT
VDYLINNAGIAGVEEMVIDMPVEGWRHTLFANLISNYSLMRKLAPLMKKQGSGYILNVSS
YFGGEKDAAIPYPNRADYAVSKAGQRAMAEVFARFLGPEIQINAIAPGPVEGDRLRGTGE
RPGLFARRARLILENKRLNELHAALIAAARTDERSMHELVELLLPNDVAALEQNPAAPTA
LRELARRFRSEGDPAASSSSALLNRSIAAKLLARLHNGGYVLPADIFANLPNPPDPFFTR
AQIDREARKVRDGIMGMLYLQRMPTEFDVAMATVYYLADRNVSGETFHPSGGLRYERTPT

GGELFGLPSPERLAELVGSTVYLIGEHLTEHLNLLARAYLERYGARQVVMIVETETGAET
MRRLLHDHVEAGRLMTIVAGDQIEAAIDQAITRYGRPGPVVCTPFRPLPTVPLVGRKDSD
WSTVLSEAEFAELCEHQLTHHFRVARKIALSDGASLALVTPETTATSTTEQFALANFIKT
TLHAFTATIGVESERTAQRILINQVDLTRRARAEEPRDPHERQQELERFIEAVLLVTAPL
PPEADTRYAGRIHRGRAITV

## Claims

1. A genetically modified microorganism comprising a heterologous nucleic acid sequence from a prokaryotic organism encoding an accA-accD fused subunit, an accB non-fused subunit, and an accC non-fused subunit wherein molar ratios or ratios for accA:accB:accC:accD are about 1:2:1:1, or comprising a heterologous nucleic acid sequence from a prokaryotic organism encoding an accA-accD fused subunit, an accB non-fused subunit, and an accC non-fused subunit wherein molar ratios or ratios for said accDAfusion:accB:accC are about 1:2:1.

2. The genetically modified microorganism of claim 1, comprising about 10 contiguous polynucleotides or amino acids of any one of SEQ ID NOs: 1-100, and/or comprising an exogenous polynucleotide or polypeptide having at least 70% homology to a polynucleotide or polypeptide of any one of SEQ ID NOs 1-100.

3. The genetically modified microorganism of claim 1 or 2, having at least one disrupted gene selected from the group consisting of: araD, araB, lacZ, rhaD, rhaB, hsdR, ldhA, pflB, mgsA, poxB, pta-ack, fabI, fabB, fabF, fabD, aldA, aldB, puuC, and any combination thereof.

4. A method of producing a product, comprising employing the genetically modified organism of any of the above claims to produce the product, wherein said product is selected from the group consisting of acetyl-CoA, malonyl-CoA, malonate semialdehyde, 3-hydroxypropionic acid (3-HP), acrylic acid, 1,3 propanediol, malonic acid, ethyl 3-HP, propiolactone, acrylonitrile, acrylamide, methyl acrylate, a polymer, a superabsorbent polymer, polyacrylic acid, consumer product, and any combination thereof, or wherein said product is 3-hydroxypropionic acid (3-HP), a derivative of 3-HP, 1,4-butanediol, butanol, isobutanol, a polyketide chemical product, a C4-C18 fatty acid chain, or any combination thereof, optionally to which an alkyl or aryl addition is made, and/or to which halogens, aromatic amines or amides, and/or aromatic hydrocarbons are added.

## Patentansprüche

1. Genetisch veränderter Mikroorganismus, der eine heterologe Nukleinsäuresequenz aus einem prokaryotischen Organismus umfasst, der für eine accA-accD-fusionierte Untereinheit, eine accB-nicht fusionierte Untereinheit und eine accC-nicht fusionierte Untereinheit codiert, wobei Molverhältnisse oder Verhältnisse für accA:accB:accC:accD etwa 1:2:1:1 betragen, oder der eine heterologe Nukleinsäuresequenz aus einem prokaryotischen Organismus umfasst, der für eine accA-accD-fusionierte Untereinheit, eine accB-nicht fusionierte Untereinheit und eine accC-nicht fusionierte Untereinheit codiert, wobei Molverhältnisse oder Verhältnisse für die accDAfusion:accB:accC etwa 1:2:1 betragen.

2. Genetisch veränderter Mikroorganismus nach Anspruch 1, der etwa 10 zusammenhängende Polynukleotide oder Aminosäuren einer beliebigen einen der SEQ ID NOs: 1-100 umfasst und/oder ein exogenes Polynukleotid oder Polypeptid umfasst, das wenigstens 70 % Homologie zu einem Polynukleotid oder Polypeptid einer beliebigen einen der SEQ ID NOs 1-100 aufweist.

3. Genetisch veränderter Mikroorganismus nach Anspruch 1 oder 2, der wenigstens ein unterbrochenes Gen aufweist, das aus der Gruppe ausgewählt ist, die aus Folgendem besteht: araD, araB, lacZ, rhaD, rhaB, hsdR, IdhA, pflB, mgsA, poxB, pta-ack, fabl, fabB, fabF, fabD, aldA, aldB, puuC und einer beliebigen Kombination davon.

4. Verfahren zum Erzeugen eines Erzeugnisses, das ein Nutzen des genetisch veränderten Organismus nach einem der vorherigen Ansprüche umfasst, um das Erzeugnis zu erzeugen, wobei das Erzeugnis aus der Gruppe ausgewählt ist, die aus Acetyl-CoA, Malonyl-CoA, Malonatsemialdehyd, 3-Hydroxypropionsäure (3-HP), Acrylsäure, 1,3-Propandiol, Malonsäure, Ethyl-3-HP, Propiolacton, Acrylnitril, Acrylamid, Methylacrylat, einem Polymer, einem superabsorbierenden Polymer, Polyacrylsäure, Verbrauchererzeugnis und einer beliebigen Kombination davon besteht, oder wobei das Erzeugnis 3-Hydroxypropionsäure (3-HP), ein Derivat von 3-HP, 1,4-Butandiol, Butanol, Isobutanol, ein chemisches Polyketiderzeugnis, eine C4-C18-Fettsäurekette oder eine beliebige Kombination davon ist, an dem optional eine Alkyl- oder Aryladdition vorgenommen wird und/oder zu dem Halogene, aromatische Amine oder Amide und/oder aromatische Kohlenwasserstoffe zugegeben werden.

## Revendications

1. Micro-organisme génétiquement modifié comprenant une séquence d'acide nucléique hétérologue issue d'un organisme procaryote codant pour une sous-unité fusionnée accA-accD, une sous-unité non fusionnée accB et une sous-unité non fusionnée accC, des rapports molaires ou des rapports pour accA:accB:accC:accD étant d'environ 1:2:1:1, ou comprenant une séquence d'acide nucléique hétérologue issue d'un organisme procaryote codant pour une sous-unité fusionnée accA-accD, une sous-unité non fusionnée accB et une sous-unité non fusionnée accC, des rapports molaires ou des rapports pour lesdits accDAfusion:accB:accC étant d'environ 1:2:1.

2. Microorganisme génétiquement modifié selon la revendication 1, comprenant environ 10 polynucléotides ou acides aminés contigus de l'une quelconque des SEQ ID N° : 1 à 100, et/ou comprenant un polynucléotide ou polypeptide exogène présentant au moins 70 % d'homologie avec un polynucléotide ou polypeptide de l'une quelconque des SEQ ID N° 1 à 100.

3. Microorganisme génétiquement modifié selon la revendication 1 ou 2, ayant au moins un gène disrupté choisi dans le groupe constitué de : araD, araB, lacZ, rhaD, rhaB, hsdR, IdhA, pflB, mgsA, poxB, pta-ack, fabl, fabB, fabF, fabD, aldA, aldB, puuC, et toute combinaison de ceux-ci.

4. Procédé de production d'un produit, comprenant l'emploi de l'organisme génétiquement modifié selon l'une quelconque des revendications précédentes pour produire le produit, ledit produit étant choisi dans le groupe constitué de l'acétyl-CoA, du malonyl-CoA, du malonate semialdéhyde, de l'acide 3-hydroxypropionique (3-HP), de l'acide acrylique, du propanediol-1,3, de l'acide malonique, du 3-HP d'éthyle, de la propiolactone, de l'acrylonitrile, de l'acrylamide, de l'acrylate de méthyle, d'un polymère, d'un polymère superabsorbant, de l'acide polyacrylique, d'un produit de consommation et de toute combinaison de ceux-ci, ledit produit étant l'acide 3-hydroxypropionique (3-HP), un dérivé de 3-HP, le butanediol-1,4, le butanol, l'isobutanol, un produit chimique à base de polycétide, une chaîne d'acide gras en C4 à C18, ou toute combinaison de ceux-ci, à laquelle est éventuellement effectuée une addition alkyle ou aryle, et/ou à laquelle des halogènes, des amines aromatiques ou des amides et/ou des hydrocarbures aromatiques sont ajoutés.
